(19) Europäisches Patentamt
European Patent Office
Office européen des brevets

(11) Publication number: 0 184 257 B1

(12) **EUROPEAN PATENT SPECIFICATION**

(45) Date of publication of patent specification: **25.07.90**

(21) Application number: **85201937.1**

(22) Date of filing: **22.11.85**

(51) Int. Cl.$^5$: **C 07 D 413/12**, C 07 D 417/12, C 07 D 413/14, C 07 D 417/14, C 07 D 498/04, C 07 D 513/04, A 61 K 31/445, A 61 K 31/42, A 61 K 31/425

(54) **Benzoxazol- and benzothiazolamine derivatives.**

(30) Priority: **03.12.84 US 677412**

(43) Date of publication of application: **11.06.86 Bulletin 86/24**

(45) Publication of the grant of the patent: **25.07.90 Bulletin 90/30**

(84) Designated Contracting States: **AT BE CH DE FR GB IT LI LU NL SE**

(56) References cited:
**DE-A-1 804 450**
**DE-A-2 545 645**
**US-A-2 623 878**

**Chemical Abstracts, vol. 97, no. 5, August 2, 1982, Columbus, Ohio, USA Marchalin, Miroslav; Povazanec, Frantisek; Martvon, Augustin "A study of the reaction of 2-pyridyl isothiocyanate with azoimide." page 570, column 2, abstract no. 38 892h**

**Chemical Abstracts, vol. 78, no. 25, June 25, 1973, Columbus, Ohio, USA Hisano, Takuzo; Yabuta, Yuko "Synthesis of organosulfur compounds. VIII.Cyclization products from the modified Willgerodt-Kindler reaction." page 408, column 1, abstract no. 159 505a**

**Chemical Abstracts, vol. 90, no. 21, May 21, 1979, Columbus, Ohio, USA Simov, D.; Davidkov, K. "Valence vibrations of the azomethine group of 2-aminobenzoxazole derivatives." page 518, column 2, abstract no. 167 524u**

**Chemical Abstracts, vol. 98, no. 21, May 23, 1983, Columbus, Ohio,USA Merchan, F.L.;**

(73) Proprietor: **JANSSEN PHARMACEUTICA N.V.**
**Turnhoutsebaan 30**
**B-2340 Beerse (BE)**

(72) Inventor: **Stokbroekx, Raymond Antoine**
**Rode Kruisstraat 13**
**B-2340 Beerse (BE)**
Inventor: **Luyckx, Marcel Gerebernus Maria**
**Eksterstraat 24**
**B-2440 Geel (BE)**
Inventor: **Janssens, Frans Eduard**
**Tinstraat 79**
**B-2830 Bonheiden (BE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 184 257 B1

(56) References cited:

Garin, J.; Melendez, E.; Tejero, T. "2-(-2-Benzimidazolylamino)benzothiazoles and 2-(2-imidazolidinylidenamino)benzothiazoles." page 651, column 1, abstract no. 179 281r

Chemical Abstracts, vol. 98, no. 13, March 28, 1983, Columbus, Ohio, USA Ahuja, Padma; Singh, Jujhar; Nigam, M.B.; Sardana, Vinod; Kar, K.; Anand, Nitya "Lactam acetals. Part VII.Synthesis and biological activities of some heterocyclic amidines and diamidines." page 584, column 1, abstract no. 107 106q

Chemical Abstracts, vol. 99, no. 7, August 15, 1983, Columbus, Ohio, USA Singh S.P.; Prakash, Indra; Tomer, R.K.; Prakash, O.M.; Sawhney, S.N. "Synthesis and mass spectra of some substituted 2-(2'-benzazolylamino)pyrimidines." page 553, column 2, abstract no. 53 691b

## Description

In U.S. Patent No. 4,219,559 there are described a number of *N*-heterocyclyl-4-piperidinamines, wherein said heterocyclyl is imidazole being condensed with benzene or pyridine, which compounds are useful as antihistaminic agents.

The compounds of the present invention containing an analogous heterocyclyl radical differ therefrom by the fact that said heterocyclyl is oxazole or thiazole being condensed with benzene or pyridine and by their unexpected anit-anoxic properties.

This invention is concerned with novel benzothiazol- and benzoxazol-amine derivatives which structurally may be represented by the formula

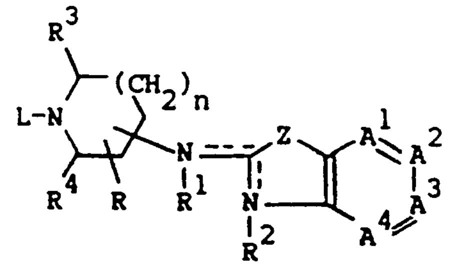

(I)

the pharmaceutically acceptable acid addition salts and the possible stereochemically isomeric forms thereof, wherein:

$-A^1=A^2-A^3=A^4-$ is a bivalent radical having the formula

| | |
|---|---|
| $-CH=CH-CH=CH-$ | (a); |
| $-N=CH-CH=CH-$ | (b); |
| $-CH=N-CH=CH-$ | (c); |
| $-CH=CH-N=CH-$ | (d); or |
| $-CH=CH-CH=N-$ | (e); |

wherein one or two hydrogen atoms in the radical $-A^1=A^2-A^3=A^4-$ may, each independently from each other, be replaced by halo, $C_{1-6}$ alkyl, hydroxy, $C_{1-6}$ alkyloxy, or trifluoromethyl;

Z is O or S;

n is 0 or the integer 1;

R is a member selected from the group consisting of hydrogen, $C_{1-6}$ alkyl, hydroxy and $C_{1-6}$ alkyloxy;

$R^1$ or $R^2$ is hydrogen, $C_{1-6}$ alkyl, or aryl $C_{1-6}$ alkyl; the dotted line between the nitrogen atom bearing $R^1$, the interjacent carbon atom and the nitrogen atom bearing $R^2$ indicating that a double bond exists between the nitrogen bearing $R^1$ and said interjacent carbon atom, in which case $R^1$ is absent, or a double bond exists between the interjacent carbon and the nitrogen bearing $R^2$, in which case $R^2$ is absent; and wherein said interjacent carbon atom is the carbon atom positioned between the nitrogen bearing $R^1$ and the nitrogen bearing $R^2$;

$R^3$ or $R^4$ are both hydrogens or $R^3$ and $R^4$ combined may form a bivalent radical of formula $-CH_2-CH_2-$;

L is a member selected from the group consisting of $C_{1-12}$ alkyl, substituted $C_{1-6}$ alkyl, aryl $C_{2-6}$ alkenyl, $C_{3-6}$ cycloalkyl, substituted $C_{3-6}$ cycloalkyl, a radical of formula

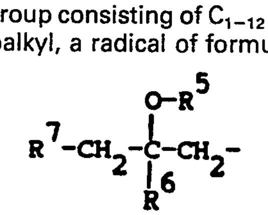

(a),

and a radical of formula

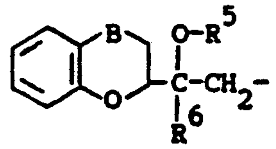

(b);

$R^5$ being hydrogen or $C_{1-12}$ alkylcarbonyl;

$R^6$ being hydrogen or $C_{1-6}$ alkyl;

$R^7$ being aryl, a radical $R^8-O$ or a radical $R^8-S$;

B being a bivalent radical of formula $-CH_2-$ or $-O-$; and

said $R^8$ being hydrogen; aryl; 2,3-dihydro-1*H*-indenyl; benzodioxolyl; (2,3-dihydro-1,4-benzodioxin-2-yl)methyl; (2*H*-1-benzopyran-2-yl)methyl; or phenyl substituted with $C_{2-6}$ alkenyloxy, $C_{1-6}$ alkylcarbonylamino or $C_{1-6}$ alkylphenylcarbonyl; and

wherein said substituted $C_{3-6}$ cycloalkyl is $C_{3-6}$ cycloalkyl being substituted with up to two substituents each independently selected from the group consisting of aryl, aryloxy and cyano;

said substituted $C_{1-6}$ alkyl is $C_{1-6}$ alkyl being substituted with a member selected from the group consisting of $C_{3-6}$cycloalkyl, pyridinyl, pyridinyloxy, aryl, benzimidazolyl, indolyl, isoxazolyl being optionally substituted with phenyl, 3-oxo-1,2,4-triazolo[4,5-a]pyridin-2(3*H*)-yl, a radical of formula ArY—, a radical of formula $R^9O$— and a radical of formula

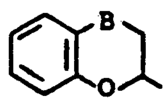

(c);

said Ar being aryl;

Y being O, S, NH, —CONH—, —CHCN, —$CHOR^{10}$ or —$SO_2$;

$R^9$ being hydrogen, aryl $C_{1-6}$ alkyl, $C_{3-6}$ cycloalkyl, phenyl substituted with phenyl, or phenyl substituted with $C_{1-6}$ alkyloxycarbonyl;

$R^{10}$ being hydrogen or $C_{1-6}$ alkyl; and

wherein aryl is phenyl optionally substituted with up to three substituents each independently selected from the group consisting of $C_{1-6}$ alkyl, $C_{1-6}$ alkyloxy, halo, trifluoromethyl, cyano, $C_{1-6}$ alkylcarbonyl, nitro, amino and aminocarbonyl.

As used herein the term halo is generic to fluoro, chloro, bromo and iodo; "$C_{1-6}$ alkyl" is meant to include straight and branched saturated hydrocarbon radicals, having from 1 to 6 carbon atoms, such as, for example, methyl, ethyl, 1-methylethyl, 1,1-dimethylethyl, propyl, butyl, pentyl, hexyl and the like; $C_{1-12}$ alkyl is meant to include straight and branched saturated hydrocarbon radicals, having from 1 to 12 carbon atoms; "$C_{2-6}$ alkenyl" refers to alkenyl radicals having from 2 to 6 carbon atoms, such as, for example, 2-propenyl, 2-butenyl, 3-butenyl, 2-pentenyl and the like; and "$C_{3-6}$ cycloalkyl" embraces cyclopropyl, cyclobutyl, cyclopentyl and cyclohexyl.

Preferred compounds within the invention are those wherein L is aryl $C_{2-6}$ alkenyl, $C_{1-6}$ alkyl being substituted with aryloxy, a radical of formula (a) or a radical of formula (b) and aryl is as defined hereinabove.

Particularly preferred compounds within the invention are those wherein L is as described hereinabove for the preferred compounds and —$A^1$=$A^2$—$A^3$=$A^4$— is —CH=CH—CH=CH—, n is the integer 1, $R^2$ is absent and a double bond exists between the nitrogen bearing $R^2$ and the interjacent carbon atom, and $R^3$ and $R^4$ are both hydrogen atoms.

More particularly preferred compounds within the invention are those wherein —$A^1$=$A^2$—$A^3$=$A^4$-, n, $R^2$ and $R^3$ are as defined hereinabove for the particularly preferred compounds and wherein a double bond exists between the nitrogen bearing $R^2$ and the interjacent carbon atom, and wherein L is $C_{1-6}$ alkyl substituted with aryloxy, a radical of formula (a) wherein $R^5$ and $R^6$ are both hydrogen and $R^7$ is a radical $R^8$—O—, wherein $R^8$ is aryl, or L is a radical of formula (b), wherein B is 0, $R^5$ and $R^6$ are both hydrogen radicals, and aryl is as defined hereinabove.

Especially preferred compounds within the invention are those particularly preferred compounds wherein $R^1$ is $C_{1-6}$ alkyl and aryl is halophenyl.

The most preferred compounds are selected from the group consisting of 4-[(2-benzothiazolyl)methylamino]-α-[(4-fluorophenoxy)methyl]-1-piperidineethanol and the pharmaceutically acceptable acid addition salts thereof.

In order to simplify the structural representations of the compounds of formula (I) and of certain precursors and intermediates thereof the

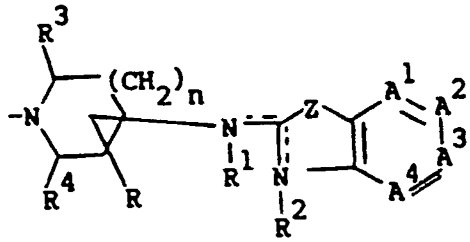

-radical will hereafter be represented by the symbol D.

The compounds of formula (I) can generally be prepared by *N*-alkylating an intermediate of formula (III) with a reagent of formula (II).

$$\underset{(II)}{L\text{-}W} + \underset{(III)}{H\text{-}D} \xrightarrow{\underline{\text{N-alkylation}}} L\text{-}D \qquad (I)$$

In (II) W represents an appropriate reactive leaving group such as, for example, halo, e.g., chloro, bromo or iodo, or a sulfonyloxy group, e.g. methylsulfonyloxy or 4-(methylphenyl)sulfonyloxy. The alkylation reaction is conveniently conducted in an inert organic solvent such as, for example, an aromatic hydrocarbon, e.g., benzene, methylbenzene, dimethylbenzene; a $C_{1-6}$ alkanol, e.g., methanol, ethanol, 1-butanol; a ketone, e.g., 2-propanone, 4-methyl-2-pentanone; an ether, e.g., 1,4-dioxane, 1,1'-oxybisethane, tetrahydrofuran; *N,N*-dimethylformamide (DMF); *N,N*-dimethylacetamide (DMA); dimethyl sulfoxide (DMSO); nitrobenzene; 1-methyl-2-pyrrolidinone. The addition of an appropriate base such as, for example, an alkali metal carbonate or hydrogen carbonate, sodium hydride or an organic base such as, for example, *N,N*-diethylethanamine or *N*-(1-methylethyl)-2-propanamine may be utilized to pick up the acid which is liberated during the course of the reaction. In some circumstances the addition of an iodide salt, preferably an alkali metal iodide, is appropriate. Somewhat elevated temperatures may enhance the rate of the reaction.

The compounds of formula (I) can also be prepared by the reductive N-alkylation reaction of (III) with an appropriate carbonyl-compound of formula L'=C=O (IV), said L'=C=O being a compound of formula L—H wherein a —$CH_2$— radical is oxidated to a carbonyl radical.

$$\underset{(IV)}{L'{=}C{=}O} + (III) \xrightarrow[\text{reaction}]{\text{reductive } \underline{N}\text{-alkylation}} L\text{-}D \qquad (I)$$

Said reductive *N*-alkylation reaction may conveniently be carried out by catalytically hydrogenating a stirred and heated mixture of the reactants in a suitable reaction-inert organic solvent according to art-known catalytic hydrogenating procedures. The reaction mixture may be stirred and/or heated in order to enhance the reaction rate. Suitable solvents are, for example, water; $C_{1-6}$ alkanols, e.g. methanol, ethanol, 2-propanol; cyclic ethers, e.g. 1,4-dioxane; halogenated hydrocarbons, e.g. trichloromethane; *N,N*-dimethylformamide) dimethyl sulfoxide; or a mixture of 2 or more of such solvents. The term "art-known catalytic hydrogenating procedures" means that the reaction is carried out under hydrogen atmosphere and in the presence of an appropriate catalyst such as, for example, palladium-on-charcoal, platinum-on-charcoal. In order to prevent the undesired further hydrogenation of certain functional groups in the reactants and the reaction products it may be advantageous to add an appropriate catalyst-poison to the reaction mixture, e.g. thiophene.

The compounds of formula (I) can also be prepared by oxidatively cyclizing an urea or thiourea derivative of formula (V) in the presence of an appropriate oxidant.

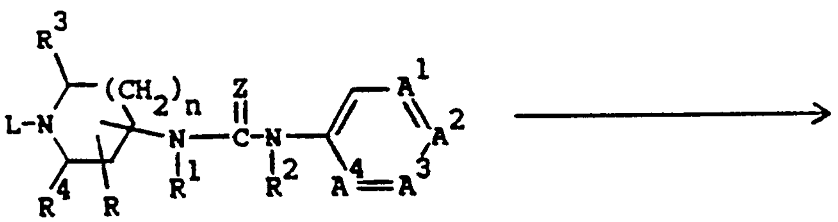
(I)

(V)

Appropriate oxidants are, for example, halogens, e.g. chlorine and bromine, thionyl chloride, sulfuryl chloride, thionyl bromide, sulfuryl bromide. The said cyclization reaction is most conveniently conducted in a reacton-inert solvent such as, for example, an halogenated hydrocarbon, e.g. tetrachloromethane, trichloromethane. Elevated temperatures may be appropriate to enhance the reaction rate.

The compounds of formula (I) wherein L is a radical of formula (a) or (b) wherein $R^5$ is hydrogen can also be prepared by reacting an appropriate oxirane derivative of formula (VI), respectively of formula (VIII) with (III). The thus obtained compounds of formula (I-a-1) respectively of formula (I-a-3) may further be converted into the corresponding compounds of formula (I-a-2), respectively (I-a-4) by an appropriate acylation reaction.

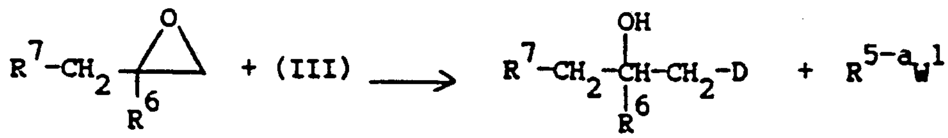

(VI) + (III) → (I-a-1) + (VII)

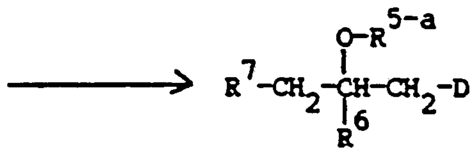

(I-a-2)

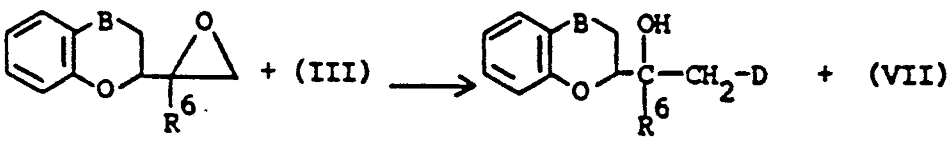

(VIII) + (III) → (I-a-3) + (VII)

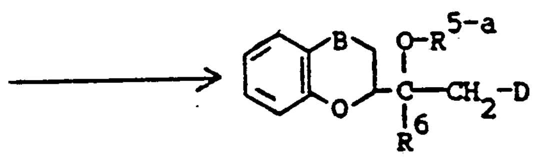

(I-a-4)

The reagent $R^{5-a}W^1$ represents an alkanoic acid or a reactive derivative thereof such as, for example, an ester, an acid halide or anhydride.

The reaction of (VI) or (VIII) with (III) may conveniently be conducted in an appropriate solvent such as, for example, an aromatic hydrocarbon, e.g. benzene, methylbenzene, a $C_{1-6}$ alkanol, e.g. methanol and ethanol. Elevated temperatures may be appropriate to enhance the reaction rate.

The compounds of formula (I), wherein $R^2$ is absent and a double bond exists between the nitrogen bearing $R^2$ and the previously defined interjacent carbon atom, said compounds being represented by the formula (I-b) can also be prepared by reacting an appropriate reagent of formula (IX) with a benzothiazole or benzoxazole of formula (X).

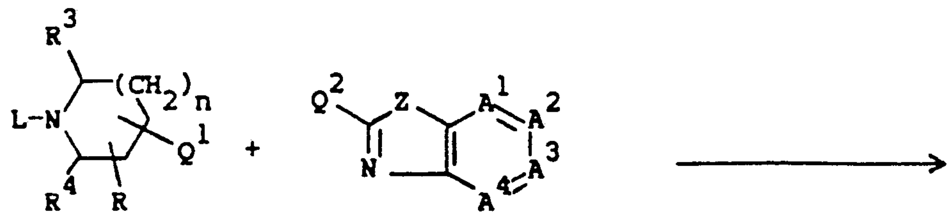

(IX) (X)

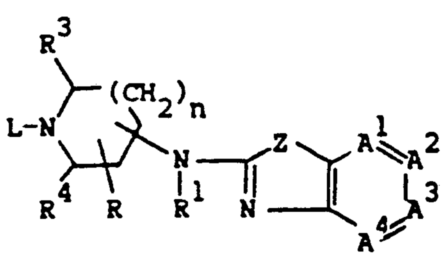

(I-b)

In (IX) and (X) $Q^1$ and $Q^2$ are selected so that during the alkylation reaction a radical of formula $—NR^1—$ is formed. For example, where $Q^1$ is an appropriate leaving group, $Q^2$ is a radical of formula $—NHR^1$, or where $Q^1$ is a radical of formula $—NHR^1$, $Q^2$ is an appropriate leaving group.

The compounds of formula (I-b) wherein Z is O and $R^1$ is hydrogen, said compounds being represented by the formula (I-b-1), can also be prepared by cyclodesulfurizing an intermediate of formula (XIV), which may conveniently be formed in situ by condensing an isothiocyanate (XII) with an aromatic aminoalcohol of formula (XIII).

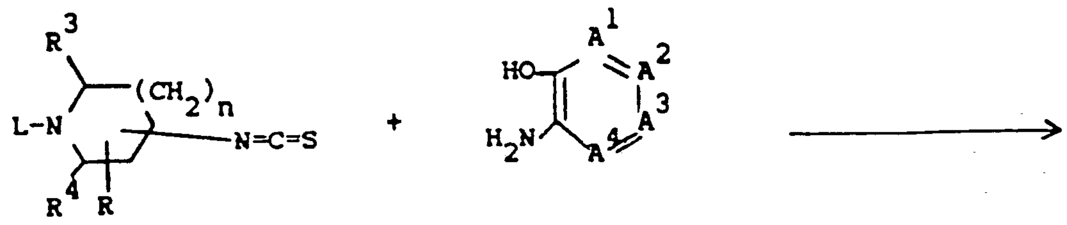

(XII) (XIII)

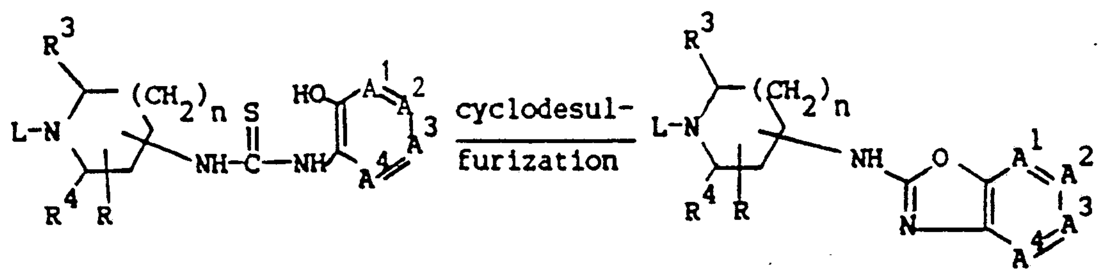


(XIV) (I-b-1)

Said cyclodesulfurization reaction may be carried out by the reaction of (XIV) with an appropriate alkyl halide, preferably iodomethane in an appropriate reaction-inert organic solvent, e.g., a $C_{1-6}$ alkanol such as methanol, ethanol, 2-propanol. Otherwise, the cyclodesulfurization reaction may be carried out by the reaction of (XIV) with an appropriate metal oxide or salt in an appropriate solvent according to art-known procedures. For example, the compounds of formula (I) can easily be prepared by the reaction of (XIV) with an appropriate Hg(II) or Pb(II) oxide or salt, such as, for example HgO, $HgCl_2$, $Hg(OAc)_2$, PbO or $Pb(OAc)_2$. In certain instances it may be appropriate to supplement the reaction mixture with a small amount of sulfur. Even so methanediimines, especially *N,N'*-methanetetraylbis-[cyclohexanamine] may be used as cyclodesulfurizing agents.

The compounds of formula (I-b) wherein $R^1$ is hydrogen, said compounds being represented by the formula (I-b-2), may be prepared by cyclizing an intermediate (XVII), which in situ may be formed by reacting an isocyanate or isothiocyanate (XV) with an aromatic amine (XVI).

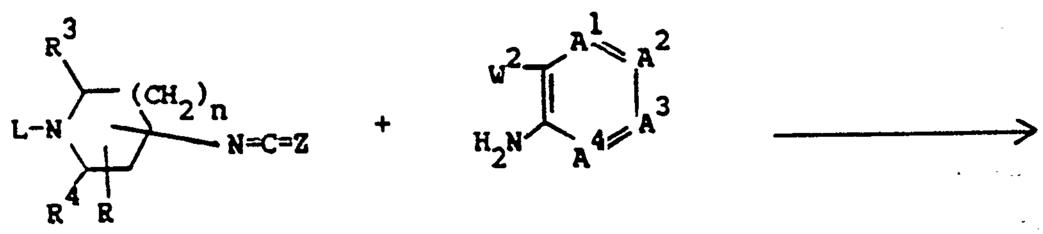

(XV) (XVI)

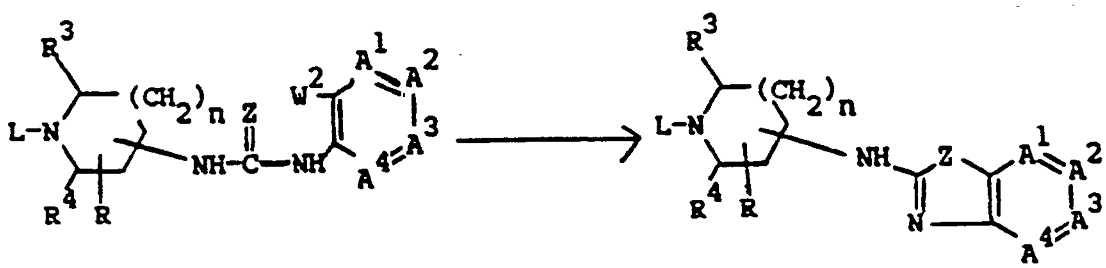

(XVII) (I-b-2)

In (XVI) and (XVII) $W^2$ represents an appropriate leaving group such as, for example, halo, e.g. chloro and bromo; $C_{1-6}$ alkyloxy and $C_{1-6}$ alkylthio.

The compounds of formula (I) wherein L is a $C_{1-12}$ alkyl radical or a substituted $C_{1-6}$ alkyl radical, wherein said $C_{1-12}$ alkyl or $C_{1-6}$ alkyl contains at least two carbon atoms, said compounds being represented by the formula (I-c), may also be prepared by reacting an alkene of formula (XVIII) with an intermediate of formula (III) by stirring and, if desired, heating the reactants together, preferably in a suitable solvent such as, for example, an alcohol, e.g. methanol, ethanol, butanol.

$$R^{11}\text{—}C_{2-6}\text{ alkenediyl-H (XVIII)} + \text{(III)} \rightarrow R^{11}\text{—}C_rH_{2r}\text{-D (I-c)}$$

In (XVIII) $R^{11}$ represents $C_{1-6}$ alkyl, $C_{3-6}$ cycloalkyl, pyridinyl, pyridinyloxy, aryl, benzimidazolyl, indolyl, isoxazolyl being substituted with phenyl, 3-oxo-1,2,4-triazolo[4,5-a]pyridin-2(3*H*)-yl, a radical of formula ArY, a radical of formula $R^9O$ and a radical of formula (g); and in (I-c) r is an integer of from 2 to 6 included.

Additionally, the compounds of formula (I), wherein L is a radical of formula Ar—CO—NH—$CH_2$—$CH_2$—, said compounds being represented by the formula (I-d), can also be prepared by reacting an aziridine of formula (XIX) with (III), preferably in the presence of a suitable solvent such as, for example, an aromatic hydrocarbon, e.g. benzene, methylbenzene.

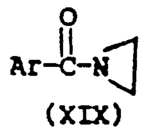

(XIX)

+ (III)

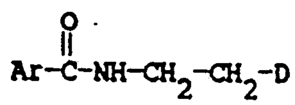

(I-d)

The compounds of formula (I) can also be converted into each other following art-known procedures. The compounds of formula (I) wherein $R^1$ is hydrogen may be converted into compounds of formula (I) wherein $R^1$ is other than hydrogen by reacting the former compounds with an appropriate *N*-alkylating reagent.

The compounds of formula (I) wherein L is a $C_{1-6}$ alkyl radical substituted with hydroxy may further be *O*-alkylated with an appropriate reagent following art-known *O*-alkylating procedures.

In all of the foregoing and in the following preparations, the reaction products may be isolated from the reaction mixture and, if necessary, further purified according to methodologies generally known in the art.

The compounds of formula (I) have basic properties and, consequently, they may be converted to their therapeutically active non-toxic acid addition salt forms by treatment with appropriate acids, such as, for example, inorganic acids, such as hydrohalic acid, e.g. hydrochloric, hydrobromic, and sulfuric acid, nitric acid, phosphoric acid; or organic acids, such as, for example, acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3-propanetricarboxylic, methanesulfonic, ethanesulfonic, benzenesulfonic, 4-methylbenzenesulfonic, cyclohexanesulfamic, benzoic, 2-hydroxybenzoic, 3-phenyl-2-propenoic, α-hydroxybenzeneacetic, 4-amino-2-hydroxybenzoic acid. Conversely the salt form can be converted by treatment with alkali into the free base form.

A number of intermediates and starting materials in the foregoing preparations are known compounds which may be prepared according to art-known methodologies of preparing said or similar compounds. A number of such preparation methods will be described hereinafter in more detail.

The intermediates of formula (II) wherein L is $C_{1-6}$ alkyl being substituted with pyridinyloxy, aryloxy, arylthio, arylamino or a radical $R^{9-a}O$—, said $R^{9-a}$ having the previously defined meaning of $R^9$, provided that $R^{9-a}$ is other than hydrogen, can be prepared by alkylating an appropriate alcohol, thioalcohol or amine with a reagent W—$C_{1-6}$ alkyl-W, or alternatively with a reagent W-Alk-OH, and subsequently converting the thus formed alcohol with a reagent capable of converting an alcohol function into a reactive leaving group, e.g. thionyl chloride, methylsulfonyl chloride.

The intermediates of formula (III) can generally be prepared by oxidatively cyclizing an urea or thiourea derivative of formula (XX), wherein P is an appropriate leaving group such as, for example, $C_{1-6}$ alkyloxycarbonyl, phenylmethoxycarbonyl, phenylmethyl, following the same procedures described hereinabove for the preparation of (I) starting from (V) and, subsequently eliminating the protective group P in the thus obtained intermediate (XXI)

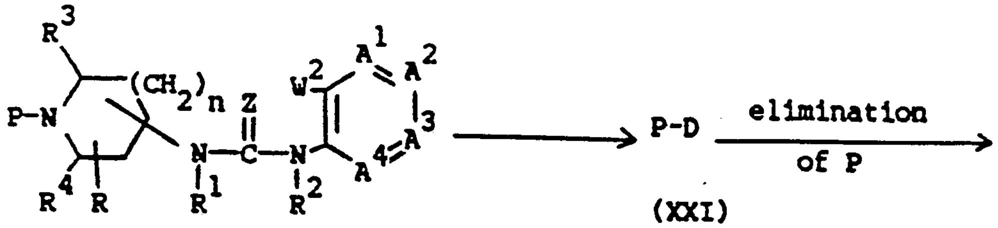


(XX)

(XXI)

(III)

The elimination of the protective group P in (XXI) may generally be carried out following art-known procedures such as, for example, by hydrolysis in alkaline or acidic aqueous medium.

The intermediates of formula (III) wherein $R^2$ is absent and a double bond exists between the nitrogen bearing said $R^2$ and the previously defined interjacent carbon atom, and wherein Z is O, said intermediates being represented by the formula (III-a) may alternatively be prepared by cyclodesulfurizing a thiourea of formula (XXIII), which may in situ be formed by condensing an isothiocyanate of formula (XXII) with an aromatic alcohol (XIII) following the procedures described hereinabove for the preparation of (I-b-1) starting from (XII) and (XIII), and subsequently eliminating the protective group P in the thus obtained intermediate (XXIV) as described hereinabove.

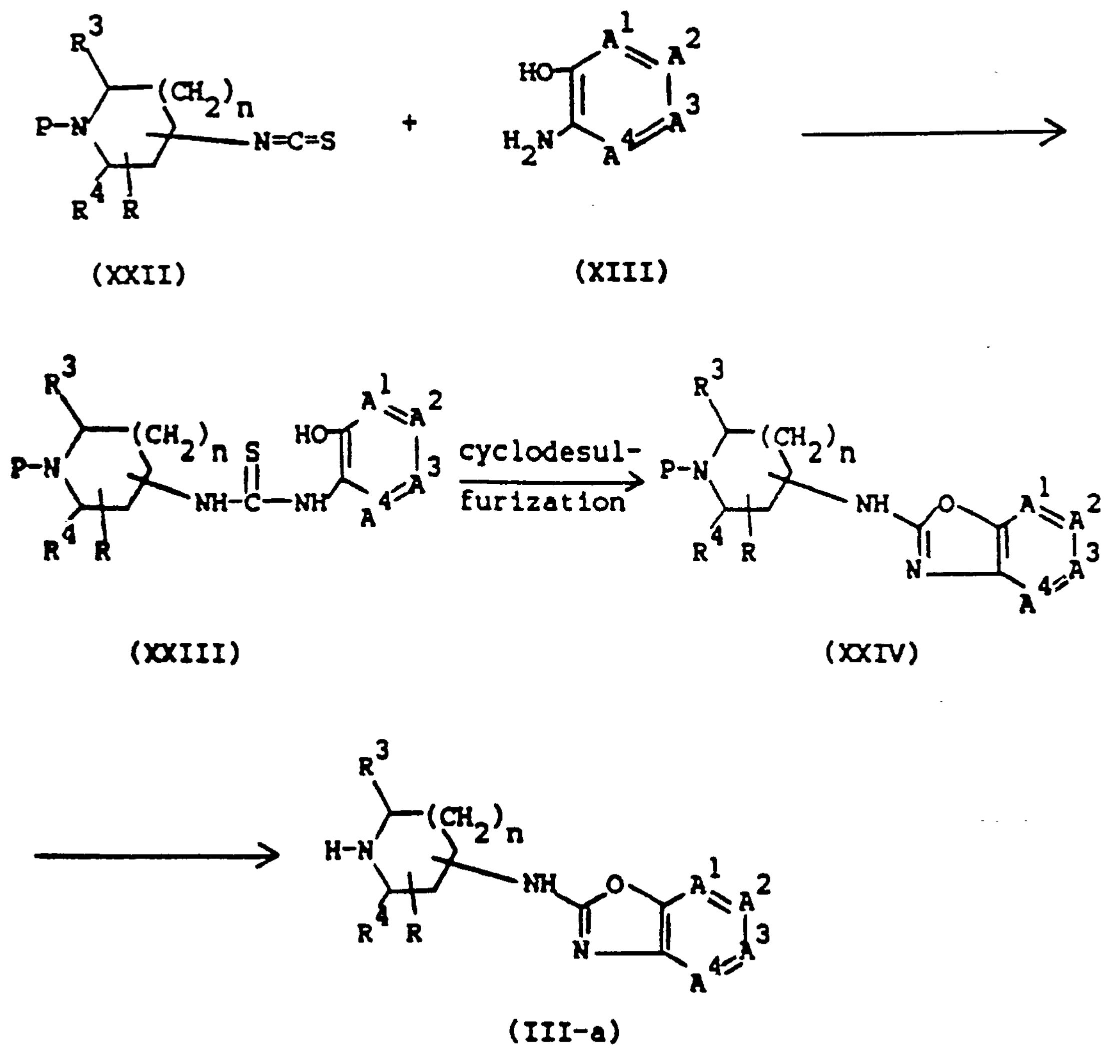


(XXII) (XIII)

(XXIII) (XXIV)

(III-a)

The intermediates of formula (III) wherein $R^2$ is absent and a double bond exists between the nitrogen bearing said $R^2$ and the previously defined interjacent carbon atom, said intermediates being represented by the formula (III-b) can also be prepared by cyclizing an intermediate (XXVI) which in situ may be formed by reacting an isocyanate or isothiocyanate (XXV) with (XVI), and subsequently eliminating the protective group P as described hereinabove.

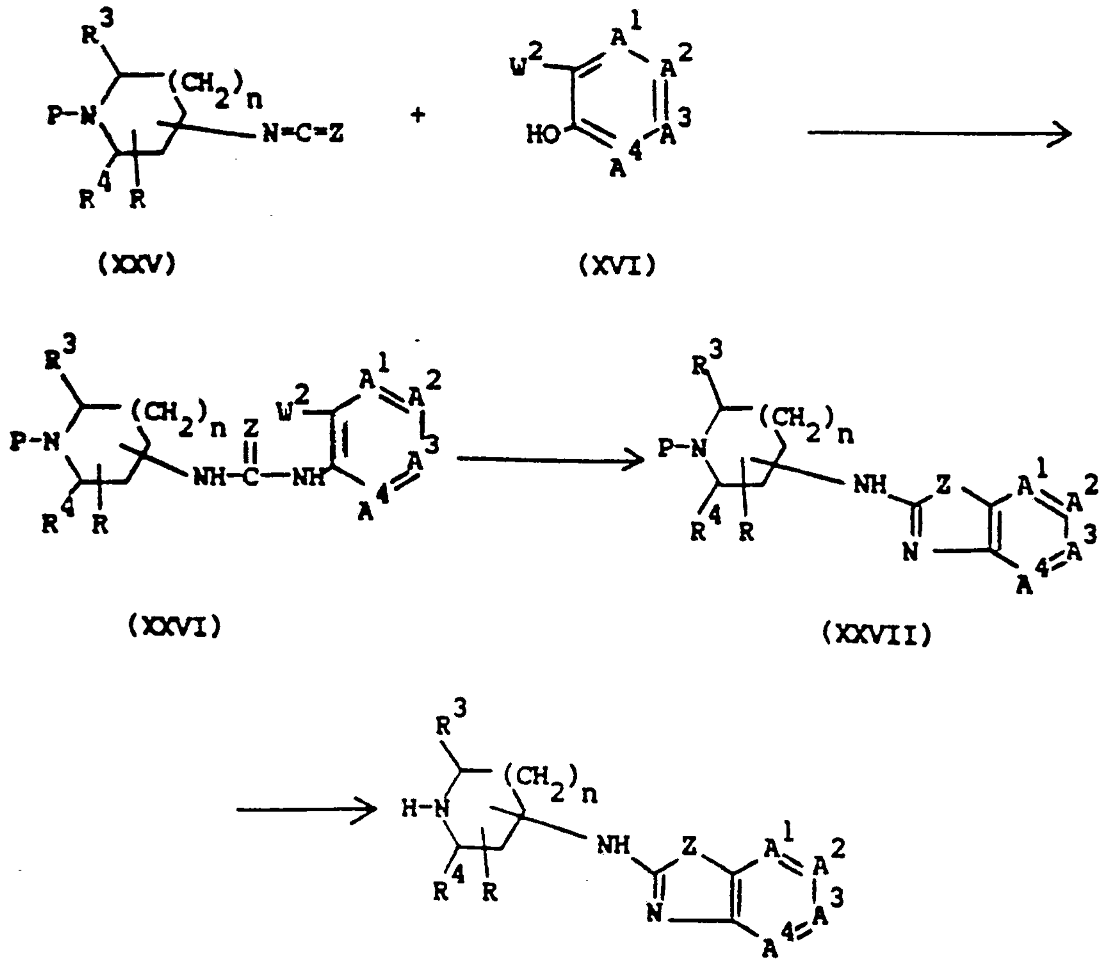

(XXV) (XVI)

(XXVI) (XXVII)

(III-b)

The previously described intermediates can also be converted into each other following art-known functional group transformation procedures.

For example, the intermediates of formula (V) wherein $R^1$ is hydrogen and the intermediates of formula (V) wherein $R^2$ is hydrogen, said intermediates being represented by the formula (V-a), respectively (V-b) can be prepared by reacting an isocyanate or isothiocyanate (XXVIII) with an aromatic amine (XXIX), respectively by reacting an amine (XXX) with an aromatic isocyanate or isothiocyanate (XXXI).

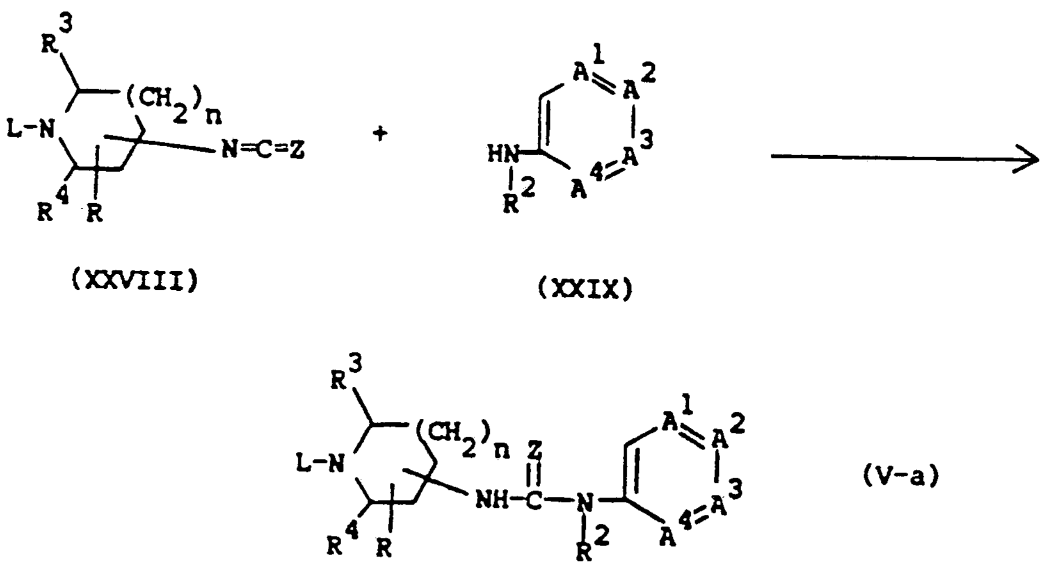

(XXVIII) (XXIX)

(V-a)

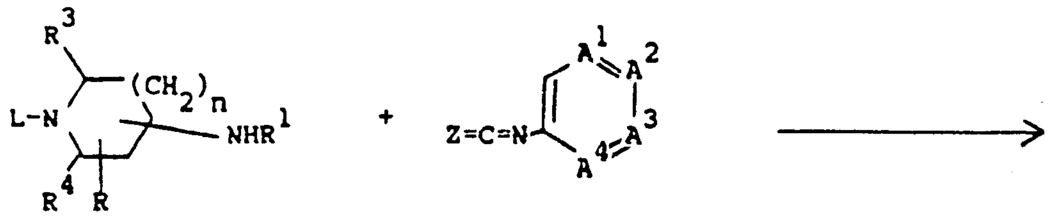

(XXX) (XXXI)

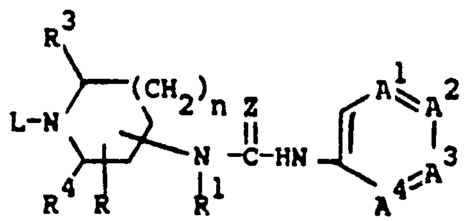

(V-b)

The intermediates of formula (VI) and of formula (VII) may be prepared by epoxidizing an alkene reagent of formula (XXXII) respectively of formula (XXXIII) with an appropriate epoxidizing reagent, e.g. with an aromatic peroxoacid, in a suitable solvent.

$R^7-CH_2-CR^6=CH_2$

(XXXII)

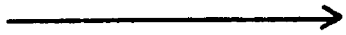

(VI)

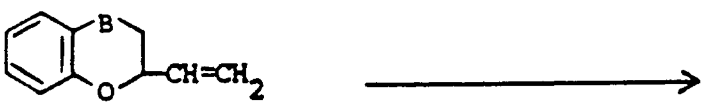

(VII)

(XXXIII)

The intermediates of formula (XIX) may be prepared following the procedures described hereinabove for the preparation of (V-a) and (V-b).

During one of the reactions the intermediates wherein $R^1$ is hydrogen may be converted into the corresponding intermediates wherein $R^1$ is other than hydrogen following art-known *N*-alkylating procedures.

From formula (I) it is evident that the compounds of this invention may have several asymmetric carbon atoms in their structure. Each of these chiral centers may be present in a R- and a S-configuration, this R- and S-notation being in correspondence with the rules described by R. S. Cahn, C. Ingold and V. Prelog in Angew. Chem., Int. Ed., Engl., *5*, 385, 511 (1966).

Pure stereochemically isomeric forms of the compounds of formula (I) may be obtained by the application of art-known procedures. Diastereoisomers may be separated by physical separation methods such as selective crystallization and chromatographic techniques, e.g., counter current distribution, and enanatiomers may be separated from each other by the selective crystallization of their diastereomeric salts with optically active acids.

Pure stereochemically isomeric forms may also be derived from the corresponding pure stereochemically isomeric forms of the appropriate starting materials, provided that the reaction occurs stereospecifically.

It is evident that the cis and trans diastereomeric racemates may be further resolved into their optical isomers, cis(+), cis(−), trans(+) and trans(−) by the application of methodologies known to those skilled in the art.

Stereochemically isomeric forms of the compounds of formula (I) are naturally intended to be embraced within the scope of the invention.

The compounds of formula (I), their pharmaceutically acceptable acid-addition salts and stereochemically isomeric forms possess useful pharmacological properties. For example, they show useful anti-hypoxic and anti-anoxic properties, which activity is clearly evidenced by the experimental data obtained in e.g. the "KCN Test in Rats".

In view of their anti-anoxic or anti-hypoxic activity, the compounds of formula (I), their acid addition salts and/or their possible stereoisomers are very useful in the treatment of warm-blooded animals

suffering from anoxia or hypoxia, for example, in such situations where the oxygen supply is reduced or blocked, such as shock, cardiac arrest and severe blood losses.

In view of their anti-anoxic and anti-hypoxic activity the subject compounds may be formulated into various pharmaceutical forms for administration purposes. To prepare the pharmaceutical compositions of this invention, an effective amount of the particular compound, in base or acid-addition salt form, as the active ingredient is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions: or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed.

Acid addition salts of (I) due to their increased water solubility over the corresponding base form, are obviously more suitable in the preparation of aqueous compositions.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and segregated multiples thereof.

Although the amounts to be administered to subjects may vary within rather large limits, daily doses varying of from 0.1 mg/kg body weight to 40 mg/kg body weight are preferred.

In the following examples unless otherwise stated all parts therein are by weight.

## EXPERIMENTAL PART

A) Preparation of Intermediates

### Example 1

A mixture of 100 parts of ethyl 4-oxo-1-piperidinecarboxylate, 42 parts of 2-propanamine, 2 parts of a solution of thiophene in methanol 4% and 400 parts of methanol was hydrogenated at normal pressure and at room temperature with 5 parts of palladium-on-charcoal catalyst 10%. After the calculated amount of hydrogen was taken up, the catalyst was filtered off and the filtrate was evaporated, yielding 118 parts (94%) of ethyl 4-[(1-methylethyl)amino]-1-piperidinecarboxylate (1).

In a similar manner there were also prepared:

ethyl 3-(methylamino)-8-azabicyclo[3,2,1]octane-8-carboxylate (2);

ethyl 3-(methylamino)-1-piperidinecarboxylate (3);

*cis*-ethyl 3-(phenylmethoxy)-4-[(phenylmethyl)amino]-1-piperidinecarboxylate (4);

ethyl 4-(butylamino)-1-piperidinecarboxylate (5);

*cis*-ethyl 3-methoxy-4-(methylamino)-1-piperidinecarboxylate monohydrochloride; mp. 169.1°C (6);

*cis*-ethyl 4-(methylamino)-3-(phenylmethoxy)-1-piperidinecarboxylate monohydrochloride; mp. 181.5°C (7);

ethyl *(cis+trans)*-3-methyl-4-(methylamino)-1-piperidinecarboxylate (8); and

ethyl 3-(methylamino)-1-pyrrolidinecarboxylate (9).

### Example 2

A mixture of 150 parts of *cis*-ethyl 4-(methylamino)-3-(phenylmethoxy)-1-piperidinecarboxylate and 560 parts of methanol was hydrogenated at normal pressure and at 50°C with 10 parts of palladium-on-charcoal catalyst 10%. After the calculated amount of hydrogen was taken up, the catalyst was filtered off and the filtrate was evaporated, yielding 101 parts (95%) of ethyl *cis*-3-hydroxy-4-(methylamino)-1-piperidinecarboxylate (10).

In a similar manner there was also prepared:

*cis*-ethyl 4-amino-3-hydroxy-1-piperidinecarboxylate; bp. 175—185°C at 0.4 mm pressure (11).

### Example 3

A mixture of 8.6 parts of sodium hydroxide and 120 parts of water was stirred at a temperature below 10°C and there were added successively 17 parts of carbon disulfide and 35 parts of ethyl 3-amino-1-pyrrolidinecarboxylate. Stirring was continued for 3 hours. Then there were added dropwise 23.5 parts of ethyl carbonochloridate. Upon completion, stirring was continued for 2 hours at 60°C. The reaction mixture

was extracted with methylbenzene. The extract was dried, filtered and evaporated, yielding 55 parts (100%) of ethyl 3-isothiocyanato-1-pyrrolidinecarboxylate (12).

In a similar manner there was also prepared: ethyl 4-isothiocyanato-1-piperidinecarboxylate (13).

Example 4

A mixture of 107 parts of ethyl 4-isothiocyanato-1-piperidine-carboxylate, 53.5 parts of *N*-methylbenzenamine, 1 part of *N,N*-dimethyl-4-pyridinamine and 450 parts of methylbenzene was stirred and refluxed overnight. After cooling, the reaction mixture was evaporated. The residue was crystallized from 2,2'-oxybispropane. The product was filtered off and dried, yielding 68 parts (42%) of ethyl 4-[[(methylphenylamino)thioxomethyl]aminho]-1-pieridinecarboxylate; mp. 95.2°C (14).

In a similar manner there were also prepared: ethyl 4-[(phenylamino)thioxomethylamino]-1-piperidinecarboxylate (15); and

ethyl 4-[[(2-hydroxyphenyl)aminothioxomethyl]amino]-1-piperidinecarboxylate; mp. 130°C (16).

Example 5

To a stirred solution of 91 parts of ethyl 4-(methylamino)-1-piperidinecarboxylate in 420 parts of 2,2'-oxybispropane were added dropwise 75 parts of 1-fluoro-3-isothiocyanatobenzene. Upon completion, stirring was continued for 1 hour. The precipitated product was filtered off and dried, yielding 150 parts (90%) of ethyl 4-[[(3-fluorophenyl)aminothioxomethyl]methylamino]-1-piperidinecarboxylate; mp. 175.2°C (17).

In a similar manner there were also prepared:

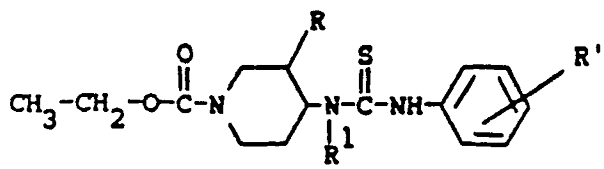

base

| No. | $R^1$ | R' | R | isomerism | mp.(°C) |
|---|---|---|---|---|---|
| 18 | H | H | OH | cis | 191.1 |
| 19 | $CH_3$ | H | H | - | 166.0 |
| 20 | $C_2H_5$ | H | H | - | oil |
| 21 | $n.C_4H_9$ | H | H | - | - |
| 22 | $CH_3$ | 4-$CH_3$ | H | - | - |
| 23 | $C_6H_5CH_2$ | H | H | - | - |
| 24 | $CH_3$ | 4-F | H | - | 178.6 |
| 25 | $CH_3$ | H | $OCH_3$ | cis | 166.0 |
| 26 | $CH_3$ | 3-OH | H | - | - |
| 27 | $CH_3$ | H | $CH_3$ | cis+trans | - |
| 28 | $i.C_3H_7$ | H | H | - | - |
| 29 | $CH_3$ | 3-$CH_3O$ | H | - | - |
| 30 | $CH_3$ | 3-Cl | H | - | - |
| 31 | $CH_3$ | 4-OH | H | - | 200.0 |
| 32 | $CH_3$ | 4-Cl | H | - | 193.1 |
| 33 | $CH_3$ | 4-$CH_3O$ | H | - | - |
| 34 | $CH_3$ | H | OH | cis | - |

In a similar manner there were also prepared:

ethyl 3-[methyl[(phenylamino)thioxomethyl]amino]-8-azabicyclo[3.2.1]octane-8-carboxylate; mp. 143.9°C (35);

ethyl 3-[methyl[(phenylamino)thioxomethyl]amino]-1-piperidinecarboxylate (36); and

ethyl 3-[methyl[(phenylamino)thioxomethyl]amino]-1-pyrrolidinecarboxylate; mp. 145.4°C (37).

Example 6

A mixture of 55 parts of ethyl 3-isothiocyanato-1-pyrrolidinecarboxylate, 23.6 parts of 2-aminophenol and 320 parts of acetonitrile was stirred and refluxed for 4 hours. Then there were added 93 parts of mercury(II) oxide and 0.5 parts of sulfur while stirring vigorously. The reaction mixture was stirred and refluxed overnight. After cooling, the mixture was filtered over diatomaceous earth and the filtrate was evaporated. The residue was purified by column chromatography over silica gel using a mixture of trichloromethane and methanol (99:1 by volume) as eluent. The pure fractions were collected and the eluent was evaporated, yielding 38 parts (64%) of ethyl 3-[(2-benzoxazolyl)amino]-1-pyrrolidinecarboxylate as a residue (38).

In a similar manner there were also prepared:

ethyl 4-(2-benzoxazolylamino)-1-piperidinecarboxylate monohydrobromide (39).

ethyl 4-[(oxazolo[4,5-b]pyridin-2-yl)amino]-1-piperidinecarboxylate monohydrobromide; mp. 203.0°C (40).

In a similar manner there is also prepared:

ethyl 4-[(oxazolo[4,5-d]pyridin-2-yl)amino]-1-piperidinecarboxylate (41).

Example 7

To a stirred mixture of 196 parts of ethyl 4-[ethyl[(phenylamino)thioxomethyl]amino]-1-piperidinecarboxylate and 1600 parts of tetrachloromethane were added dropwise 89 parts of bromine at room temperature. Upon completion, stirring was continued first for 30 minutes at room temperature and further for 4 hours at reflux. The reaction mixture was cooled and the tetrachloromethane was decanted, yielding 194 parts (100%) of ethyl 4-[(2-benzothiazolyl)ethylamino]-1-piperidinecarboxylate as an oily residue (42).

In a similar manner there were also prepared:

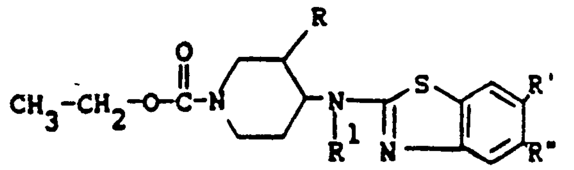

| No. | $R^1$ | R' | R | R" | isomerism | salt/base | mp.(°C) |
|---|---|---|---|---|---|---|---|
| 43 | H | H | H | H | - | HBr | - |
| 44 | $CH_3$ | H | H | H | - | HBr | - |
| 45 | $n.C_4H_9$ | H | H | H | - | HBr | oil |
| 46 | $CH_3$ | $CH_3$ | H | H | - | base | - |
| 47 | $C_6H_5CH_2$ | H | H | H | - | base | - |
| 48 | $CH_3$ | H | $OCH_3$ | H | cis | base | - |
| 49 | $CH_3$ | H | H | F | - | base | oil |
| 50 | $CH_3$ | H | H | OH | - | HBr | - |
| 51 | $CH_3$ | H | $CH_3$ | H | cis+trans | HBr | - |
| 52 | $i.C_3H_7$ | H | H | H | - | HBr | - |
| 53 | $CH_3$ | H | H | $OCH_3$ | - | base | - |
| 54 | $CH_3$ | H | H | Cl | - | HBr | - |
| 55 | $CH_3$ | OH | H | H | - | HBr | - |
| 56 | $CH_3$ | Cl | H | H | - | HBr | - |
| 57 | $CH_3$ | H | OH | H | cis | base | - |
| 58 | H | H | OH | H | cis | base | - |

In a similar manner there were also prepared:
ethyl 3-[(2-benzothiazolyl)methylamino]-8-azabicyclo[3.2.1]octane-8-carboxylate (59);
ethyl 3-[(2-benzothiazolyl)methylamino]-1-piperidinecarboxylate (60);
ethyl 3-[(2-benzothiazolyl)methylamino]-1-pyrrolidinecarboxylate monohydrobromide (61); and
ethyl 4-[(3-methyl-2(3*H*)-benzothiazolyliden)amino-1-piperidinecarboxylate (62).

Example 8

To a stirred mixture of 103.1 parts of ethyl 4-[[[(3-chlorophenyl)amino]thioxomethyl]methylamino]-1-piperidinecarboxylate and 960 parts of tetrachloromethane were added dropwise 45.4 parts of bromine at about 20°C. Upon completion, stirring was continued first for 45 minutes at room temperature and further for 35 hours at reflux. The reaction mixture was cooled and the tetrachloromethane was decanted, yielding 126 parts (100%) of ethyl 4-[(7-chloro-2-benzothiazolyl)methylamino]-1-piperidinecarboxylate monohydrobromide as a residue (63).

Example 9

To a stirred mixture of 124 parts of ethyl 4-[[[(4-methoxyphenyl)amino]thioxomethyl]methylamino]-1-piperidinecarboxylate and 1500 parts of tetrachloromethane were added dropwise slowly 112.6 parts of bromine at room temperature. Upon completion, stirring was continued first for 30 minutes at room temperature and then for 3 hours at reflux temperature. After cooling, tetrachloromethaane was decanted,

yielding 110 parts (73%) of ethyl 4-[(5-bromo-6-methoxy-2-benzothiazolyl)methylamino]-1-piperidinecarboxylate as a residue (64).

Example 10

A mixture of 51 parts of ethyl 4-isothiocyanato-1-piperidinecarboxylate, 30 parts of 2-chloro-3-pyridinamine and 240 parts of ethanol was stirred and refluxed for 24 hours. The reaction mixture was cooled to room temperature. Upon the addition of 70 parts of 2,2'-oxybispropane, the product was allowed to crystallize. It was filtered off and dried, yielding 40 parts (50%) of ethyl 4-(thiazolo[5,4-b]pyridin-2-ylamino)-1-piperidinecarboxylate monohydrochloride (65).

Example 11

A mixture of 8.5 parts of ethyl 4-amino-1-piperidinecarboxylate and 2.6 parts of 2-(methylsulfonyl)thiazolo[4,5-c]pyridine was molten together for 1 hour at 120°C. After cooling, the mixture was taken up in trichloromethane. The whole was washed with water and sodium hydroxide, dried, filtered and evaporated. The residue was purified by column chromatography over silica gel using a mixture of trichloromethane and methanol (99:1 by volume) as eluent. The pure fractions were collected and the eluent was evaporated. The residue was stirred in warm 2,2'-oxybispropane. The product was filtered off and dried, yielding 2.8 parts (76%) of ethyl 4-[(thiazolo[4,5-c]-pyridin-2-yl)amino]-1-piperidinecarboxylate; mp. 213°C (66).

Example 12

A mixture of 20 parts of ethyl 4-[(2-benzothiazolyl)methylamino]-1-piperidinecarboxylate monohydrobromide and 300 parts of hydrobromic acid solution 48% in water was stirred and refluxed for 2 hours. The reaction mixture was evaporated and the residue was crystallized from methanol, yielding 17 parts (85%) of *N*-methyl-*N*-(4-piperidinyl)-2-benzothiazolamine dihydrobromide; mp. + 260°C (67).

A solution of 427 parts of *N*-methyl-*N*-(4-piperidinyl)-2-benzothiazolamine dihydrobromide monohydrate in 1500 parts of water was treated with a sodium hydroxide solution 50%. The product was extracted twice with trichloromethane. The combined organic layers were washed with water, dried, filtered and evaporated. The residue was crystallized from 490 parts of 2,2'-oxybispropane. The product was filtered off and dried, yielding 248 parts (100%) of *N*-methyl-*N*-(4-piperidinyl)-2-benzothiazolamine; mp. 86.9°C (68).

In a similar manner there were also prepared:

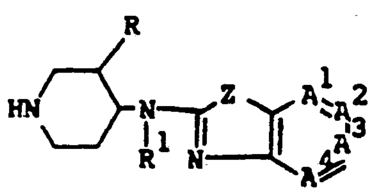

| No. | R | $R^1$ | $A^1=A^2-A^3=A^4$ | Z | isomerism | salt/base | mp.(°C) |
|---|---|---|---|---|---|---|---|
| 69 | H | H | CH=CH-CH=CH | S | - | 2HBr | 260 |
| 70 | OH | H | CH=CH-CH=CH | S | cis | $2HBr.H_2O$ | 268-300 |
| 71 | H | H | N=CH-CH=CH | S | - | 3HBr | 286.9 |
| 72 | H | $CH_3$ | N=CH-CH=CH | S | - | base | 144 |
| 73 | H | H | CH=CH-CH=CH | O | - | 2HBr | 263 |
| 74 | H | $CH_3$ | CH=CH-CH=CH | O | - | $2HBr.1/2H_2O$ | 261.2 |
| 75 | H | $CH_3$ | CH=CH-CH=CH | O | - | base | 130.3 |

| | | | | | | | |
|---|---|---|---|---|---|---|---|
| 76 | H | $C_2H_5$ | CH=CH-CH=CH | S | - | * | 191.7 |
| 77 | H | n.$C_4H_9$ | CH=CH-CH=CH | S | - | ** | 160.0 |
| 78 | H | $CH_3$ | CH=C-CH=CH<br>\|<br>$CH_3$ | S | - | base | 74.3 |
| 79 | H | $C_6H_5CH_2$ | CH=CH-CH=CH | S | - | * | 206.4 |
| 80 | $OCH_3$ | $CH_3$ | CH=CH-CH=CH | S | cis | $(COOH)_2$ | 206.4 |
| 81 | H | $CH_3$ | CH=CH-CF=CH | S | - | 2HCl.$H_2O$ | 266.4 |
| 82 | H | $CH_3$ | CH=CF-CH=CH | S | - | base | - |
| 83 | H | $CH_3$ | CH=CH-C=CH<br>\|<br>OH | S | - | 2HBr | - |
| 84 | H | i.$C_3H_7$ | CH=CH-CH=CH | S | - | * | 181 |
| 85 | H | $CH_3$ | CH=CH-C=CH<br>\|<br>$OCH_3$ | S | - | base | - |
| 86 | H | $CH_3$ | CH=CH-C=CH<br>\|<br>Cl | S | - | * | 158 |
| 87 | H | $CH_3$ | C=CH-CH=CH<br>\|<br>Cl | S | - | base | - |
| 88 | H | $CH_3$ | CH=C-CH=CH<br>\|<br>OH | S | - | 2HBr | - |
| 89 | H | $CH_3$ | CH=C-CH=CH<br>\|<br>Cl | S | - | 2HCl.$H_2O$ | 237.8 |
| 90 | H | $CH_3$ | CH=C-CBr=CH<br>\|<br>$OCH_3$ | S | - | base | 137.0 |
| 91 | OH | $CH_3$ | CH=CH-CH=CH | S | cis | base | - |

*: (Z)-2-butenedioate (1:1)
**: (Z)-2-butenedioate (2:3)

In a similar manner there were also prepared:
*N*-(1-methylthiazolo[5,4]pyridin-2(1*H*)-ylidene)-4-piperidinamine (92);
*N*-(2-benzothiazolyl)-*N*-methyl-8-azabicyclo[3.2.1]octan-3-amine (E)-2-butenedioate(1:1); mp. 236.1°C (93);
*N*-methyl-*N*-(3-piperidinyl)-2-benzothiazolamine (94);
*N*-methyl-*N*-(3-pyrrolidinyl)-2-benzothiazolamine ethanedioate(1:1); mp. 157.0°C (95);
*N*-(3-methyl-2(3*H*)-benzothiazolylidene)-4-piperidinamine; mp. 198.5°C (96);
*N*-methyl-*N*-(3-pyrrolidinyl)-2-benzoxazolamine (97);
*N*-(4-piperidinyl)thiazolo[4,5-c]pyridin-2-amine (98); and
*N*-(4-piperidinyl)oxazolo[4,5-b]pyridin-2-amine (99).
In a similar manner there is also prepared:
*N*-(4-piperidinyl)oxazolo(4,5-d]pyridin-2-amine (100).

Example 13

A mixture of 100 parts of ethyl *(cis+trans)*-4-[(2-benzothiazolyl)-methylamino]-3-methyl-1-piperidinecarboxylate monohydrobromide and 600 parts of a hydrobromic acid solution 48% in water was stirred and refluxed for 3 hours. After cooling, the reaction mixture was evaporated. The residue was

crystallized from 2-propanol : two fractions were obtained. The first fraction was taken up in water. The solution was treated with sodium hydroxide. The product was extracted with trichloromethane. The organic layer was washed with water, dried, filtered and evaporated, yielding 30 parts (48%) of (*cis+trans*)-*N*-methyl-*N*-(3-methyl-4-piperidinyl)-2-benzothiazolamine (101) as an oily residue. The second fraction was taken up in water. The solution was treated with sodium hydroxide. The product was extracted with trichloromethane. The organic layer was washed with water, dried, filtered and evaporated. The residue was converted into the hydrobromide salt in methanol. The salt was filtered off and dried, yielding 16.6 parts (16%) of *cis*-*N*-methyl-*N*-(3-methyl-4-piperidinyl)-2-benzothiazolamine dihydrobromide; mp. 266.7°C (102).

Example 14

To a stirred mixture of 38 parts of ethyl 3-[(2-benzoxazolyl)amino]-1-pyrrolidinecarboxylate, 150 parts of dimethyl sulfoxide and 135 parts of benzene were added portionwise 7.25 parts of a sodium hydride dispersion 50% at a temperature below 10°C. After stirring for a while, 21.6 parts of iodomethane were added dropwise at the same temperature. Upon completion, stirring was continued overnight at 50°C. 1000 parts of water were added and the product was extracted with methylbenzene. The extract was washed with water, dried, filtered and evaporated. The residue was converted into the hydrochloride salt in 2-propanol. The salt was filtered off and dried, yielding 40 parts (80%) of ethyl 3-[(2-benzoxazolyl)methylamino]-1-pyrrolidinecarboxylate dihydrochloride (103).

In a similar manner there were also prepared:

ethyl 4-[(2-benzoxazolyl)methylamino]-1-piperidinecarboxylate monohydrobromide; mp. 152.6°C (104);

ethyl 4-(1-methylthiazolo[5,4-b]-pyridin-2(1*H*)-ylidenamino)-1-piperidinecarboxylate (105); and

ethyl 4-[methyl(thiazolo[5,4-b)pyridin-2-yl)amino]-1-piperidinecarboxylate (106).

Example 15

To a stirred mixture of 32.1 parts of 3-fluorophenol, 122 parts of 1,6-dibromohexane and 200 parts of water was dropwise added a solution of 20 parts of sodium hydroxide in 100 parts of water at reflux temperature. Upon completion, stirring was continued for 24 hours at reflux. After cooling, the product was extracted three times with trichloromethane. The combined extracts were dried, filtered and evaporated. The residue was distilled, yielding 40 parts (50%) of 1-[(6-bromohexyl)oxy]-3-fluorobenzene; bp. 45—90°C at 0.05 mm pressure (107).

In a similar manner there were also prepared:

$$Ar—Y—(CH_2)_n—W$$

| No. | Ar | Y | n | W | physical data |
|---|---|---|---|---|---|
| 108 | $4-C_6H_5-C_6H_4$ | O | 3 | Cl | mp. 61.5-63°C |
| 109 | $2-CH_3CO,4F-C_6H_3$ | O | 3 | Cl | bp. 130-147°C (0.2 mm Hg) |
| 110 | $4-CH_3OCO-C_6H_4$ | O | 4 | Cl | bp. 157°C (0.4 mm Hg) |
| 111 | $2F-C_6H_4$ | O | 5 | Br | bp. 110-112°C (0.6 mm Hg) |
| 112 | $4-C_2H_5-C_6H_4$ | O | 5 | Br | bp. 128-134°C (0.2 mm Hg) |
| 113 | $3-CH_3O-C_6H_4$ | O | 5 | Br | bp. 150-154°C(0.4 mm Hg) |
| 114 | 1-naphthalenyl | O | 5 | Br | bp. 165-170°C (0.6 mm Hg) |
| 115 | $3-Cl-C_6H_4$ | O | 5 | Br | bp. 135-137°C (0.7 mm Hg) |

| | | | | | |
|---|---|---|---|---|---|
| 116 | 3-$CH_3CO-C_6H_4$ | O | 3 | Cl | bp. 120°C (0.5 mm Hg) |
| 117 | 3,4,5$(Cl)_3$-$C_6H_2$ | O | 3 | Br | - |
| 118 | 2-$NH_2CO$,4F-$C_6H_2$ | O | 3 | Cl | mp. 108°C |
| 119 | 4-F-$C_6H_4$ | S | 3 | Br | bp. 78-80°C (0.2 mm Hg) |
| 120 | 2,6-$(Br)_2$,4$CH_3$-$C_6H_2$ | O | 3 | Br | bp. 118-123 (0.3 mm Hg) |
| 121 | 1-Br-2-naphthalenyl | O | 3 | Br | - |
| 122 | 3-F-$C_6H_4$ | O | 5 | Br | bp. 120°C (0.1-0.30 mm Hg) |
| 123 | 3-F-$C_6H_4$ | O | 4 | Br | - |
| 124 | 4-$CH_3CO-C_6H_4$ | O | 5 | Br | bp. 165-170°C (0.02 mm Hg) |

And following the same procedures and using the appropriate starting materials there were also prepared:

[(6-bromo-2-naphthalenyloxy)methyl]oxirane (125);

(4-methylphenyl)[4-(oxiranylmethoxy)phenyl]methanone (126):

[(2,6-dimethylphenoxy)methyl]oxirane; bp. 85—90°C at 0.2 mm pressure (127); and

((2-bromo-4-fluorophenoxy)methyl]oxirane; bp. 105°C at 66.5 Pa (128).

Example 16

To a stirred and cooled (temp. ≤0°C) solution of 63 parts of 2-phenylcyclopropanemethanol in 378 parts of 1,1′-oxybisethane were added dropwise 63 parts of phosphor tribromide. The mixture was allowed to reach room temperature while stirring and the whole was further stirred for 30 minutes at room temperature. The reaction mixture was poured onto 90 parts of ice water and the layers were separated. The organic layer was washed successively twice with water and once with a sodium hydrogen carbonate solution, dried, filtered and evaporated. The residue was distilled, yielding 67 parts (74.,5%) of (1-bromo-3-butenyl)benzene; bp. 85—94°C at 0.4 mm pressure (129).

Example 17

A balloon was charged with 32 parts of 1-[(3-chloropropyl)thio]-4-fluorobenzene, 50 parts of hydrogen peroxide and 112.5 parts of glacial acetic acid: exothermic reaction, the temperature rose to about 60°C. The whole was stirred and refluxed for one hour and further cooled while stirring for 3 hours. The cooled reaction mixture was then decomposed with 500 parts of water. The aqueous layer was separated and extracted once with 1,1′-oxybisethane. The combined organic layers were washed with water, dried over sodium sulfate and evaporated. The residue was recrystallized from 2,2′-oxybispropane, yielding, after cooling to −20°C, 18 parts of 1-[(3-chloropropyl)sulfonyl]-4-fluorobenzene (130).

Example 18

To a stirred and refluxing mixture of 56 parts of lithium aluminum hydride in 1000 parts of tetrahydrofuran was added dropwise a solution of 359 parts of 2-(2,5-dimethylphenoxy)propanoic acid in 1000 parts of tetrahydrofuran. Upon completion, stirring was continued at reflux temperature for 1.50 hours. The reaction mixture was cooled and decomposed by the successive additions of 55 parts of water, 45 parts of a 15% sodium hydroxide solution and 190 parts of water and the whole was stirred for 30 minutes. The inorganic material was filtered off after acidification with hydrochloric acid solution. The filtrate was dried, filtered and evaporated. The residue was distilled, yielding 73 parts of 2-[2,5-dimethylphenoxy)-1-propanol; bp. 150°C at 11 mm pressure (131).

Example 19

To a stirred and cooled mixture of 33.25 parts of 2-(phenylmethoxy)-1-propanol in 20 parts of pyridine was added dropwise a solution of 25.2 parts of methanesulfonyl chloride in 8 parts of pyridine at a temperature between 0° and 5°C. Upon completion, stirring was continued for 2 hours at room temperature. The reaction mixture was poured onto a mixture of crushed ice and ice water and the product was extracted three times with trichloromethane. The combined extracts were dried, filtered and evaporated. The oily residue was distilled, yielding 41 parts of 2-(phenylmethoxy)-1-propanol, methanesulfonate ester; bp. 151—152°C at 0.03 mm pressure (132).

In a similar manner there were also prepared:

2-(3-methylphenoxy)-1-propanol, methanesulfonate ester; bp. 120°C at 0.0002 mm Hg pressure (133);

2-(2,6-dimethylphenoxy)-1-propanol, methanesulfonate ester; bp. 110°C at 0.0002 mm. pressure (134);
2-(3,5-dimethylphenoxy)-1-propanol, methanesulfonate ester; bp. 115°C at 0.0004 mm. pressure (135);
2-(2,6-dichlorophenoxy)-1-propanol, methanesulfonate ester; bp. 120°C at 0.002 mm. pressure (136);
3,4-dihydro-2*H*-1-benzopyran-2-methanol 4-methylbenzenesulfonate ester; mp. 59.4°C (137).

Example 20

A mixture of 6.72 parts of 4-fluorophenol, 8.4 parts of potassium carbonate and 200 parts of 4-methyl-2-pentanone was distilled azeotropically to dry. 17.1 Parts of 4-(phenylmethoxy)cyclohexyl methanesulfonate were added and the whole was stirred and refluxed over week-end using a water separator. After cooling, water was added and the layers were separated. The organic layer was washed with a sodium hydroxide solution and with water, dried, filtered and evaporated, yielding 17.7 parts (98.3%) of 1-fluoro-4-[[4-(phenylmethoxy)cyclohexyl]oxy]benzene as a residue (138).

A mixture of 17.1 parts of 1-fluoro-4-[[4-(phenylmethoxy)cyclohexyl]oxy]benzene and 160 parts of methanol was hydrogenated at normal pressure and at room temperature with 2 parts of palladium-on-charcoal catalyst 10%. After the calculated amount of hydrogen was taken up, the catalyst was filtered off over Hyflo and the filtrate was evaporated, yielding 8.9 parts (74.3%) of 4-(4-fluorophenoxy)cyclohexanol as a residue (139).

To a stirred and cooled mixture of 8.9 parts of 4-(4-fluorophenoxy)cyclohexanol and 160 parts of 2-propanone were added dropwise 13 parts of a solution of 26.7 parts of chromium trioxide in 42.3 parts of concentrate sulfuric acid diluted with water till a volume of 100 parts at a temperature below 5°C. Upon completion, stirring was continued for 3 hours at room temperature. After the addition of a few parts of methanol, the reaction mixture was poured onto water. The product was extracted with dichloromethane. The extract was dried, filtered and evaporated, yielding 8.4 parts (96.1%) of 4-(4-fluorophenoxy)cyclohexanone as a residue (140).

Example 21

To a stirred and cooled (ice water bath) mixture of 72 parts of 2-ethenyl-3,4-dihydro-2*H*-1-benzopyran, 45 parts of sodium hydrogen carbonate and 2860 parts of dichloromethane were added quickly 102.4 parts of 3-chlorobenzenecarboperoxoic acid at about 10°C. The mixture was allowed to reach room temperature and stirring was continued for 4 days. The reaction mixture was filtered and the filtrate was washed successively with water, a saturate sodium sulfite solution, a 5% sodium hydroxide solution and again with water. The organic phase was dried, filtered and evaporated. the oily residue was purified by column chromatography over silica gel using a mixture of trichloromethane and petroleumether (50:50 by volume) as eluent.

The first fraction (unreacted starting material) was collected and the eluent was evaporated. The residue was taken up in 1300 parts of dichloromethane and 30 parts of 3-chlorobenzenecarboperoxoic acid were added at about 10°C. After stirring for 8 hours at room temperature another 30 parts of 3-chlorobenzenecarboperoxoic acid were added and stirring was continued for 12 hours. The mixture was filtered and the filtrate was washed successively with water, a saturate sodium sulfite solution, a 5% sodium hydroxide solution and again with water. The organic phase was dried, filtered and evaporated. The oily residue was purified by column chromatography over silica gel using a mixture of trichloromethane and petroleum-ether (50:50 by volume) as eluent. The pure fractions were collected and the eluent was evaporated, yielding 14 parts of crude product. The second fraction (A+B) was collected and the eluent was evaporated, yielding 28.0 parts of crude product. The combined crude fractions (resp. 14 and 28.0 parts) were separated by HPLC over silica gel using methylbenzene as eluent. The first fraction (A-isomer) was collected, yielding 12.5 parts of (A)-3,4-dihydro-2-oxiranyl-2*H*-1-benzopyran as a residue (141). The second fraction (B-isomer) was collected and the eluent was evaporated, yielding 14 parts of (B)-3,4-dihydro-2-oxiranyl-2*H*-1-benzopyran as a residue (142).

Example 22

From a stirred mixture of 82 parts of a sodium methoxide solution 30% and 315 parts of *N,N*-dimethylformamide was distilled off 160 parts of the solvent (the temperature rose to 130°C). After cooling to room temperature, there were added first 98 parts of trimethylsulfoxonium iodide and then 300 parts of fimethyl sulfoxide. The whole was stirred first for 30 minutes at room temperature and then for 1 hour at 50°C. The mixture was cooled to room temperature and 59 parts of 1-(4-fluorophenoxy)-2-propanone were added dropwise, during a period of 30 minutes. Upon completion, stirring was continued for 1 hour at room temperature and 2 hours at 50°C. After stirring overnight at room temperature, the reaction mixture was poured onto ice water. The product was extracted with 2,2′-oxybispropane. The organic layer was dried, filtered and evaporated, yielding 54 parts (84%) of 2-[(4-fluorophenoxy)methyl]-2-methyloxirane as a residue (143).

In a similar manner there was also prepared:
($A^-$)-3,4-dihydro-2-oxiranyl-2*H*-1-benzopyran (144).

Example 23

To a stirred solution of 2.7 parts of sodium in 50 parts of 2-methoxyethanol were added 13.2 parts of 4-fluorophenol. The whole was stirred for 15 minutes. A solution of 24.8 parts of (−)-(R)-2,2-dimethyl-1,3-dioxolane-4-methanol methanesulfonate (ester) in 70 parts of 2-methoxyethanol was added dropwise quickly. Stirring was continued for 1.5 hours at reflux temperature. After cooling, the mixture was poured into ice water. The product was extracted with methylbenzene. The extract was dried, filtered and evaporated, yielding 26 parts (97.3%) of (S)-4-[(4-fluorophenoxy)methyl]-2,2-dimethyl-1,3-dioxolane as a residue (145).

A mixture of 26 parts of (S)-4-[(4-fluorophenoxy)methyl]-2,2-dimethyl-1,3-dioxolane, 35 parts of a hydrochloric acid solution 2N and 80 parts of 2-propanone was stirred for 2 hours at reflux temperature. The mixture was stirred overnight at room temperature, diluted with 240 parts of ethanol and evaporated. The residue was dissolved in trichloromethane. The organic layer was washed twice with water, dried, filtered and evaporated. The residue was crystallized from tetrachloromethane. The product was filtered off and dried, yielding 11 parts (50.4%) of (−)-(R)-3-(4-fluorophenoxy)-1,2-propanediol; $[\alpha]_D$ = −10.08°(c = 0.5% in methanol) (146).

To a stirred mixture of 11 parts of (−)-(R)-3-(4-fluorophenoxy)-1,2-propanediol, 23.3 parts of pyridine and 240 parts of trichloromethane were added 12.3 parts of 4-methylbenzenesulfonyl chloride. The whole was stirred overnight at room temperature. It was washed successively with acid water, a sodium carbonate solution and water, dried, filtered and evaporated. The residue was purified by column chromatography over silica gel using a mixture of trichloromethane and methanol (98:2 by volume) was eluent. The pure fractions were collected and the eluent was evaporated, yielding 13.5 parts (67.2%) of (+)-(S)-3-(4-fluorophenoxy)-1,2-propanediol, $o^1$-4-methylbenzenesulfonate; $[\alpha]_D$ = +11.11° (c = 0.5% in triochloromethane) (147).

To a stirred mixture of 13.4 parts of (+)-(S)-3-(4-fluorophenoxy)-1,2-propanediol, $o^1$-4-methylbenzenesulfonate and 45 parts of tetrahydrofuran were added 4.9 parts of kalium 1,1-dimethyl ethoxide. The whole was stirred for 30 minutes at room temperature. It was filtered over Hyflo, washed with tetrahydrofuran and the filtrate was evaporated. The residue was distilled, yielding 3.8 parts (57.3%) of (−)-(R)-[(4-fluorophenoxy)methyl]oxirane; bp. 54°C at 66.5 Pa; $[\alpha]_D$ = − 4.08 (c = 0.5% in trichloromethane) (148).

In a similar manner there was also prepared:

(+)-(S)-[(4-fluorophenoxy)methyl]oxirane; bp. 52—53°C at 66.5 Pa; $[\alpha]_D$ = + 5.27 (c = 0.5% in methanol) $[\alpha]_D$ = + 9.10 (c = 0.5% in dichloromethane) (149).

B) Preparation of Final compounds

Example 24

A mixture of 4.4 parts of 1-chloro-3-(4-chlorobutoxy)benzene, 3.7 parts of *N*-methyl-*N*-(4-piperidinyl)-2-benzothiazolamine, 2.1 parts of sodium carbonate and 90 parts of *N,N*-dimethylformamide was stirred for 24 hours at 60°C. The reaction mixture was cooled and poured onto water. The product was extracted with methylbenzene. The extract was washed with water, dried, filtered and evaporated. The residue was purified by column chromatography over silica gel using a mixture of trichloromethane and methanol (97:3 by volume) as eluent. The pure fractions were collected and the eluent was evaporated. The residue was converted into the (Z)-2-butenedioate salt in 2-propanone. The salt was filtered off and dried, yielding 4 parts (49%) of *N*-[1-[4-(3-chlorophenoxy)butyl]-4-piperidinyl]-*N*-methyl-2-benzothiazolamine (Z)-2-butenedioate (1:1); mp. 138.6°C (1).

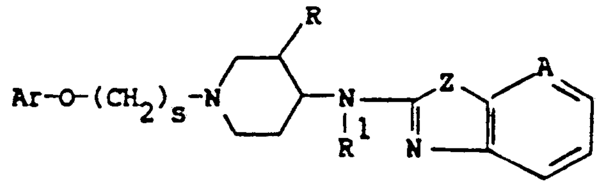

| No. | Ar | s | R | $R^1$ | Z | A | base salt | mp in °C °C |
|---|---|---|---|---|---|---|---|---|
| 2 | $C_6H_5$ | 3 | H | H | O | CH | base | 123.2 |
| 3 | $C_6H_5$ | 3 | H | $CH_3$ | O | CH | base | 95.1 |
| 4 | $C_6H_5$ | 2 | H | $CH_3$ | O | CH | 1 1/2$HNO_3$ | 126 |
| 5 | $C_6H_5$ | 2 | H | H | O | CH | base | 132 |
| 6 | 4-Cl-$C_6H_4$ | 3 | H | $CH_3$ | S | CH | base | 111.9 |
| 7 | $C_6H_5$ | 3 | H | $CH_3$ | S | CH | base | 97.6 |
| 8 | 4-Cl-$C_6H_4$ | 3 | H | $CH_3$ | O | CH | base | 87.5 |
| 9 | 3-F-$C_6H_4$ | 3 | H | $CH_3$ | S | CH | base | 92.6 |
| 10 | $C_6H_5$ | 4 | H | $CH_3$ | S | CH | * | 154.4 |
| 11 | 4-F-$C_6H_4$ | 4 | H | $CH_3$ | S | CH | base | 71.0 |

| | | | | | | | | |
|---|---|---|---|---|---|---|---|---|
| 12 | 4-Cl-$C_6H_4$ | 4 | H | $CH_3$ | S | CH | * | 142.7 |
| 13 | $C_6H_5$ | 2 | H | $CH_3$ | S | CH | * | 174.5 |
| 14 | 4-$CH_3$-$C_6H_4$ | 3 | H | $CH_3$ | S | CH | base | 90.0 |
| 15 | 4-Cl-$C_6H_4$ | 4 | H | $CH_3$ | O | CH | base | 96.4 |
| 16 | 4-F-$C_6H_4$ | 4 | H | $CH_3$ | O | CH | base | 88.4 |
| 17 | 4-Cl-$C_6H_4$ | 2 | H | $CH_3$ | S | CH | base | 89.2 |
| 18 | 2-F-$C_6H_4$ | 3 | H | $CH_3$ | S | CH | base | 129.3 |
| 19 | 4-$CH_3O$-$C_6H_4$ | 3 | H | $CH_3$ | O | CH | base | 79.3 |
| 20 | 4-$CH_3$-$C_6H_4$ | 3 | H | $CH_3$ | O | CH | * | 200.2 |
| 21 | 4-i.$C_3H_7$-$C_6H_4$ | 3 | H | $CH_3$ | S | CH | * | 180.4 |
| 22 | 2,4-$(Cl)_2$-$C_6H_3$ | 3 | H | $CH_3$ | S | CH | * | 179.3 |
| 23 | 4-Br-$C_6H_4$ | 3 | H | $CH_3$ | S | CH | * | 167.2 |
| 24 | 4-$CH_3O$-$C_6H_4$ | 3 | H | $CH_3$ | S | CH | base | 146.5 |
| 25 | 1-naphthalenyl | 3 | H | $CH_3$ | S | CH | * | 154.9 |
| 26 | 4-$CH_3O$-$C_6H_4$ | 3 | H | $CH_3$ | S | CH | base | 156.0 |
| 27 | 4-$CH_3$-$C_6H_4$ | 2 | H | $CH_3$ | S | CH | * | 172.3 |
| 28 | 4-$CH_3$-$C_6H_4$ | 4 | H | $CH_3$ | S | CH | * | 155.5 |
| 29 | 4-Cl,2-$CH_3$-$C_6H_4$ | 3 | H | $CH_3$ | S | CH | base | 106.8 |
| 30 | 2-Cl-$C_6H_4$ | 3 | H | $CH_3$ | S | CH | base | 111.4 |
| 31 | 4-$CH_3O$-$C_6H_4$ | 4 | H | $CH_3$ | S | CH | base | 89.4 |
| 32 | 4-F-$C_6H_4$ | 5 | H | $CH_3$ | S | CH | * | 143.5 |
| 33 | 4-$C_6H_5$-$C_6H_4$ | 3 | H | $CH_3$ | S | CH | base | 126.6 |
| 34 | 2,6-$Cl_2$-$C_6H_3$ | 3 | H | $CH_3$ | S | CH | base | 116.9 |
| 35 | 3,4,5-$Cl_3$-$C_6H_2$ | 3 | H | $CH_3$ | S | CH | * | 181.3 |
| 36 | $C_6H_5$ | 6 | H | $CH_3$ | S | CH | ** | 127.6 |
| 37 | 2-$NH_2CO$,4-F-$C_6H_3$ | 3 | H | $CH_3$ | S | CH | base | 146.4 |
| 38 | $C_6H_5$ | 4 | H | $CH_3$ | O | CH | base | 71.8 |
| 39 | 2,6-$(CH_3)_2C_6H_3$ | 3 | H | $CH_3$ | S | CH | * | 175.6 |
| 40 | 3-$CH_3$-$C_6H_4$ | 3 | H | $CH_3$ | S | CH | base | 86.3 |
| 41 | 2-$CH_3$-$C_6H_4$ | 3 | H | $CH_3$ | S | CH | base | 93.2 |
| 42 | 4-Br-$C_6H_4$ | 4 | H | $CH_3$ | S | CH | ** | 120.5 |
| 43 | 4-$CH_3O$-$C_6H_4$ | 5 | H | $CH_3$ | S | CH | * | 150.7 |
| 44 | 2,4,5-$Br_3$-$C_6H_2$ | 3 | H | $CH_3$ | S | CH | * | 164.2 |
| 45 | 4-$CH_3$-$C_6H_4$ | 4 | H | $CH_3$ | O | CH | base | 85.4 |

| | | | | | | | | |
|---|---|---|---|---|---|---|---|---|
| 46 | 2,4-$Cl_2$-$C_6H_4$ | 3 | H | $CH_3$ | O | CH | base | 100.9 |
| 47 | 4-$CH_3O$-$C_6H_4$ | 4 | H | $CH_3$ | O | CH | base | 69.5 |
| 48 | 3-$CH_3$-$C_6H_4$ | 3 | H | $CH_3$ | O | CH | base | 83.3 |
| 49 | 1-naphthalenyl | 5 | H | $CH_3$ | S | CH | * | 176.5 |
| 50 | 2-$CH_3O$-$C_6H_4$ | 5 | H | $CH_3$ | S | CH | base | 77.4 |
| 51 | 3-$CH_3CO$-$C_6H_4$ | 3 | H | $CH_3$ | S | CH | ** | 128.8 |
| 52 | 4-$CH_3CO$-$C_6H_4$ | 4 | H | $CH_3$ | S | CH | * | 144.3 |
| 53 | 2-Br,4-$CH_3$-$C_6H_3$ | 3 | H | $CH_3$ | S | CH | base | 116.3 |
| 54 | 1-Br-2-naphthalenyl | 3 | H | $CH_3$ | S | CH | * | 196.1 |
| 55 | $C_6H_5$ | 4 | H | H | S | CH | base | 115.3 |
| 56 | 4-$CH_3$-$C_6H_4$ | 3 | H | H | S | CH | base | 118.5 |
| 57 | 3-F-$C_6H_4$ | 3 | H | H | S | CH | base | 92.5 |
| 58 | 3-Cl-$C_6H_4$ | 4 | H | $CH_3$ | O | CH | base | 98.2 |
| 59 | 2-$CH_3$-$C_6H_4$ | 3 | H | $CH_3$ | O | CH | base | 90.1 |
| 60 | 4-Cl-$C_6H_4$ | 4 | H | n.$C_4H_9$ | S | CH | *** | 158.5 |
| 61 | 4-$CH_3O$-$C_6H_4$ | 4 | H | n.$C_4H_9$ | S | CH | *** | 136.4 |
| 62 | 4-Br-$C_6H_4$ | 4 | H | $CH_3$ | O | CH | base | 101.2 |
| 63 | 4-$CH_3$-$C_6H_4$ | 3 | H | n.$C_4H_9$ | S | CH | *** | 141.2 |
| 64 | $C_6H_5$ | 4 | H | n.$C_4H_9$ | S | CH | *** | 147.6 |
| 65 | 4-F-$C_6H_4$ | 4 | H | n.$C_4H_9$ | S | CH | *** | 144.0 |
| 66 | 3-F-$C_6H_4$ | 3 | H | n.$C_4H_9$ | S | CH | *** | 143.0 |
| 67 | 2-$CH_3$-$C_6H_4$ | 5 | H | $CH_3$ | S | CH | * | 157.3 |
| 68 | 4-t.$C_4H_9$ | 3 | H | $CH_3$ | S | CH | * | 197.0 |
| 69 | 4-Cl-$C_6H_4$ | 3 | H | $CH_3$ | S | CH | base | 111.8 |
| 70 | 4-$CH_3CO$-$C_6H_4$ | 5 | H | $CH_3$ | S | CH | $H_2O$ | 91.6 |
| 71 | 3-$CH_3O$-$C_6H_4$ | 3 | H | $CH_3$ | S | CH | ** | 133.0 |
| 72 | 3-F-$C_6H_4$ | 3 | $CH_3$ | $CH_3$ | S | CH | base cis isomer | 104.4 |
| 73 | 3-$CH_3$-$C_6H_4$ | 3 | $CH_3$ | $CH_3$ | S | CH | base cis isomer | 79.5 |
| 74 | 4-$CH_3O$-$C_6H_4$ | 4 | $CH_3$ | $CH_3$ | S | CH | base cis isomer | 73.4 |
| 75 | $C_6H_5$ | 4 | $CH_3$ | $CH_3$ | S | CH | base cis isomer | 95.7 |

| | | | | | | | | |
|---|---|---|---|---|---|---|---|---|
| 76 | 4-F-$C_6H_4$ | 4 | H | H | S | N | base | 146.5 |
| 77 | 3-F-$C_6H_4$ | 3 | H | H | S | N | base | 155.8 |
| 78 | $C_6H_5$ | 4 | H | H | S | N | base | 137.6 |
| 79 | 4-$CH_3O$-$C_6H_4$ | 4 | H | H | S | N | base | 104.2 |
| 80 | 3-Cl-$C_6H_4$ | 4 | H | H | S | N | base | 110.7 |
| 81 | 3-$CH_3$-$C_6H_4$ | 3 | H | H | S | N | base | 163.9 |
| 82 | $C_6H_5$ | 4 | H | $CH_3$ | S | N | * | 164.3 |
| 83 | 3-F-$C_6H_4$ | 3 | H | $CH_3$ | S | N | * | 144.9 |
| 84 | 3-Cl-$C_6H_4$ | 4 | H | $CH_3$ | S | N | * | 148.3 |

*: (Z)-2-butenedioate (1:1)
**: (Z)-2-butenedioate (1:2)
***: (E)-2-butenedioate (1:1)

In a similar manner there were also prepared:

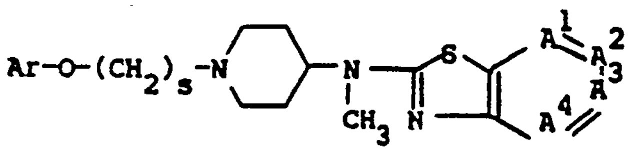

| No. | Ar | s | $A^1=A^2-A^3=A^4$ | base/ salt | mp in °C |
|---|---|---|---|---|---|
| 85 | 3-F-$C_6H_4$ | 3 | CH=CH-C(OH)=CH | base | 151.8 |
| 86 | 3-$CH_3$-$C_6H_4$ | 3 | CH=CH-C(OH)=CH | base | 135.5 |
| 87 | 3-F-$C_6H_4$ | 3 | CH=C(OH)-CH=CH | $2(COOH)_2$ | 176.3 |
| 88 | 3-$CH_3$-$C_6H_4$ | 3 | CH=C(OH)-CH=CH | **** | 212.2 |
| 89 | 3-$CH_3$-$C_6H_4$ | 3 | CH=CCl-CH=CH | base | 89.8 |
| 90 | $C_6H_5$ | 4 | CH=CCl-CH=CH | base | 84.7 |
| 91 | 4-$CH_3O$-$C_6H_4$ | 4 | CH=CCl-CH=CH | base | 94.5 |
| 92 | 3-F-$C_6H_4$ | 3 | CH=CCl-CH=CH | base | 123.2 |

****: (E)-2-butenedioate (2:1)

In a similar manner there were also prepared:

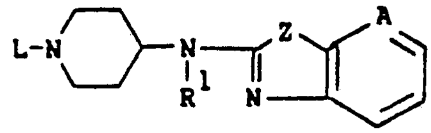

| No. | L | $R^1$ | Z | A | base salt | mp in °C |
|---|---|---|---|---|---|---|
| 93 | n.butyl | H | S | N | base | 170.5 |
| 94 | 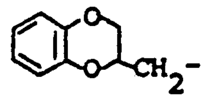 | H | S | N | base | 157.9 |
| 95 | n.butyl | H | O | CH | base | 139.2 |
| 96 | n.hexyl | H | S | N | base | 152 |
| 97 | n.butyl | $CH_3$ | S | N | HCl | 273.6 (dec.) |
| 98 | $C_6H_5(CH_2)_2$ | H | O | CH | base | 130.5 |
| 99 | 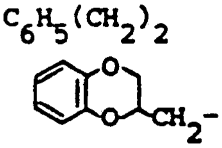 | H | O | CH | base | 144.6 |
| 100 | n.hexyl | $CH_3$ | O | CH | base | 54.8 |
| 101 | $4-CH_3O-C_6H_4-(CH_2)_2-$ | $CH_3$ | O | CH | base | 113.1 |
| 102 | $C_6H_5CH=CH-CH_2$ | $CH_3$ | O | CH | 2HBr | 247.2 |
| 103 | n.butyl | $CH_3$ | O | CH | 2HBr | 230.9 |
| 104 | 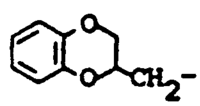 | CH3 | O | CH | 2HBr | 192.8 |
| 105 | $4-CH_3O-C_6H_4-(CH_2)_2-$ | H | O | CH | base | 147.6 |
| 106 | n.hexyl | H | O | CH | $2HNO_3$ | 141.5 |
| 107 | $C_6H_5CH=CH-CH_2-$ | H | O | CH | base | 123.4 |
| 108 | $C_6H_5-(CH_2)_5-$ | $CH_3$ | S | CH | * | 152.5 |
| 109 | n.undecyl | $CH_3$ | S | CH | * | 163.5 |
| 110 | $C_6H_5CH_2O--(CH_2)_3-$ | $CH_3$ | S | CH | * | 135.2 |
| 111 | $C_6H_5CH(OCH_3)-$ | $CH_3$ | S | CH | * | 150.3 |

| | | | | | | |
|---|---|---|---|---|---|---|
| 112 | $HO-(CH_2)_3-$ | $CH_3$ | S | CH | base | 89.2 |
| 113 | $(4-F-C_6H_4)_2CHO$ $-(CH_2)_2-$ | $CH_3$ | S | CH | $(COOH)_2$ | 171.1 |
| 114 | $4-F-C_6H_4-SO_2$ $-(CH_2)_3-$ | $CH_3$ | S | CH | base | 130.4 |

*: (Z)-2-butenedioate (1:1)

And following the same procedures and using the appropriate starting materials there were also prepared:

1-butyl-*N*-(1-methylthiazolo[5,4-b]pyridin-2(1*H*)-ylidene)-4-piperidinamine dihydrochloride.monohydrate; mp. 253.1°C (115);

*N*-(1-methylthiazolo[5,4-b]pyridin-2(1*H*)-ylidene)-1-(2-phenylethyl)-4-piperidinamine dihydrochloride.monohydrate; mp. 251.7°C (116);

*N*-methyl-*N*-[1-(1-phenyl-3-butenyl)-4-piperidinyl]-2-benzothiazolamine (Z)-2-butenedioate (1:2) monohydrate; mp. 101.7°C (117);

*N*-[1-[4-(4-fluorophenoxy)butyl]-3-pyrrolidinyl]-*N*-methyl-2-benzothiazolamine dihydrochloride.monohydrate; mp. 152.5°C (118);

*N*-[1-[4-(3-chlorophenoxy)butyl]-3-pyrrolidinyl]-*N*-methyl-2-benzothiazolamine ethanedioate(1:1); mp. 162.7°C (119);

*N*-methyl-*N*-[1-[3-(3-methylphenoxy)propyl]-3-pyrrolidinyl]-2-benzothiazolamine ethanedioate(1:1); mp. 108.4°C (120);

*N*-[1-[3-(3-fluorophenoxy)propyl]-3-pyrrolidinyl]-*N*-methyl-2-benzothiazolamine ethanedioate(1:1); mp. 137.0°C (121); and

*N*-methyl-*N*-[1-(4-phenoxybutyl)-3-pyrrolidinyl]-2-benzothiazolamine ethanedioate(1:1); mp. 150.2°C (122).

Example 25

A mixture of 5.04 parts of 1-(3-bromopropoxy)-4-methylbenzene, 7.54 parts of *N*-ethyl-*N*-(4-piperidinyl)-2-benzothiazolamine (Z)-2-butenedioate (1:1), 5.3 parts of sodium carbonate, 0.1 parts of potassium iodide and 180 parts of *N,N*-dimethylformamide was stirred and heated overnight at 60°C. The reaction mixture was poured onto water and the product was extracted with methylbenzene. The extract was washed three times with water, dried, filtered and evaporated. The residue was crystallized from 2-propanol, yielding 6 parts (75%) of *N*-ethyl-*N*-[1-[3-(4-methylphenoxy)propyl]-4-piperidinyl]-2-benzothiazolamine; mp. 101.0°C (123).

Following the same procedures and, if desired, after converting the reaction product into the desired acid addition salt, there were also prepared:

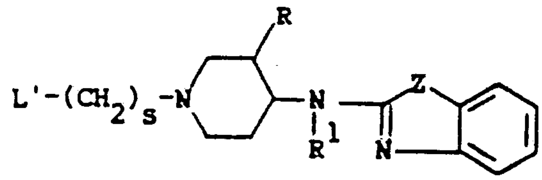

| No. | L' | s | R | $R^1$ | Z | base/salt | mp. in °C |
|---|---|---|---|---|---|---|---|
| 124 | $C_6H_5$ | 2 | H | H | S | base | 118.1 |
| 125 | $C_6H_5$ | 2 | OH | H | S | base (cis-isomer) | 140.1 |
| 126 | 2,3-dihydro-1,4-benzo-dioxin-2-yl | 1 | H | $CH_3$ | S | base | 123.6 |
| 127 | H | 4 | H | $CH_3$ | S | HCl | 232.6 |
| 128 | H | 4 | H | H | S | base | 116.8 |
| 129 | 2,3-dihydro-1,4-benzo-dioxin-2-yl- | 1 | H | H | S | base | 154.7 |
| 130 | $4-F-C_6H_4-S-$ | 3 | H | $CH_3$ | S | * | 158.0 |
| 131 | $4-F-C_6H_4-S-$ | 3 | H | $CH_3$ | O | base | 87.5 |
| 132 | $4-F-C_6H_4-S-$ | 3 | H | $CH_3$ | O | * | 173.4 |
| 133 | $C_6H_5-CH=CH-$ | 1 | H | $CH_3$ | S | base | 101.3 |
| 134 | 1<u>H</u>-benzimidazol-1-yl- | 3 | H | $CH_3$ | S | ** | 168.7 |
| 135 | $4-F-C_6H_4-CH(CN)-$ | 3 | H | $CH_3$ | S | $(COOH)_2$ | 190.1 |
| 136 | $HOCH_2-CH(OH)-$ | 1 | H | $CH_3$ | S | $2HCl.H_2O$ | 218.4 |
| 137 | $C_6H_5-NH-$ | 2 | H | $CH_3$ | S | $3HCl.2H_2O$ | 204.7 |
| 138 | $(C_6H_5)_2CH-O-$ | 2 | H | $CH_3$ | S | $(COOH)_2$ 1/2 $H_2O$ | 190.4 |
| 139 | $C_6H_5CH(CH_3)-O-$ | 2 | H | $CH_3$ | S | * | 165.0 |
| 140 | H | 10 | H | $CH_3$ | S | * | 165.3 |
| 141 | 5-phenyl-3-isoxazolyl | 2 | H | $CH_3$ | S | * | 183.0 |
| 142 | HO- | 2 | H | $CH_3$ | S | * | 175.0 |
| 143 | $4-F-C_6H_4CH(C_2H_5)-$ | 1 | H | $CH_3$ | S | * | 180.5 |
| 144 | $4-F-C_6H_4CH(OH)-$ | 3 | H | $CH_3$ | S | ** | 145.5 |
| 145 | $C_6H_5-CH=CH$ | 1 | H | $C_2H_5$ | S | $(COOH)_2$ | 199.6 |

In a similar manner there were also prepared:

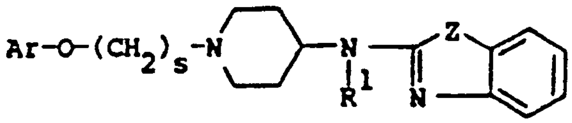

| No. | Ar | s | $R^1$ | Z | base/salt | mp. in °C |
|---|---|---|---|---|---|---|
| 146 | $4-F-C_6H_4$ | 3 | $CH_3$ | S | base | 97.7 |
| 147 | $4-F-C_6H_4$ | 3 | $CH_3$ | O | base | 113.1 |
| 148 | 2-naphthalenyl | 3 | $CH_3$ | S | base | 113.7 |
| 149 | $2,6-Br_2,4-CH_3C_6H_2$ | 3 | $CH_3$ | S | * | 169.7 |
| 150 | $4-C_2H_5-C_6H_4$ | 5 | $CH_3$ | S | * | 163.1 |
| 151 | $4-NO_2-C_6H_4$ | 3 | $CH_3$ | S | base | 138.9 |
| 152 | 2-naphthalenyl | 5 | $CH_3$ | S | * | 193.9 |
| 153 | $3-CF_3-C_6H_4$ | 3 | $CH_3$ | S | * | 149.4 |
| 154 | $3-CH_3O-C_6H_4$ | 4 | $CH_3$ | S | * | 125.8 |
| 155 | $2-F-C_6H_4$ | 5 | $CH_3$ | S | * | 165.8 |
| 156 | $4-C_2H_5-C_6H_4$ | 3 | $CH_3$ | S | * | 176.6 |
| 157 | $3-CH_3O-C_6H_4$ | 5 | $CH_3$ | S | * | 157.3 |
| 158 | $2,5-Cl_2-C_6H_3$ | 3 | $CH_3$ | S | * | 171.7 |
| 159 | $2-CH_3CO-4-F-C_6H_3$ | 3 | $CH_3$ | S | * | 144.7 |
| 160 | $3-F-C_6H_4$ | 5 | $CH_3$ | S | * | 186.7 |
| 161 | $4-CH_3O-C_6H_4$ | 4 | H | S | base | 95.4 |
| 162 | $4-F-C_6H_4$ | 4 | H | S | base | 125.8 |
| 163 | $4-Cl-C_6H_4$ | 4 | H | S | base | 149.8 |
| 164 | $4-Cl-C_6H_4$ | 4 | $C_2H_5$ | S | * | 136.5 |
| 165 | $4-CH_3O-C_6H_4$ | 4 | $C_2H_5$ | S | * | 125.1 |
| 166 | $4-F-C_6H_4$ | 4 | $C_2H_5$ | S | $(COOH)_2$ | 163.9 |
| 167 | $3-F-C_6H_4$ | 6 | $CH_3$ | S | *** | 155.9 |

*: (Z)-2-butenedioate (1:1)
**: (Z)-2-butenedioate (1:2)
***: (E)-2-butenedioate (1:1)

In a similar manner there were also prepared:

*N*-[1-[3-(4-fluorophenoxy)-2-methylpropyl]-4-piperidinyl]-*N*-methyl-2-benzothiazolamine (Z)-2-butenedioate(1:1); mp. 143.6°C (168);

5-methoxy-*N*-[1-[4-(4-methoxyphenoxy)butyl]-4-piperidinyl]-*N*-methyl-2-benzothiazolamine ethanedioate(1:1); mp. 98.2°C (169);

*N*-(3-methyl-2(3*H*)-benzothiazolylidene)-1-[3-(3-methylphenoxy)propyl]-4-piperidinamine (Z)-2-butenedioate(1:2); mp. 176.0°C (170);

*N*-(3-methyl-2(3*H*)-benzothiazolylidene)-1-(4-phenoxybutyl)-4-piperidinamine (Z)-2-butenedioate(1:2); mp. 187.6°C (171);

1-[4-(3-chlorophenoxy)butyl]-*N*-(3-methyl-2(2(3*H*)-benzothiazolylidene)-4-piperidinamine (Z)-2-butenedioate(1:2); mp. 164.6°C (172);

1-[4-(4-fluorophenoxy)butyl]-*N*-(3-methyl-2(3*H*)-benzothiazolylidene)-4-piperidinamine (Z)-2-butenedioate(1:2); mp. 172.6°C (173);

1-[4-(4-methoxyphenoxy)butyl]-*N*-(3-methyl-2(3*H*)-benzothiazolylidene)-4-piperidinamine (Z)-2-butenedioate(1:2); mp. 166.6°C (174);

*N*-(3-methyl-2(3*H*)-benzothiazolylidene)-1-(3-phenyl-2-propenyl)-4-piperidinamine (Z)-2-butenedioate(1:2); mp. 179.4°C (175);

5-bromo-*N*-[1-[4-(4-fluorophenoxy)butyl]-4-piperidinyl]-6-methoxy-*N*-methyl-2-benzothiazolamine; mp. 124.6°C (176);

*N*-[1-[3-(3-fluorophenoxy)propyl]-4-piperidinyl]-5-methoxy-*N*-methyl-2-benzothiazolamine (Z)-2-butenedioate(1:1); mp. 126.3°C (177).

Example 26

A mixture of 2.3 parts of [2-(4-methoxyphenyl)ethyl] methanesulfonate, 4 parts of *N*-(4-piperidinyl)-2-benzothiazolamine dihydrobromide, 5.3 parts of sodium carbonate, 0.1 parts of potassium iodide and 90 parts of *N,N*-dimethylformamide was stirred overnight at 70°C. The reaction mixture was poured onto water and the product was extracted with methylbenzene. The extract was washed with water, dried, filtered and evaporated. The residue was crystallized from a mixture of 2-propanol and 2,2'-oxybispropane. The product was filtered off and dried, yielding 1.5 parts (41%) of *N*-[1-[2-(4-methoxyphenyl)ethyl]-4-piperidinyl]-2-benzothiazolamine; mp. 142.5°C (178).

In a similar manner there were also prepared:

*N*-[1-[2-(4-methoxyphenyl)ethyl]-4-piperidinyl]-*N*-methyl-2-benzothiazolamine; mp. 97.6°C (179); and

*N*-[1-[2-(4-methoxyphenyl)ethyl]-4-piperidinyl]thiazolo[5,4-b]-pyridin-2-amine; mp. 158.9°C (180).

Example 27

A mixture of 4.3 parts of 1-(4-chlorobutoxy)-4-methoxybenzene, 6.6 parts of *N*-[phenylmethyl)-*N*-(4-piperidinyl)-2-benzothiazolamine (Z)-2-butenedioate(1:1), 5.3 parts of sodium carbonate, 0.1 parts of potassium iodide and 90 parts of *N,N*-dimethylacetamide was stirred overnight at 90°C. The reaction mixture was poured onto water. The product was extracted with methylbenzene. The extract was washed with water, dried, filtered and evaporated. The residue was converted into the (E)-2-butenedioate salt in ethanol. The salt was filtered off and dried, yielding 5.6 parts (60%) of *N*-[1-[4-(4-methoxyphenoxy)butyl]-4-piperidinyl]-*N*-(phenylmethyl)-2-benzothiazolamine (E)-2-butenedioate(1:1); mp. 178.2°C (181).

In a similar manner there were also prepared:

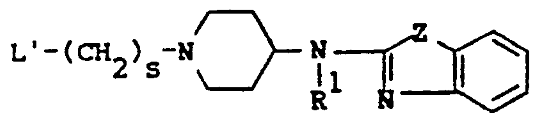

| No. | L' | s | Z | base/salt | mp. in °C |
|---|---|---|---|---|---|
| 182 | H | 12 | S | * | 161.4 |
| 183 | 3-oxo-1,2,4-triazolo-[4,5-a]pyridin-2-yl | 3 | S | * | 185.0 |
| 184 | 4-F-$C_6H_4$-CH(CN)- | 3 | O | 1 1/2$(COOH)_2$ | 139.5 |
| 185 | $(C_6H_5)_2$CH-O- | 2 | O | * | 175.3 |
| 186 | HO | 2 | O | * | 172.6 |
| 187 | $C_6H_5$-NH- | 3 | S | 2 1/2$(COOH)_2$ | 177.1 |
| 188 | 2,3-dihydro-1,4-benzo-dioxin-2-yl | 2 | O | * | 182.1 |
| 189 | $C_6H_5$-NH- | 2 | O | * | 152.6 |
| 190 | 2,3-dihydro-1,4-benzo-dioxin-2-yl | 2 | S | * | 168.6 |

*: (Z)-2-butenedioate (1:1)

In a similar manner there were also prepared:

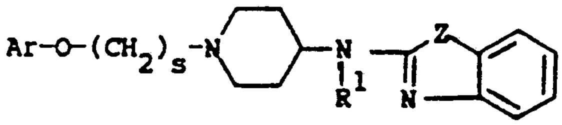

| No. | Ar | s | $R^1$ | Z | base/ salt | mp. in °C |
|---|---|---|---|---|---|---|
| 191 | $3-Cl-C_6H_4-$ | 5 | $CH_3$ | S | * | 177.7 |
| 192 | $4-CH_3-C_6H_4-$ | 5 | $CH_3$ | S | * | 165.1 |
| 193 | $2-F-C_6H_4-$ | 3 | $CH_3$ | O | * | 208.0 |
| 194 | $3,5-Cl_2-C_6H_3-$ | 3 | $CH_3$ | S | * | 178.1 |
| 195 | $3-F-C_6H_4-$ | 3 | $CH_3$ | O | * | 180.6 |
| 196 | $3-F-C_6H_4-$ | 4 | $CH_3$ | S | * | 143.8 |
| 197 | $3-F-C_6H_4-$ | 4 | $CH_3$ | O | * | 158.8 |
| 198 | $C_6H_5-$ | 4 | $C_6H_5CH_2$ | S | * | 129.9 |
| 199 | $4-CH_3-C_6H_4-$ | 3 | $C_6H_5CH_2$ | S | ** | 198.4 |
| 200 | $4-Cl-C_6H_4-$ | 4 | $C_6H_5CH_2$ | S | * | 149.5 |
| 201 | $3-F-C_6H_4-$ | 3 | $C_6H_5CH_2$ | S | * | 145.1 |
| 202 | $4-F-C_6H_4-$ | 4 | $C_6H_5CH_2$ | S | * | 153.6 |
| 203 | $4-NO_2-C_6H_4-$ | 4 | $CH_3$ | S | * | 169.7 |

*: (Z)-2-butenedioate (1:1)
**: (E)-2-butenedioate (1:1)

In a similar manner there was also prepared:
1-[3-(3-fluorophenoxy)propyl]-*N*-(3-methyl-2(3*H*)-benzothiazolylidene)-4-piperidinamine (Z)-2-butenedioate(1:2); mp. 173.4°C (204).

Example 28

A mixture of 5.5 parts of *cis*-*N*-(3-methoxy-4-piperidinyl)-*N*-methyl-2-benzothiazolamine ethanedioate(1:1), 10.6 parts of sodium carbonate, 0.1 parts of potassium iodide and 200 parts of 4-methyl-2-pentanone was stirred and refluxed for 30 minutes. After cooling to 60°C, 4.4 parts of 1-chloro-4-(4-chlorobutoxy)benzene were added. The whole was stirred and refluxed overnight. After cooling, water was added and the layers were separated. The organic layer was dried, filtered and evaporated. The residue was purified by column chromatography over silica gel using a mixture of trichloromethane and methanol (98:2 by volume) as eluent. The pure fractions were collected and the eluent was evaporated. The residue was converted into the ethanedioate salt in 2-propanol. The salt was filtered off and dried, yielding 6 parts (73%) of *cis*-*N*-[1-[4-(4-chlorophenoxy)-butyl]-3-methoxy-4-piperidinyl]-*N*-methyl-2-benzothiazolamine ethanedioate(1:1); mp. 210.4°C (205).

In a similar manner there were also prepared:

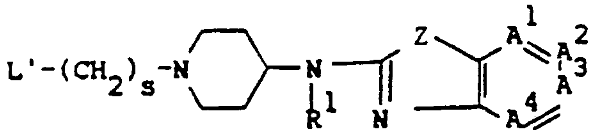

| No. | L' | s | $R^1$ | Z | $A^1=A^2-A^3=A^4$ | base salt | mp in °C |
|---|---|---|---|---|---|---|---|
| 206 | 3-oxo-1,2,4-triazolo-[4,3-a]pyridin-2-yl | 3 | $CH_3$ | S | N=CH-CH=CH | 2HCl 1/2$H_2O$ | 235.5 |
| 207 | cyclohexyloxy | 3 | $CH_3$ | S | CH=CH-CH=CH | * | 160.2 |
| 208 | 4-F-$C_6H_4$-CH=CH- | 2 | $CH_3$ | S | CH=CH-CH=CH | * | 145.0 |
| 209 | 3,4-dihydro-2*H*-1-benzopyran-2-yl | 1 | $CH_3$ | O | CH=CH-CH=CH | $(COOH)_2$ | 212.1 |
| 210 | 3,4-dihydro-2*H*-1-benzopyran-2-yl | 1 | $CH_3$ | S | CH=CH-CH=CH | base | 129.8 |
| 211 | $C_6H_5$-CH=CH- | 1 | H | S | CH=CH-CH=CH | ** | 177.8 |
| 212 | $C_6H_5$-CH=CH- | 1 | n.$C_3H_7$ | S | CH=CH-CH=CH | *** | 165.4 |
| 213 | 2-pyridinyl- | 2 | $CH_3$ | S | CH=CH-CH=CH | ** | 139.5 |
| 214 | $C_6H_5$-CH=CH- | 1 | $CH_3$ | S | CH=C($CH_3$)-CH=CH | * | 177.3 |
| 215 | 3-phenyl-5-isoxazolyl | 2 | $CH_3$ | S | CH=CH-CH=CH | * | 178.8 |
| 216 | 4-F-$C_6H_4$- | 4 | $CH_3$ | S | CH=CH-CH=CH | $(COOH)_2$ | 150.3 |
| 217 | H | 9 | $CH_3$ | S | CH=CH-CH=CH | * | 165.2 |
| 218 | $C_6H_5$-CH=CH | 1 | $CH_3$ | S | CH=CF-CH=CH | * | 174.6 |
| 219 | $C_6H_5$-CH=CH | 1 | $CH_3$ | S | CH=CH-CF=CH | * | 201.7 |
| 220 | $C_6H_5$-CH=CH | 1 | C H CH | S | CH=CH-CH=CH | * | 212.6 |
| 221 | $C_6H_5$-CH=CH | 1 | $(CH_3)$ CH | S | CH=CH-CH=CH | * | 170.5 |
| 222 | $C_6H_5$-CH=CH | 1 | $CH_3$ | S | CH=CCl-CH=CH | base | 130.0 |
| 223 | $C_6H_5$-CH=CH | 1 | $CH_3$ | S | CH=CH-CCl=CH | * | 189.8 |
| 224 | $C_6H_5$-CH=CH | 1 | $CH_3$ | S | CCl=CH-CH=CH | * | 213.5 |

*: (Z)-2-butenedioate (1:1)
**: (Z)-2-butenedioate (1:2)
***: (E)-2-butenedioate (1:1)

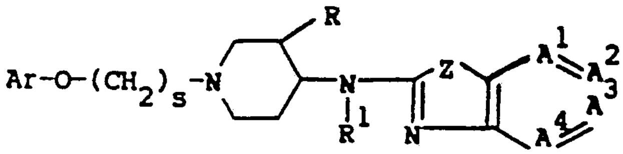

| No. | Ar | s | R | $R^1$ | Z | $A^1=A^2-A^3=A^4$ | cis /trans | base salt | mp in °C |
|---|---|---|---|---|---|---|---|---|---|
| 225 | $2-CH_3OCO-C_6H_4$ | 3 | H | $CH_3$ | S | CH=CH-CH=CH | - | * | 177.0 |
| 226 | $4-F-C_6H_4$ | 6 | H | $CH_3$ | S | CH=CH-CH=CH | - | * | 140.0 |
| 227 | $C_6H_5$ | 5 | H | $CH_3$ | S | CH=CH-CH=CH | - | * | 179.9 |
| 228 | $2-n.C_3H_7C_6H_4$ | 3 | H | $CH_3$ | S | CH=CH-CH=CH | - | * | 164.8 |
| 229 | $3-F-C_6H_4$ | 3 | $OCH_3$ | $CH_3$ | S | CH=CH-CH=CH | cis | *** | 210.5 |
| 230 | $3-CH_3-C_6H_4$ | 3 | H | $CH_3$ | S | CH=CF-CH=CH | - | base | 90.6 |
| 231 | $3-F-C_6H_4$ | 3 | H | $CH_3$ | S | $CH=C(CH_3)-CH=CH$ | - | * | 140.7 |
| 232 | $3-CH_3-C_6H_4$ | 3 | H | $CH_3$ | S | $CH=C(CH_3)-CH=CH$ | - | * | 165.6 |

| | | | | | | | | | |
|---|---|---|---|---|---|---|---|---|---|
| 233 | $C_6H_5$ | 4 | H | $CH_3$ | S | $CH=C-CH=CH$<br>$\vert$<br>$CH_3$ | - | * | 141.4 |
| 234 | $4-CH_3O-C_6H_4$ | 4 | H | $CH_3$ | S | $CH=CF-CH=CH$ | - | base | 90.7 |
| 235 | $3-F-C_6H_4$ | 3 | H | $CH_3$ | S | $CH=CF-CH=CH$ | - | * | 171.1 |
| 236 | $C_6H_5$ | 4 | H | $CH_3$ | S | $CH=CF-CH=CH$ | - | * | 142.7 |
| 237 | $4-CH_3O-C_6H_4$ | 4 | H | $CH_3$ | S | $CH=C-CH=CH$<br>$\vert$<br>$CH_3$ | - | * | 123.8 |
| 238 | $3-F-C_6H_4$ | 3 | H | $i.C_3H_7$ | S | $CH=CH-CH=CH$ | - | * | 160.1 |
| 239 | $4-F-C_6H_4$ | 4 | H | $i.C_3H_7$ | S | $CH=CH-CH=CH$ | - | * | 161.1 |
| 240 | $4-Cl-C_6H_4$ | 4 | H | $i.C_3H_7$ | S | $CH=CH-CH=CH$ | - | * | 149.7 |
| 241 | $4-CH_3-C_6H_4$ | 3 | H | $i.C_3H_7$ | S | $CH=CH-CH=CH$ | - | base | 99.2 |
| 242 | $3-F-C_6H_4$ | 3 | H | $CH_3$ | S | $CH=CH-CCl=CH$ | - | * | 153.8 |
| 243 | $4-CH_3O-C_6H_4$ | 4 | H | $i.C_3H_7$ | S | $CH=CH-CH=CH$ | - | * | 158.7 |
| 244 | $C_6H_5$ | 4 | H | $i.C_3H_7$ | S | $CH=CH-CH=CH$ | - | * | 136.2 |
| 245 | $3-F-C_6H_4$ | 3 | H | $CH_3$ | S | $CCl=CH-CH=CH$ | - | * | 181.2 |
| 246 | $3-CH_3-C_6H_4$ | 3 | H | $CH_3$ | S | $CH=CH-C=CH$<br>$\vert$<br>$OCH_3$ | - | ** | 161.2 |
| 247 | $C_6H_5$ | 4 | $OCH_3$ | $CH_3$ | S | $CH=CH-CH=CH$ | cis | **** | 198.3 |
| 248 | $3-CH_3-C_6H_4$ | 3 | $OCH_3$ | $CH_3$ | S | $CH=CH-CH=CH$ | cis | **** | 187.2 |
| 249 | $4-CH_3O-C_6H_4$ | 4 | $OCH_3$ | $CH_3$ | S | $CH=CH-CH=CH$ | cis | **** | 214.6 |
| 250 | $3-CH_3-C_6H_4$ | 3 | H | $CH_3$ | S | $CH=CH-CCl=CH$ | - | * | 151.0 |
| 251 | $3-CH_3-C_6H_4$ | 3 | H | $CH_3$ | S | $CCl=CH-CH=CH$ | - | * | 178.4 |
| 252 | $4-CH_3O-C_6H_4$ | 4 | H | $CH_3$ | S | $CH=CH-CF=CH$ | - | * | 157.2 |
| 253 | $4-CH_3O-C_6H_4$ | 4 | H | $CH_3$ | S | $CCl=CH-CH=CH$ | - | * | 171.1 |
| 254 | $3-CH_3-C_6H_4$ | 3 | H | $CH_3$ | S | $CH=CH-CF=CH$ | - | * | 163.8 |
| 255 | $C_6H_5$ | 4 | H | $CH_3$ | S | $CH=CH-CF=CH$ | - | * | 185.1 |
| 256 | $3-F-C_6H_4$ | 3 | H | $CH_3$ | S | $CH=CH-CF=CH$ | - | * | 167.9 |
| 257 | $C_6H_5$ | 4 | H | $CH_3$ | S | $CH=CH-CCl=CH$ | - | * | 200.4 |
| 258 | $4-CH_3O-C_6H_4$ | 4 | H | $CH_3$ | S | $CH=CH-CCl=CH$ | - | * | 143.1 |
| 259 | $C_6H_5$ | 4 | H | $CH_3$ | S | $CCl=CH-CH=CH$ | - | * | 176.8 |
| 260 | $4-Cl-C_6H_4$ | 4 | H | H | O | $CH=CH-CH=CH$ | - | 2HCl | 251.2 |
| 261 | $4-F-C_6H_4$ | 4 | H | H | O | $CH=CH-CH=CH$ | - | 2HCl | 239.8 |
| 262 | $4-CH_3-C_6H_4$ | 3 | H | H | O | $CH=CH-CH=CH$ | - | 2HCl | 246.6 |

| | | | | | | | | | |
|---|---|---|---|---|---|---|---|---|---|
| 263 | 4-$CH_3O$-$C_6H_4$ | 4 | H | H | O | CH=CH-CH=CH | - | 2HCl | 237.3 |
| 264 | 3-$CH_3$-$C_6H_4$ | 3 | H | H | O | CH=CH-CH=CH | - | 2HCl. $H_2O$ | 219.2 |
| 265 | 3-Cl-$C_6H_4$ | 4 | H | H | O | CH=CH-CH=CH | - | 2HCl | 217.3 |
| 266 | $C_6H_5$ | 4 | H | H | O | CH=CH-CH=CH | - | 2HCl. 1/2$H_2O$ | 231.7 |
| 267 | 3-F-$C_6H_4$ | 3 | H | H | O | CH=CH-CH=CH | - | 2HCl. $H_2O$ | 224.8 |
| 268 | $C_6H_5$ | 4 | OH | H | S | CH=CH-CH=CH | cis | base | 158.2 |
| 269 | 3-$CH_3$-$C_6H_4$ | 3 | OH | H | S | CH=CH-CH=CH | cis | base | 146.9 |
| 270 | 4-Cl-$C_6H_4$ | 4 | OH | H | S | CH=CH-CH=CH | cis | base | 179.7 |
| 271 | 3-F-$C_6H_4$ | 3 | OH | H | S | CH=CH-CH=CH | cis | base | 165.8 |

*: (Z)-2-butenedioate (1:1)
**: (E)-2-butenedioate (1:1)
***: ethanedioate (1:1) hemihydrate
****: ethanedioate (1:1)

In a similar manner there were also prepared:

*N*-methyl-*N*-[1-[2-(phenylmethoxy)propyl]-4-piperidinyl]-2-benzothiazolamine (Z)-2-butenedioate (1:1); mp. 150.5°C (272);

*N*-methyl-*N*-[1-(2-methyl-3-phenoxypropyl)-4-piperidinyl]-2-benzothiazolamine (Z)-2-butenedioate (1:1); mp. 177.0°C (273);

*N*-methyl-*N*-[1-(2-(3-methylphenoxy)propyl]-4-piperidinyl]-2-benzothiazolamine (Z)-2-butenedioate (1:1); mp. 168.6°C (274);

*N*-[1-(2-(2,6-dimethylphenoxy)propyl]-4-piperidinyl]-*N*-methyl-2-benzothiazolamine (Z)-2-butenedioate (1:1); mp. 130.4°C (275);

*N*-[1-[2-(2,6-dichlorophenoxy)propyl]-4-piperidinyl]-*N*-methyl-2-benzothiazolamine; mp. 108.0°C (276);

*N*-[1-[2-(3,5-dimethylphenoxy)propyl]-4-piperidinyl]-*N*-methyl-2-benzothiazolamine (Z)-2-butenedioate (1:1); mp. 172°C (277);

*N*-(2-benzothiazolyl)-8-[3-(3-fluorophenoxy)propyl]-*N*-methyl-8-azabicyclo[3.2.1]-octan-3-amine (E)-2-butenedioate (1:1); mp. 171.3°C (278);

(E)-*N*-methyl-*N*-[8-(3-phenyl-2-propenyl)-8-azabicyclo[3.2.1]oct-3-yl]-2-benzothiazolamine ethanedioate (1:1); mp. 216.0°C (279);

*N*-(2-benzothiazolyl)-*N*-methyl-8-(4-phenoxybutyl)-8-azabicyclo[3,2,1]-octan-3-amine (E)-2-butenedioate (1:1); mp. 158.0°C (280);

*cis*-*N*-methyl-*N*-[3-methyl-1-(3-phenyl-2-propenyl)-4-piperidinyl]-2-benzothiazolamine (Z)-2-butenedioate (1:1); mp. 173.7°C (281);

*cis*-*N*-[3-methoxy-1-(3-phenyl-2-propenyl)-4-piperidinyl]-*N*-methyl-2-benzothiazolamine ethanedioate (1:1); mp. 230.2°C (282);

*N*-(2-benzothiazolyl)-*N*-methyl-8-[3-(3-methylphenoxy)propyl]-8-azabicyclo[3.2.1]octan-3-amine ethanedioate (1:1); mp. 183.3°C (283);

*N*-methyl-*N*-[1-(4-phenoxybutyl)-3-piperidinyl]-2-benzothiazolamine ethanedioate (1:1); mp. 152.5°C (284);

*N*-[1-[3-(3-fluorophenoxy)propyl]-3-piperidinyl]-*N*-methyl-2-benzothiazolamine ethanedioate (1:1); mp. 164.5°C (285);

*N*-methyl-*N*-[1-(3-phenyl-2-propenyl)-3-piperidinyl]-2-benzothiazolamine ethanedioate (1:1); mp. 202.8°C (286);

*N*-methyl-*N*-[1-[3-(3-methylphenoxy)propyl]-3-piperidinyl]-2-benzothiazolamine ethanedioate (1:1); mp. 151.3°C (287);

*N*-[1-[4-(4-methoxyphenoxy)butyl]-3-piperidinyl]-*N*-methyl-2-benzothiazolamine ethanedioate (1:1); mp. 152.3°C (288);

*N*-[1-[4-(4-fluorophenoxy)butyl]-3-piperidinyl]-*N*-methyl-2-benzothiazolamine ethanedioate (1:1); mp. 163.2°C (289);

*N*-[1-[3-(4-fluorophenoxy)-1-methylpropyl]-4-piperidinyl]-*N*-methyl-2-benzothiazolamine; mp. 115.0°C (290);

*N*-[1-[4-(3-chlorophenoxy)butyl]-3-piperidinyl]-*N*-methyl-2-benzothiazolamine (E)-2-butenedioate (1:1); mp. 138.6°C (291);

*N*-methyl-*N*-[1-(3-phenyl-2-propenyl)-3-pyrrolidinyl]-2-benzothiazolamine 4-methylbenzenesulfonate (1:2); mp. 198.8°C (292); and

*cis*-4-(2-benzothiazolylamino)-1-(3-phenyl-2-propenyl)-3-piperidinol; mp. 198.3°C (293).

Example 29

A mixture of 3 parts of 1-bromo-3-methylbutane, 3.7 parts of *N*-methyl-*N*-(4-piperidinyl)-1-benzothiazolamine, 4.2 parts of potassium carbonate and 80 parts of 2-propanone was stirred and refluxed overnight. The reaction mixture was poured onto water and the product was extracted with methylbenzene. The extract was dried, filtered and evaporated. The residue was converted into the (Z)-2-butenedioate salt in 2-propanol. The salt was filtered off and dried, yielding 5 parts (77%) of *N*-methyl-*N*-[1-(3-metylbutyl)-4-piperidinyl]-2-benzothiazolamine (Z)-2-butenedioate (1:1); mp. 197.2°C (294).

Example 30

A mixture of 3 parts of (3-chloro-1-propenyl)benzene, 2.6 parts of 2-[methyl(4-piperidinyl)amino]-5-benzothiazolol, 3.5 parts of *N,N*-diethylethanamine and 90 parts of *N,N*-dimethylformamide was stirred and heated for 24 hours at 60°C. The reaction mixture was cooled, poured onto water and the product was extracted with methylbenzene. The extract was washed with water, dried, filtered and evaporated. The residue was purified by column chromatography over silica gel using a mixture of trichloromethane and methanol (97:3 by volume) as eluent. The pure fractions were collected and the eluent was evaporated. The residue was converted into the (E)-2-butenedioate salt in ethanol. The salt was filtered off and dried, yielding 1 part (20%) of 2-[methyl[1-(3-phenyl-2-propenyl)-4-piperidinyl]amino]-5-benzothiazolol (E)-2-butenedioate (1:1); mp. 228.4°C (295).

Example 31

To a stirred mixture of 1.1 parts of a sodium hydride dispersion 50% and 45 parts of *N,N*-dimethylformamide were added 3.1 parts of 4-[(2-benzothiazoly)methylamino]-1-piperidinepropanol and the whole was stired and heated for 2 hours at about 60°C. After cooling to about 15°C, 1.65 parts of 4-chloropyridine hydrochloride were added and stirring was continued overnight at 60°C. The reaction mixture was cooled and the solvent was evaporated. The residue was taken up in water and extracted with methylbenzene. The extract was dried; filtered and evaporated. The residue was purified by filtration over silica gel using a mixture of trichloromethane and methanol (98:2 by volume) as eluent. The pure fractions were collected and the eluent was evaporated. The residue was crystallized from 2,2'-oxybispropane, yielding, after drying, 1.5 parts (38%) of *N*-methyl-*N*-[1-[3-(4-pyridinyloxy)propyl]-4-piperidinyl]-2-benzothiazolamine monohydrate; mp. 96.3°C (296).

Example 32

A mixture of 4.75 parts of cyclopentanone, 4.1 parts of *N*-(1-methylethyl)-*N*-(4-piperidinyl)-2-benzothiazolamine and 200 parts of methanol was hydrogenated at normal pressure and at room temperature with 2 parts of palladium-on-charcoal catalyst 10%. After the calculated amount of hydrogen was taken up, the catalyst was filtered off over Hyflo and the filtrate was evaporated. The residue was purified by column chromatography over silica gel using a mixture of trichloromethane and methanol (98.5:1.5 by volume) as eluent. The pure fractions were collected and the eluent was evaporated. The residue was converted into the (Z)-2-butenedioate salt in 2-propanol. The salt was filtered off and dried, yielding 3.9 parts (56.6%) of *N*-(1-cyclopentyl-4-piperidinyl)-*N*-(1-methylethyl)-2-benzothiazolamine (Z)-2-butenedioate (1:1); mp. 178.5°C (297).

In a similar manner there were also prepared:

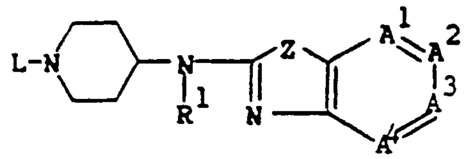

| No. | L | $R^1$ | Z | $A^1=A^2-A^3=A^4$ | base salt | mp in °C |
|---|---|---|---|---|---|---|
| 298 | $CH_3$ | H | S | CH=CH-CH=CH | 2HCl. 1/2$H_2O$ | 283.1 |
| 299 | $CH_3$ | H | S | N=CH-CH=CH | base | 170.6 |
| 300 | $CH_3$ | $CH_3$ | S | N=CH-CH=CH | 2HCl | 266.5 |
| 301 | cyclohexyl | H | O | CH=CH-CH=CH | base | 137.4 |
| 302 | $CH_3$ | H | O | CH=CH-CH=CH | base | 132.1 |
| 303 | $C_2H_5$ | $CH_3$ | O | CH=CH-CH=CH | 2HBr.$H_2O$ | 240.9 |
| 304 | $CH_3$ | $CH_3$ | O | CH=CH-CH=CH | 2$HNO_3$ | 142.9 |
| 305 | i.$C_3H_7$ | $CH_3$ | S | CH=CH-CH=CH | 2HCl.$H_2O$ | 265.9 |
| 306 | cyclohexyl | $CH_3$ | S | CH=CH-CH=CH | 2HCl | 278.8 |
| 307 | cyclopentyl | $CH_3$ | S | CH=CH-CH=CH | 2HCl | 268.8 |
| 308 | cyclopentyl | $CH_3$ | O | CH=CH-CH=CH | 2HCl. 2$H_2O$ | 276.2-280.8 (dec) |
| 309 | cyclohexyl | $CH_3$ | O | CH=CH-CH=CH | 2HCl.$H_2O$ | 300 |
| 310 | cyclopropylmethyl | $CH_3$ | S | CH=CH-CH=CH | 2HCl | 236.4 |
| 311 | cyclopentyl | H | S | CH=CH-CH=CH | base | 172.7 |
| 312 | cyclopentyl | $C_2H_5$ | S | CH=CH-CH=CH | * | 145.1 |
| 313 | cyclopentyl | n.$C_4H_9$ | S | CH=CH-CH=CH | ** | 181.6 |
| 314 | cyclopentyl | $C_6H_5CH_2$ | S | CH=CH-CH=CH | * | 202.1 |
| 315 | cyclopropylmethyl | $CH_3$ | O | CH=CH-CH=CH | * | 184.9 |

*: (Z)-2-butenedioate (1:1)
**: (E)-2-butenedioate (1:1)

In a similar manner there were also prepared:

*cis*-4-[4-[(2-benzothiazolyl)methylamino]-1-piperidinyl]-1-(4-fluorophenyl)cyclohexanecarbonitrile; mp. 184.7°C (316);

(*cis* + *trans*)-*N*-[1-[4-(4-fluorophenoxy)cyclohexyl]-4-piperidinyl]-*N*-methyl-2-benzothiazolamine; mp. 198.4°C (317);

*cis*-4-[4-[(2-benzoxazolyl)methylamino]-1-piperidinyl]-1-(4-fluorophenyl)cyclohexanecarbonitrile; mp. 199.6°C (318); and

*N*-[1-(1*H*-indol-3-ylmethyl)-4-piperidinyl]-*N*-methyl-2-benzothiazolamine; mp. 178.3°C (319).

Example 33

A mixture of 2.5 parts of [(4-fluorophenoxy)methyl]oxirane, 3.7 parts of *N*-methyl-*N*-(4-piperidinyl)-2-benzothiazolamine, 45 parts of methylbenzene and 40 parts of methanol was stirred and refluxed for 24 hours. The reaction mixture was evaporated. The residue was converted into the hydrochloride salt in 2-propanol. The salt was filtered off and dried, yielding 6 parts (85%) of 4-[(2-benzothiazolyl)methylamino]-α-[(4-fluorophenoxy)methyl]-1-piperidineethanol monohydrochloride; mp. 168.4—178.8°C (320).

Following the same procedure and using equivalent amounts of the appropriate starting materials, there were also prepared:

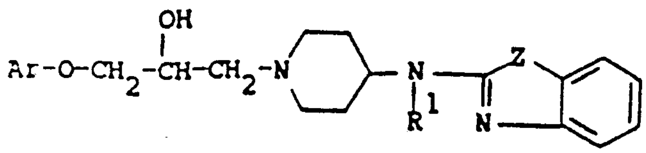

| No. | Ar | $R^1$ | Z | base/ salt | mp. in °C |
|---|---|---|---|---|---|
| 321 | $C_6H_5$ | H | O | base | 119.8 |
| 322 | $C_6H_5$ | $CH_3$ | O | HBr | 231.6 |
| 323 | $4-F-C_6H_4$ | $CH_3$ | O | HCl | 233.5 |
| 324 | $4-Cl-C_6H_4$ | $CH_3$ | O | HCl | 237.5 |
| 325 | $2-C_2H_5O-C_6H_4$ | $CH_3$ | O | 2HCl | 203.3 |
| 326 | 2-propenyloxyphenyl | $CH_3$ | O | HCl | 144.6 |
| 327 | $4-CH_3CONH-C_6H_4$ | $CH_3$ | O | 2HCl | 236.0 |
| 328 | $4-CH_3CONH-C_6H_4$ | $CH_3$ | S | 2HCl | 169.6-182.2 |
| 329 | 1-naphthalenyl | $CH_3$ | S | $(COOH)_2$ | 198.1-199.9 |
| 330 | 2-propenyloxyphenyl | $CH_3$ | S | $(COOH)_2$ | 120.3-129.5 |
| 331 | $4-Cl-C_6H_4$ | $CH_3$ | S | 2HCl | 206.0-225.6 |
| 332 | 1-naphthalenyl | $CH_3$ | O | $(COOH)_2$ | 232.1-233.1 |
| 333 | 2-acetylphenyl | $CH_3$ | O | $(COOH)_2$ | 203.2-203.6 |
| 334 | 2-acetylphenyl | $CH_3$ | S | $(COOH)_2$ | 222.8-223.1 |
| 335 | $2-C_2H_5O-C_6H_4$ | $CH_3$ | S | $(COOH)_2$ $H_2O$ | 112.9-118.6 |
| 336 | $2-Cl-C_6H_4$ | $CH_3$ | S | $(COOH)_2$ $1/2H_2O$ | 180.9-182.8 |
| 337 | $2-Cl-C_6H_4$ | $CH_3$ | O | $(COOH)_2$ $1/2H_2O$ | 172.6-182.1 |
| 338 | $C_6H_5$ | $CH_3$ | S | $(COOH)_2$ | 166.0-169.6 |
| 339 | $2-CN-C_6H_4$ | $CH_3$ | O | $(COOH)_2$ | 173.9-175.6 |
| 340 | $2-CN-C_6H_4$ | $CH_3$ | S | * | 150.5 |
| 341 | $3-Cl-C_6H_4$ | $CH_3$ | S | base | 129.0 |
| 342 | $2,4-Cl_2-C_6H_3$ | $CH_3$ | O | * | 187.7 |
| 343 | $2,4-Cl_2-C_6H_3$ | $CH_3$ | S | * | 167.8 |

| | | | | | |
|---|---|---|---|---|---|
| 344 | 3-$CF_3$-$C_6H_4$ | $CH_3$ | S | 2HCl | 220.9 |
| 345 | 2,3-dihydro-1H-inden-5-yl | $CH_3$ | S | base | 95.9 |
| 346 | 2-Br-$C_6H_4$ | $CH_3$ | S | * | 167.0 |
| 347 | 6-Br-2-napthalenyl | $CH_3$ | S | * | 192.9 |
| 348 | 4-Cl,3,5-$(CH_3)_2C_6H_2$ | $CH_3$ | S | base | 131.7 |
| 349 | 4-(4-methylbenzoyl)phenyl | $CH_3$ | S | base | 174.2 |
| 350 | 2,4,6-$(Cl_2)_3$-$C_6H_2$ | $CH_3$ | S | base | 126.4 |
| 351 | 2,6-$(CH_3)_2$-$C_6H_2$ | $CH_3$ | S | base | 146.8 |
| 352 | 3-$CH_3O$-$C_6H_4$ | $CH_3$ | S | ** | 118.5 |
| 353 | 2-n.$C_3H_7$-CO-$C_6H_4$ | $CH_3$ | S | $(COOH)_2$ | 159.0 |
| 354 | 3-Cl-$C_6H_4$ | $CH_3$ | O | * | 123.7 |
| 355 | 4-Cl-$C_6H_4$ | H | S | base | 164.8 |
| 356 | 4-F-$C_6H_4$ | H | S | base | 162.8 |
| 357 | 4-Cl-$C_6H_4$ | $C_2H_5$ | S | base | 103.1 |
| 358 | 4-F-$C_6H_4$ | $C_2H_5$ | S | *** | 205.7 |
| 359 | 4-Cl,2-$CH_3$-$C_6H_3$ | $CH_3$ | S | base | 115.5 |
| 360 | 2-F-$C_6H_4$ | $CH_3$ | S | base | 100.4 |
| 361 | 2-$CH_3CO$,4-F-$C_6H_3$ | $CH_3$ | S | base | 108.7 |
| 362 | 4-F-$C_6H_4$ | $CH_3$ | S | *** | 169.5 |
| 363 | 4-$CH_3CO$-$C_6H_4$ | $CH_3$ | S | base | 98.9 |
| 364 | 4-F-$C_6H_4$ | i.$C_3H_7$ | S | * | 161.8 |
| 365 | 4-F-$C_6H_4$ | $CH_3$ | S | base | 101.7 |
| 366 | 4-Cl-$C_6H_4$ | i.$C_3H_7$ | S | * | 169.8 |
| 367 | 4-CN-$C_6H_4$ | $CH_3$ | S | base | 122.1 |
| 368 | $C_6H_5$ | $CH_3$ | S | * | 135.6 |
| 369 | 4-F-$C_6H_4$ | $CH_3$ | S | 2HCl | 209.4 |
| 370 | 4-$NO_2$-$C_6H_4$ | $CH_3$ | S | base | 140.1 |
| 371 | 4-F-$C_6H_4$ | H | O | 2HCl | 227.9 |
| 372 | 3-Cl-$C_6H_4$ | H | O | 2HCl | 213.2 |
| 373 | 3,4,5-$(CH_3O)_3C_6H_2$ | $CH_3$ | S | * | 176.9 |
| 374 | 4-$CH_3O$-$C_6H_4$ | $CH_3$ | S | base | 129.6 |
| 375 | 3,5-$(CH_3O)_2$-$C_6H_3$ | $CH_3$ | S | * | 145.2 |
| 376 | 1,3-benzodioxol-5-yl | $CH_3$ | S | *** | 181.7 |

*: (Z)-2-butenedioate (1:1);
**: (Z)-2-butenedioate (1:2);
***: (E)-2-butenedioate (2:1)

In a similar manner there were also prepared:

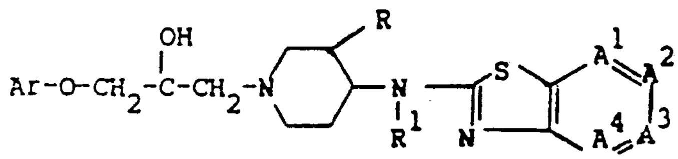

| No. | Ar | R | $R^1$ | $A^1=A^2-A^3=A^4$ | cis/ trans | base salt | mp in °C |
|---|---|---|---|---|---|---|---|
| 377 | $C_6H_5$ | $CH_3O$ | $CH_3$ | CH=CH-CH=CH | cis | * | 163.6 |
| 378 | $4-F-C_6H_4$ | H | $CH_3$ | CH=CF-CH=CH | - | base | 101.5 |
| 379 | $C_6H_5$ | H | $CH_3$ | CH=CF-CH=CH | - | base | 130.6 |
| 380 | $4-F-C_6H_4$ | $CH_3O$ | $CH_3$ | CH=CH-CH=CH | cis | * | 178.9 |
| 381 | $4-F-C_6H_4$ | $CH_3$ | $CH_3$ | CH=CH-CH=CH | cis | * | 150.0 |
| 382 | $C_6H_5$ | $CH_3$ | $CH_3$ | CH=CH-CH=CH | cis | * | 165.1 |
| 383 | $4-F-C_6H_4$ | H | $CH_3$ | CH=CH-CF=CH | - | base | 118.1 |
| 384 | $4-F-C_6H_4$ | H | $CH_3$ | CCl=CH-CH=CH | - | * | 154.0 |
| 385 | $4-F-C_6H_4$ | H | $CH_3$ | CH=CH-CCl=CH | - | * | 144.6 |
| 386 | $4-F-C_6H_4$ | H | H | N=CH-CH=CH | - | base | 168.2 |
| 387 | $C_6H_5$ | H | $CH_3$ | CH=CCl-CH=CH | - | base | 177.4 |
| 388 | $4-F-C_6H_4$ | H | $CH_3$ | CH=CCl-CH=CH | - | base | 172.7 |
| 389 | $4-F-C_6H_4$ | H | $CH_3$ | CH=CH-C(OCH$_3$)=CH | - | ** | 176.1 |
| 390 | $C_6H_5$ | H | $CH_3$ | CCl=CH-CH=CH | - | * | 174.8 |
| 391 | $C_6H_5$ | H | $CH_3$ | CH=CH-CCl=CH | - | * | 171.1 |
| 392 | $4-F-C_6H_4$ | H | $CH_3$ | N=CH-CH=CH | - | base | 103.6 |
| 393 | $3-Cl-C_6H_4$ | H | $CH_3$ | N=CH-CH=CH | - | base | 119.9 |

*: (Z)-2-butenedioate (1:1)
**: (E)-2-butenedioate (1:1)

In a similar manner there were also prepared:

4-[(2-benzothiazolyl)methylamino]-α-(4-methoxyphenyl)-1-piperidineethanol; mp. 168.1°C (394);

4-[(2-benzothiazolyl)methylamino]-α-(4-fluorophenyl)-1-piperidineethanol; mp. 178.5°C (395);

3-[(2-benzothiazolyl)methylamino]-α-(phenoxymethyl)-8-azabicyclo[3.2.1]octane-8-ethanol ethanedioate (1:1); mp. 188.6°C 396);

3-[(2-benzothiazolyl)methylamino]-α-[(4-fluorophenoxy)methyl]-1-piperidineethanol ethanedioate (1:1); mp. 182.7°C (397);

3-[(2-benzothiazolyl)methylamino]-α-[(3-chlorophenoxy)methyl]-1-piperidineethanol ethanedioate (1:1); mp. 172.9°C (398);

3-[(2-benzothiazolyl)methylamino]-α-(phenoxymethyl)-1-piperidineethanol ethanedioate (1:1); mp. 180.2°C (399);

3-[(2-benzothiazolyl)methylamino]-α-[(3-chlorophenoxy)methyl]-1-pyrrolidineethanol ethanedioate (1:1); mp. 158.5°C (400);

α-[(3-chlorophenoxy)methyl]-4-(thiazolo[5,4-b]pyridin-2-ylamino)-1-piperidineethanol; mp. 127.4°C (401);

(A)-4-(2-benzoxazolylamino)-α-(2,3-dihydro-1,4-benzodioxin-2-yl)-1-piperidineethanol dihydrochloride; mp. 229.8°C (402);

(B)-4-(2-benzoxazolylamino)-α-(2,3-dihydro-1,4-benzodioxin-2-yl)-1-piperidineethanol dihydrochloride, hemihydrate; mp. 245.3°C (403);

4-[(2-benzoxazolyl)methylamino]-α-(4-methoxyphenyl)-1-piperidineethanol dihydrochloride; mp. 231.1°C (404);

3-[(2-benzothiazolyl)methylamino]-α-[(4-fluorophenoxy)methyl]-1-pyrrolidineethanol 4-methylbenzenesulfonate (1:2); mp. 199.8°C (405);

α-[(4-fluorophenoxy)methyl]-4-[(1-methylthiazolo[5,4-b]pyridin-2-yliden)amino]-1-piperidineethanol; mp. 150.2°C (406);

4-[(3-methyl-2(3*H*)-benzothiazolyliden)amino]-α-(phenoxymethyl)-1-piperidineethanol (Z)-2-butenedioate(1:2); mp. 152.8°C (407);

4-[(2-benzothiazolyl)methylamino]-α-[[(2,4-dichlorophenyl)methoxy]methyl]-1-piperidineethanol ethanedioate (1:1); mp. 158.1°C (408);

α-[(4-fluorophenoxy)methyl]-4-[(oxazolo[4,5-b]pyridin-2-yl)amino]-1-piperidineethanol (E)-2-butenedioate (1:1); mp. 195.4°C (409);

(+)-(R)-4-[(2-benzothiazolyl)methylamino]-α-[(4-fluorophenoxy)methyl]-1-piperidineethanol dihydrochloride mp. 179.2°C, [α] = + 12.49° (c = 0.5% in methanol) (410); and

(−)-(S)-4-[(2-benzothiazolyl)methylamino]-α-[(4-fluorophenoxy)methyl]-1-piperidineethanol dihydrochloride; mp. 175.7°C, [α] = −11.37°(c = 0.5% in methanol) (411).

In a similar manner there is also prepared:

α-[(4-fluorophenyl)methyl]-4-[(oxazolo[4,5-d]pyridin-2-yl)amino]-1-piperidineethanol (412).

## Example 34

A mixture of 2.7 parts of 2,3-dihydro-2-oxiranyl-1,4-benzodioxin, 3.5 parts of *N*-methyl-*N*-(4-piperidinyl)-2-benzoxazolamine, 45 parts of methylbenzene and 40 parts of methanol was stirred and refluxed overnight. The reaction mixture was evaporated and the residue was separated by column chromatography over LiChroprep. RP 18 using a mixture of water, ammonium acetate and methanol (30:0.5:70 by volume) as eluent. The first fraction (A-isomer) was collected and the eluent was evaporated. The residue was taken up in water and the solution was treated with sodium hydroxide. The product was extracted with trichloromethane. The extract was washed with water, dried, filtered and evaporated. The residue was crystallized from 2-propanol, yielding 0.3 parts (5%) of (A)-4-[(2-benzoxazolyl)methylamino]-α-(2,3-dihydro-1,4-benzodioxin-2-yl)-1-piperidineethanol; mp. 160.4°C (413). The second fraction (B-isomer) was collected and the eluent was evaporated. The residue was taken up in water and the solution was treated with sodium hydroxide. The product was extracted with trichloromethane. The extract was washed with water, dried, filtered and evaporated. The residue was crystallized from a mixture of 2,2'-oxybispropane and methanol, yielding 1.3 parts (21%) of (B)-4-[(2-benzoxazolyl)methylamino]-α-(2,3-dihydro-1,4-benzodioxin-2-yl)-1-piperidineethanol; mp. 120.9°C (414).

In a similar manner there were also prepared:

(A)-4-[(2-benzothiazolyl)methylamino]-α-(2,3-dihydro-1,4-benzodioxin-2-yl)-1-piperidineethanol; mp. 142.0°C (415).

(B)-4-[(2-benzothiazolyl)methylamino]-α-(2,3-dihydro-1,4-benzodioxin-2-yl)-1-piperidineethanol; mp. 134.2°C (416).

## Example 35

A mixture of 3.6 parts of [(3-chlorophenoxy)methyl]oxirane, 3.3 parts of *N*-methyl-*N*-(3-pyrrolidinyl)-2-benzoxazolamine and 90 parts of methylbenzene was stirred and refluxed overnight. The reaction mixture was evaporated. The residue was purified by column chromatography over silica gel using a mixture of trichloromethane and methanol (99:1 by volume) as eluent. The pure fractions were collected and the eluent was evaporated. The residue was converted into the 4-methylbenzenesulfonate salt in 2-propanol. The salt was filtered off and dried, yielding 5 parts (58%) of 3-[(2-benzoxazolyl)methylamino]-α-[(3-chlorophenoxy)methyl]-1-pyrrolidineethanol 4-methylbenzenesulfonate (1:1); mp. 96.8°C (417).

In a similar manner there were also prepared:

4-[(2-benzothiazolyl)methylamino]-α-[(3-fluorophenoxy)methyl-1-piperidineethanol dihydrochloride; mp. 205.2°C (418);

4-[(2-benzothiazolyl)(phenylmethyl)amino]-α-[(4-fluorophenoxy)methyl]-1-piperidineethanol (E)-2-butenedioate(2:1); mp. 163.5°C (419);

4-[methyl(6-methyl-2-benzothiazolyl)amino]-α-(phenoxymethyl)-1-piperidineethanol; mp. 140.6°C (420);

α-[(4-fluorophenoxy)methyl]4-[methyl(6-methyl-2-benzothiazolyl)amino]-1-piperideneethanol: mp. 105.5°C (421);

α-[(3-fluorophenoxy)methyl]-4-[methyl(6-methyl-2-benzothiazolyl)amino]-1-piperidineethanol; mp. 109.6°C (422);

4-[(2-benzothiazolyl)(phenylmethyl)amino]-α-[(4-chlorophenoxy)methyl]-1-piperideethanol (E)-2-butenedioate(2:1); mp. 172.0°C (423);

4-[(2-benzothiazolyl)methylamino]-α-[(4-fluorophenoxy)methyl]-α-methyl-1-piperidineethanol 4-methylbenzenesulfonate(1:2); mp. 207.3°C (424);

*cis*-4-[(22-benzothiazolyl)methylamino]-3-hydroxy-α-(phenoxymethyl)-1-piperidineethanol; mp. 152.2°C (425);

*cis*-4-[(2-2-benzothiazolyl)amino]-α-[(4-fluorophenoxy)methyl]-3-hydroxy-1-piperidineethanol (Z)-2-butenedioate(1:2); mp. 156.7°C (426); and

4-[(2-benzothiazolyl)methylamino]-α-[[(2,3-dihydro-1,4-benzodioxin-2-yl)-methoxy]methyl]-1-piperidineethanol (E)-2-butenedioate(1:1); mp. 146.5°C (427).

## Example 36

A mixture of 2.77 parts of [(3-chlorophenoxy)methyl]oxirane, 4.92 parts of *N*-(3-methyl-2(3*H*)-benzothaizolylidene)-4-piperidinamine methanol and 68 parts of methylbenzene was stirred and refluxed overnight. The reaction mixture was cooled and the solvent was evaporated. The residue was purified by filtration over silica gel using a mixture of trichloromethane and methanol (99:1 by volume), as eluent. The pure fractions were collected and the eluent was evaporated. The residue was converted into the (Z)-2-butanedioate salt in 2-propanol and methanol. The salt was filtered off and dried, yielding 5.5 parts (55%) of α-[(3-chlorophenoxy)methyl]-4-[(3-methyl-2(3*H*)-benzothiazolyliden)amino]-1-piperidineethanol (Z)-2-butenedioate(1:2); mp. 157.6°C (428).

In a similar manner there were also prepared:

α-[(4-fluorophenoxy)methyl]-4-[(3-methyl-2(3*H*)-benzothiazolyliden)amino]-1-piperideethanol (Z)-2-butenedioate (1:2); mp. 140.2°C (429); and

3-[(2-benzothiazolyl)methylamino]-α-(phenoxymethyl)-1-pyrrolidineethanol 4-methylbenzenesulfonate(1:2); mp. 1336.3°C (430).

## Example 37

A mixture of 2.5 parts of [(4-fluorophenoxy)methyl]oxirane, 4.3 parts of *N*-butyl-*N*-(4-piperidinyl)-2-benzothiazolamine, 45 parts of benzene and 40 parts of methanol was stirred and refluxed overnight. The reaction mixture was evaporated. The residue was purified by column chromatography over silica gel using a mixture of trichloromethane and methanol (97:3 by volume) as eluent. The pure fractions were collected and the eluent was evaporated. The residue was converted into the (E)-2-butenedioate salt in ethanol. The salt was filtered off and dried, yielding 5.6 parts (72%) of 4 - [(2 - benzothiazolyl)butylamino] - α - [(4 - fluorophenoxy)methyl] - 1 - piperidineethanol (E)-2-butenedioate(2:1); mp. 196.8°C (431).

In a similar manner there were also prepared:

4-[(2-benzothiazolyl)butylamino]-α-[(4-chlorophenoxy)methyl]-1-piperidineethanol (E)-2-butenedioate(2:1); mp. 192.2°C (432).

## Example 38

A mixture of 1.8 parts of (B)-3,4-dihydro-2-oxiranyl-2*H*-1-benzopyran, 2.3 parts of *N*-methyl-*N*-(4-piperidinyl)-2-benzoxazolamine and 40 parts of ethanol was stirred and refluxed overnight. The reaction mixture was evaporated. The oily residue was purified by column chromatography over silica gel using a mixture of trichloromethane and methanol (90:10 by volume) as eluent. The pure fractions were collected and the eluent was evaporated. The oily residue was converted into the ethanedioate salt in 2-propanol. The salts was filtered off (slowly), washed with 2-propanol and 2,2'-oxybispropane and dried, yielding 3.82 parts (77.6%) of (B) - 4 - [(2 - benzoxazolyl)methylamino] - α - (3,4 - dihydro - 2*H* - 1 - benzopyran - 2 - yl) - 1 - piperidineethanol ethanedioate(1:1); mp. 192.5°C (433).

Following the same procedure and using equivalent amounts of the appropriate starting materials, there were also prepared:

(A)-4-[(2-benzothiazolyl)methylamino]-α-(3,4-dihydro-2*H*-1-benzopyran-2-y)-1-piperidineethanol ethanedioate(1:1); mp. 195.0°C (434);

(B)-4-[(2-benzothiazolyl)methylamino]-α-(3,4-dihydro-2*H*-1-benzopyran-2-yl)-1-piperidineethanol; mp. 133.4°C (435);

(A)-4-[(2-benzoxazolyl)methylamino]-α-(3,4-dihydro-2*H*-1-benzopyran-2-yl)-1-piperidineethanol ethanedioate(1:1); mp. 206.0°C (436);

$A^+$-4-[(2-benzothiazolyl)methylamino]-α-(3,4-dihydro-2*H*-1-benzopyran-2-yl)-1-piperidineethanol ethanedioate(2:5); mp. 159.0°C (437);

($A^-$)-4-[(2-benzothiazolyl)methylamino]-α-(3,4-dihydro-2*H*-1-benzopyran-2-yl)-1-piperidineethanol; mp. 186.3°C; $[\alpha]^D = -4.61°$ (c = 1% in methanol) (438); and

$A^+$-4-[(2-benzothiazolyl)methylamino]-α-(3,4-dihydro-2*H*-1-benzopyran-2-yl)-1-piperidineethanol; mp. 138.5°C; $[\alpha]^{25} = 47.60°$ (c = 1% in methanol) (439).

Example 39

A mixture of 3.7 parts of [(phenylthio)methyl]oxirane, 5.0 parts of *N*-methyl-*N*-(4-piperidinyl)-2-benzothiazolamine and 120 parts of 2-propanol was stirred overnight at reflux temperature. The reaction mixture was evaporated. The residue was converted into the (Z)-2-butenedioate salt in 2-propanol and 2,2'-oxybispropane (5:1 by volume). The salt was filtered off and dried, yielding 8.3 parts (78.3%) of 4-[(2-benzothiazolyl)methylamino]-α-(phenylthio)methyl]-1-piperidineethanol (Z)-2-butenedioate(1:1) mp. 160.2°C (440).

In a similar manner there were also prepared:

4-[(2-benzothiazolyl)methylamino]-α-[(2-bromo-4-fluorophenyl)methyl]-1-piperidineethanol mp. 124.6°C (441);

4-[(2-benzothiazolyl)methylamino]-α-[[(4-fluorophenyl)thio]methyl]-1-piperidineethanol (Z)-2-butenedioate(1:1); mp. 140.6°C (442);

4-[(2-benzothiazolyl)methylamino]-α-[[(4-chlorophenyl)thio]methyl]-1-piperidineethanol (Z)-2-butenedioate(1:1); mp. 174.1°C (443);

4-[(2-benzothiazolyl)methylamino]-α-[[(4-fluorophenyl)thio]methyl]-1-piperidineethanol (Z)-2-butenedioate(1:1); mp. 141.7°C (444);

4-[(2-benzothiazolyl)methylamino]-α-[[[(4-chlorophenyl)thio]methyl-1-piperidineethanol (Z)-2-butenedioate(1:1); mp. 168.8°C (445);

4-[(2-benzothiazolyl)methylamino]-α-[(phenylthio)methyl]-1-piperidineethanol (E)-2-butenedioate(2:1); mp. 180.2°C (446); and

α-[(4-fluorophenoxy)methyl]-4-[(thiazolo[4,5-c]pyridin-2-yl)amino]-1-piperidineethanol ethanedioate(1:2); mp. 195.9°C (447).

Example 40

A mixture of 1.65 parts of 1-(4-fluorobenzoyl)aziridine (solution in benzene 1.2M), 2.1 parts of *N*-(4-piperidinyl)-2-benzoxazolamine and 90 parts of methylbenzene was stirred and refluxed overnight. The reaction mixture was evaporated and the residue was crystallized from 2-propanol. The product was filtered off and recrystallized from 2-propanone, yielding 1.3 parts (34%) of *N*-[2-[4-[(2-benzoxazolyl)amino]-1-piperidinyl]ethyl])-4-fluorobenzamide; mp. 173.3°C (448).

In a similar manner there were also prepared:

*N*-[2-[4-[2-(benzoxazolyl)methylamino]-1-piperidinyl]ethyl]-4-fluorobenzamide; mp. 162.9°C (449).

Example 41

A mixture of 3.2 parts of 4-(1-ethenyl)pyridine, 2.1 parts of *N*-(4-piperidinyl)-2-benzoxazolamine and 80 parts of 1-butanol was stirred and refluxed for 48 hours. The reaction mixture was evaporated. The residue was purified by column chromatography over silica gel using a mixture of trichloromethane and methanol (97:3 by volume) saturated with ammonia, as eluent. The pure fractions were collected and the eluent was evaporated. The residue was crystallized from 2,2'-oxybispropane, yielding 2 parts (62%) of *N*-[1-[2-(4-pyridinyl)ethyl]-4-piperidinyl]-2-benzoxazolamine; mp. 146.9°C (450).

In a similar manner there were also prepared:

*N*-[1-[2-(2-pyridinyl)ethyl]-4-piperidinyl]-2-benzoxazolamine; mp. 130°C (451); and

*N*-methyl-*N*-[1-[2-(2-pyridinylethyl]-4-piperidinyl]-2-benzoxazolamine; mp. 102.3°C (452).

Example 42

A mixture of 4.2 parts of 4-[(2-benzothiazolyl)methylamino]-α-[(4-fluorophenoxy)methyl]-1-piperidineethanol, 4.1 parts of acetic acid anhydride, 0.1 parts of 4-methylbenzenesulfonic acid and 135 parts of methylbenzene was stirred and refluxed overnight. After cooling to room temperature, the reaction mixture was washed with a sodium hydroxide solution 5% and with water, dried, filtered and evaporated. The residue was crystallized from 2,2'-oxybispropane. The product was filtered off and dried, yielding 3.5 parts (77%) of 4-[(2-benzothiazolyl)methylamino]-α-[(4-fluorophenoxy)methyl]-1-piperidineethanol acetate(ester); mp. 100.0°C (453).

Example 43

To a stirred and hot (60°C) solution of 4.2 parts of 4 - [2 - benzothiazolyl)methylamino] - α - [(4 - fluorophenoxy)methyl] - 1 - piperidineethanol in 90 parts of *N,N*-dimethylformamide were added dropwise 2.3 parts of decanoyl chloride. Upon completion, stirring was continued for 24 hours at 60°C. After cooling to room temperature, the reaction mixture was poured onto water and the whole was treated with sodium hydroxide. The aqueous phase was decanted and the residual product was washed three times with water and dissolved in trichloromethane. The organic layer was dried, filtered and evaporated. The residue was purified by column chromatography over silica gel using a mixture of trichloromethane and methanol (97:3 by volume) as eluent. The pure fractions were collected and the eluent was evaporated. The residue was converted into the (Z)-2-butenedioate salt in a mixture of 2-propane and 2,2'-oxybisopropane. The salt was filtered off and dried, yielding 1.1 parts (16%) of 2 - [4 - [(2 - benzothiazolyl)-methylamino] - 1 - piperidinyl] - 1 - [(4 - fluorophenoxy)methyl]ethyl]decanoate (Z)-2-butenedioate(1:1); mp. 134.2°C (454).

Example 44

3.4 Parts of (*cis* + *trans*)-*N*-[1-[4-(4-fluorophenoxy)cyclohexyl]-4-piperidinyl]-*N*-methyl-2-benzothiazolamine were separated by column chromatography (HPLC) over silica gel using a mixture of trichloromethane and methanol (98:2 by volume) was eluent.

The first fraction (A-isomer) was collected and the eluent was evaporated. The residue was crystallized from 2-propanol, yielding 0.34 parts of (A) - *N* - [1 - [4 - (4 - fluorophenoxy)cyclohexyl] - 4 - piperidinyl] - *N* - methyl - 2 - benzothiazolamine; mp. 155.5°C (455).

The second fraction (B-isomer) was collected and the eluent was evaporated. The residue was crystallized from 2-propanol, yielding 0.34 parts of (B) - *N* - [1 - [4 - (4 - fluorophenoxy)cyclohexyl] - 4 - piperidinyl] - *N* - methyl - 2 - benzothiazolamine; mp. 116.5°C (456).

Example 45

To a stirred solution of 5 parts of 4 - [(2 - benzothiazolyl)methylamino] - α - [(4 - fluorophenoxy)-methyl] - 1 - piperidineethanol in 80 parts of ethanol were added 1.8 parts of (+)-[R-(R*,R*)]-2,3-dihydroxybutanedioic acid while boiling. The product was allowed to crystallize. It was filtered off and dried, yielding 5.5 parts (94%) of (+)-4-[(2-benzothiazolyl)methylamino]-α-[(4-fluorophenoxy)methyl]-1-piperidineethanol [R-(R*,R*)]-2,3-dihydroxybutanedioate (2:1); mp. 189.1°C (457).

In a similar manner there was also prepared:

4-[(2-benzothiazolyl)methylamino]-α-[(4-fluorophenoxy)methyl]-1-piperidineethanol 2-hydroxy-1,2,3-propanetricarboxylate(1:1); mp. 146.7°C (458) and

4-[(2-benzothiazolyl)methylamino]-α-[(4-fluorophenoxy)methyl]-1-piperidineethanol (Z)-2-butenedioate (1:1); mp. 127.6°C (459).

c. Pharmacological Examples

The useful anti-anoxic properties of the compounds of formula (I) can be demonstrated in the following test procedures.

Example 46

KCN Test in Rats

Histotoxic anoxia is produced by a rapid intravenous injection of potassium cyanide (KCN) in rats.

KCN in control rats induces abdominal contractions and clonic seizures and is lethal within 2 minutes. The protection from KCN induced lethality appears to be a simple way of evaluating quantitatively the anti-anoxic properties of the test compounds. Male Wistar rats weighing + 200 g were treated subcutaneously with a test compound. One hour after treatment there was injected intravenously a KCN solution at a dose of 5 mg/kg body weight. Survival after 2 minutes was evaluated and $ED_{50}$-values were determinated.

The $ED_{50}$-values of the compounds of formula (I) are listed in table I. Said $ED_{50}$-values are those values in mg/kg body weight capable of protecting 50% of the tested animals against KCN-induced lethality.

The compounds listed in table 1 are not given for the purpose of limiting the invention thereto but only to exemplify the useful pharmacological activities of all the compounds within the scope of formula (I).

| Compound number | $ED_{50}$-values in mg/kg body weight |
|---|---|
| 1 | 5 |
| 9 | 2.5 |
| 10 | 5 |
| 11 | 1.25 |
| 15 | 0.63 |

| | |
|---|---|
| 18 | 5 |
| 24 | 5 |
| 31 | 5 |
| 32 | 5 |
| 42 | 2.5 |
| 48 | 1.25 |
| 50 | 2.5 |
| 75 | 10 |
| 103 | 5 |
| 107 | 2.5 |
| 108 | 5 |
| 118 | 5 |
| 120 | 2.5 |
| 121 | 2.5 |
| 123 | 5 |
| 133 | 5 |
| 146 | 5 |
| 148 | 5 |
| 151 | 5 |
| 184 | 5 |
| 191 | 2.5 |
| 195 | 1.25 |
| 309 | 5 |
| 320 | 1.8 |
| 322 | 5 |
| 323 | 1.25 |
| 324 | 2.5 |
| 357 | 0.63 |
| 405 | 10 |
| 410 | 5 |
| 411 | 5 |
| 413 | 5 |

| | |
|---|---|
| 415 | 10 |
| 438 | 10 |
| 440 | 10 |
| 442 | 2.5 |
| 443 | 10 |
| 446 | 10 |
| 452 | 5 |

D) Composition Examples

The following formulations exemplify typical pharmaceutical compositions in dosage unit form suitable for systemic administration to animal and human subjects in accordance with the instant invention.

"Active ingredient" (A.I.) as used throughout these examples relates to a compound of formula (I) or pharmaceutically acceptable acid addition salt thereof.

Example 47

Oral Drops

500 Grams of the A.I. was dissolved in 0.5 liters of 2-hydroxypropanoic acid and 1.5 liters of the polyethylene glycol at 60—80°C. After cooling to 30—40 there were added 35 liters of polyethylene glycol and the mixture was stirred well. Then there was added a solution of 1750 grams of sodium saccharin in 2.5 liters of purified water and while stirring there were added 2.5 liters of cocoa flavor and polyethylene glycol q.s. to a volume of 50 liters, providing an oral drop solution comprising 10 milligrams of the A.I. per milliliter. The resulting solution was filled into suitable containers.

Example 48

Oral Solution

9 Grams of methyl 4-hydroxybenzoate and 1 gram of propyl 4-hydroxybenzoate were dissolved in 4 liters of boiling purified water. In 3 liters of this solution were dissolved first 10 grams of 2,3-dihydroxybutanedioic acid and thereafter 20 grams of the A.I. The latter solution was combined with the remaining part of the former solution and 12 liters 1,2,3-propanetriol and 3 liters of sorbitol 70% solution were added thereto. 40 Grams of sodium saccharin were dissolved in 0.5 liters of water and 2 milliliters of raspberry and 2 milliliters of gooseberry essence were added. The later solution was combined with the former, water was added q.s. to a volume of 20 liters providing an oral solution comprising 20 milligrams of the active ingredient per teaspoonful (5 milliliters). The resulting solution was filled in suitable containers.

Example 49

Capsules

20 Grams of the A.I., 6 grams lauryl sulfate, 56 grams starch, 56 grams lactose, 0.8 grams colloidal silicon dioxide, and 1.2 grams magnesium stearate were vigorously stirred together. The resulting mixture was subsequently filled into 1000 suitable hardened gelating capsules, comprising each 20 milligrams of the active ingredient.

Example 50

Film-Coated Tablets

Preparation of tablet core

A mixture of 100 grams of the A.I., 570 grams lactose and 200 grams starch was mixed well and thereafter humidified with a solution of 5 grams sodium dodecyl sulfate and 10 grams polyvinylpyrrolidone (Kollidon-K 90®) in about 200 milliliters of water. The wet powder mixture was sieved, dried and sievedi again. Then there was added 100 grams microcrystalline cellulose (Advicel®) and 15 grams hydrogenated vegetable oil (Sterotex®). The whole was mixed well and compressed into tablets, giving 10.000 tablets, each containing 10 milligrams of the active ingredient.

Coating

To a solution of 10 grams methyl cellulose (Methocel 60 HG®) in 75 milliliters of denaturated ethanol there was added a solution of 5 grams of ethyl cellulose (Ethocel 22 cps®) in 150 milliliters of dichloromethane. Then there were added 75 milliliters of dichloromethane and 2.5 milliliters 1,2,3-propanetriol. 10 Grams of polyethylene glycol was molten and dissolved in 75 milliliters of dichloromethane. The latter solution was added to the former and then there were added 2.5 grams of

magnesium octadecanoate, 5 grams of polyvinylpyrrolidone and 30 milliliters of concentrated colour suspension (Opaspray K-1-2109®) and the whole was homogenated.

The tablet cores were coated with the thus obtained mixture in a coating apparatus.

Example 51

Injectable Solution

1.8 Grams methyl 4-hydroxybenzoate and 0.2 grams propyl 4-hydroxybenzoate were dissolved in about 0.5 liters of boiling water for injection. After cooling to about 50°C there were added while stirring 4 grams lactic acid, 0.05 grams propylene glycol and 4 grams of A.I. The solution was cooled to room temperature and supplemented with water for injection q.s. ad 1 liter volume, giving a solution of 4 milligrams A.I. per milliliters. The solution was sterilized by filtration (U.S.P. XVII p. 811) and filled in sterile containers.

Example 52

Suppositories

3 rams A.I. was dissolved in a solution of 3 grams 2,3-dihydroxybutanedioic acid in 25 milliliters polyethylene glycol 400. 12 Grams surfactant (SPAN®) and triglycerides (Witepsol 555®) q.s. ad 300 grams were molten together. The latter mixture was mixed well with the former solution. The thus obtained mixture was poured into moulds at a temperature of 37—38°C to form 100 suppositories each containing 30 milligrams of the active ingredient.

**Claims**

1. A chemical compound having the formula

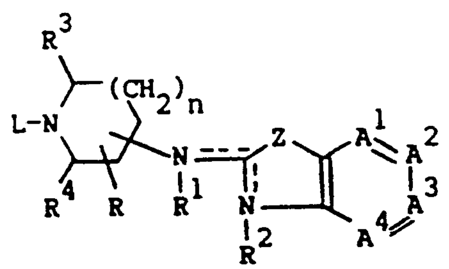

(I)

the pharmaceutically acceptable acid addition salts and the possible stereochemically isomeric form thereof, wherein:

$-A^1=A^2-A^3=A^4-$ is a bivalent radical having the formula

| | |
|---|---|
| —CH=CH—CH=CH— | (a); |
| —N=CH—CH=CH— | (b); |
| —CH=N—CH=CH— | (c); |
| —CH=CH—N=CH— | (d); or |
| —CH=CH—CH=N— | (e); |

wherein one or two hydrogen atoms in the radical $-A^1=A^2-A^3=A^4-$ may, each independently from each other, be replaced by halo, $C_{1-6}$ alkyl, hydroxy, $C_{1-6}$ alkyloxy, or trifluoromethyl;

Z is O or S;

n is 0 or the integer 1;

R is a member selected from the group consisting of hydrogen, $C_{1-6}$ alkyl, hydroxy and $C_{1-6}$ alkyloxy;

$R^1$ or $R^2$ is hydrogen, $C_{1-6}$ alkyl, or aryl $C_{1-6}$ alkyl; the dotted line between the nitrogen atom bearing $R^1$, the interjacent carbon atom and the nitrogen atom bearing $R^2$ indicating that a double bond exists between the nitrogen bearing $R^2$ indicating that a double bond exists between the nitrogen bearing $R^1$ and said interjacent carbon atom, in which case $R^1$ is absent, or a double bond exists between the interjacent carbon and the nitrogen bearing $R^2$, in which case $R^2$ is absent; and wherein said interjacent carbon atom is the carbon atom positioned between the nitrogen bearing $R^1$ and the nitrogen bearing $R^2$;

$R^3$ or $R^4$ are both hydrogens or $R^3$ and $R^4$ combined may form a bivalent radical of formula $-CH_2-CH_2-$;

L is a member selected from the group consisting of $C_{1-12}$ alkyl, substituted $C_{1-6}$ alkyl, aryl $C_{2-6}$ alkenyl, $C_{3-6}$ cycloalkyl, substituted $C_{3-6}$ cycloalkyl, a radical of formula

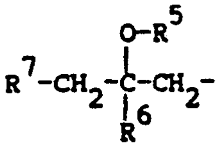

(a),

and a radical of formula

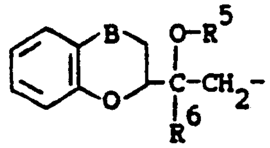

(b);

$R^5$ being hydrogen or $C_{1-12}$ alkylcarbonyl;
$R^6$ being hydrogen or $C_{1-6}$ alkyl;
$R^7$ being aryl, a radical $R^8$—O or a radical $R^8$—S;
B being a bivalent radical of formula —$CH_2$— or —O—; and
said $R^8$ being hydrogen; aryl; 2,3-dihydro-1*H*-indenyl; benzodioxolyl; (2,3-dihydro-1,4-benzodioxin-2-yl)methyl; (2*H*-1-benzopyran-2-yl)methyl; or phenyl substituted with $C_{2-6}$ alkenyloxy, $C_{1-6}$ alkylcarbonylamino or $C_{1-6}$ alkylphenylcarbonyl; and

wherein said substituted $C_{3-6}$ cycloalkyl is $C_{3-6}$ cycloalkyl being substituted with up to two substituents each independently selected from the group consisting of aryl, aryloxy and cyano;

said substituted $C_{1-6}$ alkyl is $C_{1-6}$ alkyl being substituted with a member selected from the group consisting of $C_{3-6}$cycloalkyl, pyridinyl, pyridinyloxy, aryl, benzimidazolyl, indolyl, isoxazolyl being optionally substituted with phenyl, 3-oxo-1,2,4-triazolo[4,5-a]pyridin-2(3*H*)-yl, a radical of formula ArY—, a radical of formula $R^9$O— and a radical of formula

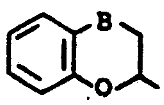

(c);

said Ar being aryl;
Y being O, S, NH, —CONH—, —CHCN, —CHOR$^{10}$ or —$SO_2$;
$R^9$ being hydrogen, aryl $C_{1-6}$ alkyl, $C_{3-6}$ cycloalkyl, phenyl substituted with phenyl, or phenyl substituted with $C_{1-6}$ alkyloxycarbonyl;
$R^{10}$ being hydrogen or $C_{1-6}$ alkyl; and
wherein aryl is phenyl optionally substituted with up to three substituents each independently selected from the group consisting of $C_{1-6}$ alkyl, $C_{1-6}$ alkyloxy, halo, trifluoromethyl, cyano, $C_{1-6}$ alkylcarbonyl, nitro, amino and aminocarbonyl.

2. A chemical compound according to claim 1, wherein L is aryl $C_{2-6}$ alkenyl, $C_{1-6}$ alkyl being substituted with aryloxy, a radical of formula (a) or a radical of formula (b), wherein aryl is as defined in claim 1.

3. A chemical compound according to claim 2, wherein —$A^1$=$A^2$—$A^3$=$A^4$— is —CH=CH—CH=CH—, n is the integer 1, $R^2$ is absent and a double bond exists between the nitrogen bearing $R^2$ and the interjacent carbon atoms, and $R^3$ and $R^4$ are both hydrogen atoms.

4. A chemical compound according to claim 3 wherein in the radical of formula (a), $R^5$ and $R^6$ are both hydrogen and $R^7$ is a radical $R^8$—O—, wherein $R^8$ is aryl; or wherein in the radical of formula (b), B is O and $R^5$ and $R^6$ are both hydrogen radicals, wherein aryl is as defined in claim 1.

5. A chemical compound according to claim 4 wherein $R^1$ is $C_{1-6}$ alkyl and aryl is halophenyl.

6. A chemical compound according to claim 1, wherein the compound is 4-[(2-benzothiazolyl)methylamino]-α-[(4-fluorophenoxy)methyl]-1-piperidineethanol.

7. A pharmaceutical composition comprising a suitable pharmaceutical carrier and as an active ingredient a therapeutically effective amount of a compound as claimed in any one of claims 1 to 6.

8. An anti-anoxic or anti-hypoxic composition comprising a suitable pharmaceutical carrier and as an active ingredient an effective anti-anoxic or anti-hypoxic amount of a compound as claimed in any one of claims 1 to 6.

9. A method of preparing a pharmaceutical composition, characterized in that a therapeutically effective amount of a compound as claimed in any one of claims 1 to 6 is intimately mixed with suitable pharmaceutical carriers.

10. A compound as claimed in any one of claims 1 to 6 for use as a medicine.

11. A compound as claimed in any one of claims 1 to 6 for use as an anti-anoxic or anti-hypoxic medicine.

12. A process for preparing a chemical compound as claimed in claim 1, characterized by

a) *N*-alkylating a piperidine of formula

H—D (III),

with a reagent of formula

L—W (II),

wherein W represents a reactive leaving group, in a reaction-inert solvent;

b) reductively *N*-alkylating a piperidine of formula (III) with a carbonyl-compound of formula

L′=C=O (IV),

whereby L′=C=O is a compound of formula L—H wherein a —$CH_2$— radical is oxidated to carbonyl radical, in a reaction-inert solvent;

c) cyclizing an urea or thiourea derivative of formula

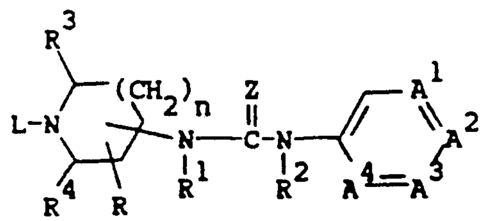

(V)

in a reaction-inert solvent in the presence of an oxidant;

d) reacting a piperidine of formula (III) with an oxirane of formula

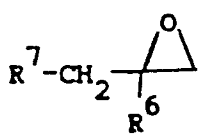

(VI)

in a reaction-inert solvent, thus preparing a compound of formula

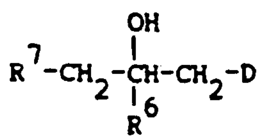

(I-a-1),

and, if desired, acylating (I-a-1) with a reagent

$R^{5-a}-W^1$ (VII),

said $R^{5-a}$-$W^1$ representing an alkanoic acid or a reactive derivative thereof, thus preparing a compound of formula

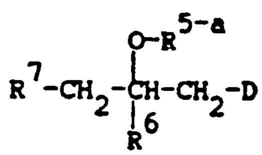

(I-a-2);

e) reacting a piperidine of formula (III) with an oxirane of formula

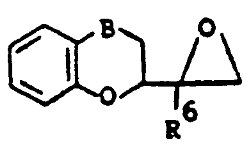

(VIII)

in a reaction-inert solvent, thus preparing a compound of formula

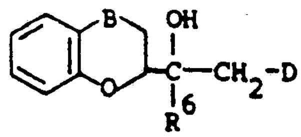

(I-a-3)

and, if desired, acylating (I-a-3) with a reagent (VII), thus preparing a compound of formula

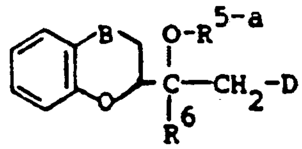

(I-a-4)

f) reacting a benzothiazole or benzoxazole of formula

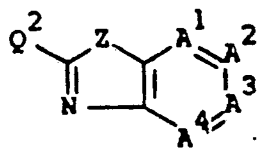

(X)

with a reagent of formula

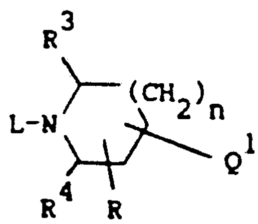

(IX)

in a reaction-inert solvent, wherein $Q^1$ is a reactive leaving group and $Q^2$ is a radical of formula —$NHR^1$, or alternatively, $Q^1$ is a radial of formula —$NHR^1$ and $Q^2$ is a reactive leaving group, thus preparing a compound of formula

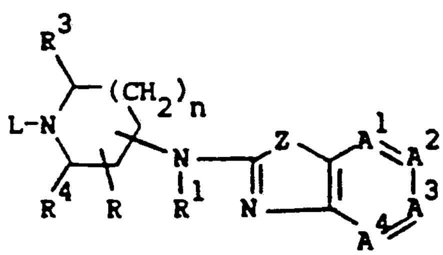

(I-b);

g) cyclodesulfurizing a thiourea derivative of formula

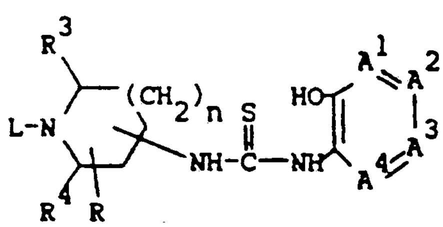

(XIV)

which in situ may be prepared by condensing an isothiocyanate

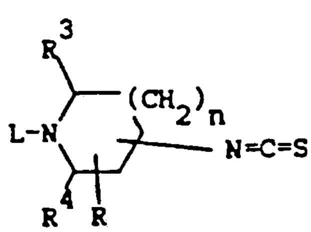

(XII)

with an aromatic aminoalcohol of formula

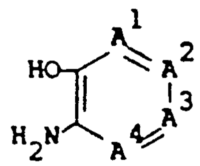

(XIII),

thus preparing a compound of formula

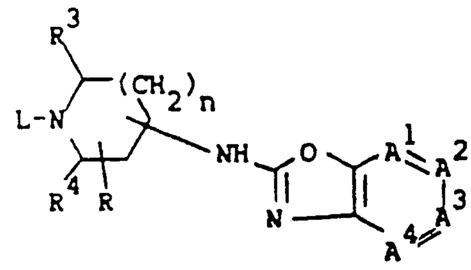

(I-b-1);

h) cyclizing an urea or thiourea derivative of formula

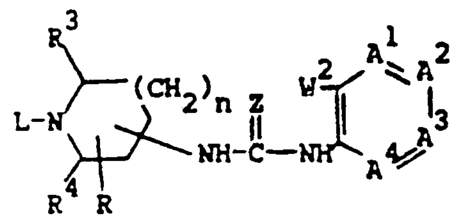

(XVII)

which in situ may be formed by reacting an isocyanate or isothiocyanate of formula

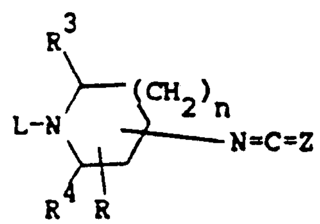

(XV)

with an aromatic amine of formula

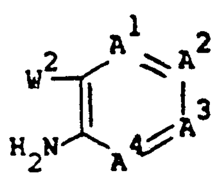

(XVI),

wherein $W^2$ represents a leaving group, thus preparing a compound of formula

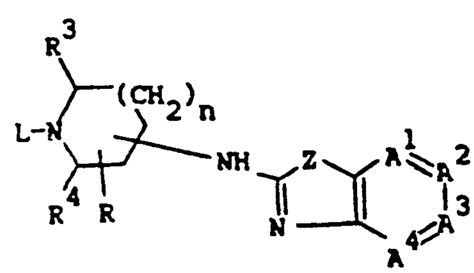

(I-b-2)

i) reacting a piperidine of formula (III) with an alkene derivative of formula

$R^{11}$—$C_{2-6}$ alkenediyl-H (XVIII)

in a reaction-inert solvent, thus preparing a compound of formula

$$R^{11}-C_rH_{2r}-D \quad (I\text{-}c)$$

wherein $R^{11}$ represents $C_{1-6}$ alkyl, $C_{3-6}$ cycloalkyl, pyridinyl, pyridinyloxy, aryl, benzimidazolyl, indolyl, isoxazolyl being substituted with phenyl, 3-oxo-1,2,4-triazolo[4,5-a]pyridin-2(3*H*)-yl, a radical of formula ArY, a radical of formula $R^9O$ and a radical of formula (g); and r is an integer of from 2 to 6 included;

j) reacting a piperidine of formula (III) with an aziridine of formula

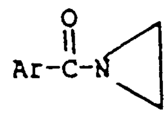

(XIX)

in a reaction-inert solvent thus preparing a compound of formula

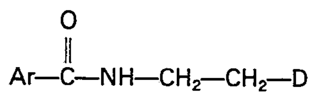

(I-d);

k) converting the compounds of formula (I) wherein $R^1$ is hydrogen into the compounds of formula (I) wherein $R^1$ is other than hydrogen by reacting the former with an *N*-alkylating agent.

l) *O*-alkylating the compounds of formula I) wherein L is a $C_{1-6}$alkyl radical substituted with hydroxy to prepare the corresponding *O*-alkylated compounds of formula (I);

wherein D represents a radical of formula

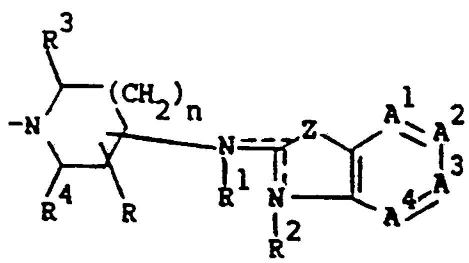

and, if desired, converting the compounds into each other following art-known functional groups-transformation reactions; and if further desired, converting the compounds of formula (I) into a therapeutically active non-toxic acid-addition salt form by treatment with an appropriate acid or, conversely, converting the acid-addition salt into the free base form with alkali; and/or preparing stereochemically isomeric forms thereof.

**Patentansprüche**

1. Chemische Verbindung der Formel

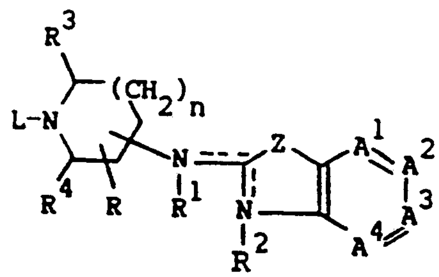

(I)

ein pharmazeutisch annehmbares Säureadditionssalz oder eine mögliche stereochemisch isomere Form hievon, worin:

$-A^1=A^2-A^3=A^4-$ ein zweiwertiger Rest mit der Formel

| | |
|---|---|
| $-CH=CH-CH=CH-$ | (a); |
| $-N=CH-CH=CH-$ | (b); |
| $-CH=N-CH=CH-$ | (c); |
| $-CH=CH-N=CH-$ | (d); |
| oder $-CH=CH-CH=N-$ | (e) |

ist; worin ein oder zwei Wasserstoffatome im Rest $-A^1=A^2-A^3=A^4-$ unabhängig voneinander durch

Halogen, $C_{1-6}$Alkyl, Hydroxy, $C_{1-6}$Alkyloxy oder Trifluormethyl ersetzt sein können;

Z für O oder S steht;

n den Wert O besitzt oder die ganze Zahl 1 ist;

R ein Rest ist, welcher aus der aus Wasserstoff $C_{1-6}$Alkyl, Hydroxy und $C_{1-6}$Alkyloxy bestehenden Gruppe ausgewählt ist,

$R^1$ oder $R^2$ Wasserstoff, $C_{1-6}$Alkyl oder Aryl $C_{1-6}$alkyl darstellen; wobei die punktierte Linie zwischen dem $R^1$ tragenden Stickstoffatom, dem dazwischenliegenden Kohlenstoffatom und dem $R^2$ tragenden Stickstoffatom darauf hinweist, daß zwischen dem $R^1$ tragenden Stickstoff und dem genannten dazwischenliegenden Kohlenstoffatom eine Doppelbindung besteht, in welchem Fall $R^1$ fehlt oder eine Doppelbindung zwischen dem dazwischenliegenden Kohlenstoff und dem $R^2$ tragenden Stickstoff vorliegt, in welchem Falle $R^2$ fehlt; und worin das genannte dazwischenliegende Kohlenstoffatom das Kohlenstoffatom ist, welches zwischen dem $R^1$ tragenden Stickstoff und dem $R^2$ tragenden Stickstoff positioniert ist;

$R^3$ oder $R^4$ beide Wasserstoffe darstellen oder $R^3$ und $R^4$ gemeinsam einen zweiwertigen Rest der Formel $-CH_2-CH_2-$ bilden können;

L ein Rest ist, welcher aus der aus $C_{1-12}$Alkyl, substituiertem $C_{1-6}$Alkyl, Aryl $C_{2-6}$Alkenyl, $C_{3-6}$Cycloalkyl, substituiertem $C_{3-6}$Cycloalkyl, einem Rest der Formel

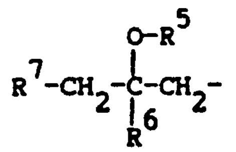

(a)

und einem Rest der Formel

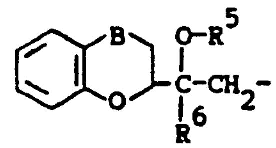

(b)

bestehenden Gruppe ausgewählt ist, worin

$R^5$ für Wasserstoff oder $C_{1-12}$Alkylcarbonyl steht;

$R^6$ Wasserstoff oder $C_{1-6}$Alkyl darstellt;

$R^7$ für Aryl, einen Rest $R^8$—O oder einen Rest $R^8$—S steht;

B ein zweiwertiger Rest der Formel $-CH_2-$ oder —O— ist; und das genannte $R^8$ Wasserstoff; Aryl; 2,3-Dihydro-1*H*-indenyl; Benzodioxolyl; (2,3-Dihydro-1,4-benzodioxin-2-yl)methyl; (2*H*-1-Benzopyran-2-yl)-methyl; oder Phenyl, substituiert mit $C_{2-6}$-Alkenyloxy, $C_{1-6}$Alkylcarbonylamino oder $C_{1-6}$Alkylphenylcarbonyl, ist; und worin das genannte substituierte $C_{3-6}$Cycloalkyl für $C_{3-6}$Cycloalkyl steht, welches mit bis zu zwei Substituenten substituiert ist, welche unabhängig voneinander aus der aus Aryl, Aryloxy und Cyano bestehenden Gruppe ausgewählt sind; das genannte substituierte $C_{1-6}$Alkyl für $C_{1-6}$Alkyl steht, welches mit einem Rest substituiert ist, der aus der aus $C_{3-6}$Cycloalkyl, Pyridinyl, Pyridinyloxy, Aryl, Benzimidazolyl, Indolyl, Isoxazolyl, wahlweise substituiert mit Phenyl, 3-Oxo-1,2,4-triazolo[4,5-a]-pyridin-2(3*H*)-yl, einem Rest der Formel ArY-, einem Rest der Formel $R^9O$— und einem Rest der Formel

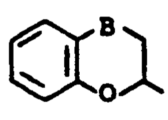

**(c);**

ausgewählt ist; welches Ar Aryl ist; worin Y für O, S, NH, —CONH—, —CHCN, —CHOR$^{10}$ oder $-SO_2$ steht; $R^9$ Wasserstoff, Aryl$C_{1-6}$alkyl, $C_{3-6}$Cycloalkyl, Phenyl substituiert mit Phenyl oder Phenyl substituiert mit $C_{1-6}$Alkyloxycarbonyl darstellt; $R^{10}$ Wasserstoff oder $C_{1-6}$Alkyl bedeutet; und worin Aryl Phenyl darstellt, das gegebenenfalls mit bis zu 3 Substituenten substituiert ist, welche unabhängig voneinander aus der Gruppe bestehend aus $C_{1-6}$Alkyl, $C_{1-6}$Alkyloxy, Halogen, Trifluormethyl, Cyano, $C_{1-6}$Alkylcarbonyl, Nitro, Amino und Aminocarbonyl ausgewählt sind.

2. Chemische Verbindung nach Anspruch 1, worin L Aryl$C_{2-6}$alkenyl, $C_{1-6}$Alkyl, substituiert mit Aryloxy, einen Rest der Formel (a) oder einen Rest der Formel (b) darstellt, wobei Aryl wie in Anspruch 1 definiert ist.

3. Chemische Verbindung nach Anspruch 2, worin $-A^1=A^2-A^3=A^4-$ für —CH=CH—CH=CH— steht, n die ganze Zahl 1 bedeutet, $R^2$ fehlt und zwischen dem $R^2$ tragenden Stickstoff und dem dazwischenliegenden Kohlenstoffatom eine Doppelbindung vorliegt und $R^3$ und $R^4$ beide Wasserstoffatome darstellen.

4. Chemische Verbindung nach Anspruch 3, worin im Rest der Formel (a) $R^5$ und $R^6$ beide Wasserstoff sind und $R^7$ einen Rest $R^8$—O— darstellt, worin $R^8$ für Aryl steht; oder worin im Rest der Formel (b) B für 0 steht und $R^5$ und $R^6$ beide Wasserstoffreste sind, wobei Aryl wie in Anspruch 1 definiert ist.

5. Chemische Verbindung nach Anspruch 4, worin $R^1$ $C_{1-6}$Alkyl bedeutet und Aryl Halogenphenyl ist.

6. Chemische Verbindung nach Anspruch 1, worin die Verbindung 4-[(2-Benzothiazolyl)methylamino]-alpha-[(4-fluorphenoxy)methyl]-1-piperidinethanol ist.

7. Pharmazeutische Zusammensetzung, umfassend einen geeigneten pharmazeutischen Träger und als wirksamen Bestandteil eine therapeutisch wirksame Menge einer Verbindung nach einem der Ansprüche 1 bis 6.

8. Anti-Anoxie oder anti-Hypoxie-Zusammensetzung, umfassend einen geeigneten pharmazeutischen Träger und als wirksamen Bestandteil eine wirksame anti-Anoxie- oder anti-Hypoxie-Menge einer Verbindung nach einem der Ansprüche 1 bis 6.

9. Verfahren zur Herstellung einer pharmazeutischen Zusammensetzung, dadurch gekennzeichnet, daß eine therapeutisch wirksame Menge einer Verbindung nach einem der Ansprüche 1 bis 6 mit geeigneten pharmazeutischen Trägern innig vermischt wird.

10. Verbindung nach einem der Ansprüche 1 bis 6 zur Verwendung als Arzneimittel.

11. Verbindung nach einem der Ansprüche 1 bis 6 zur Verwendung als ein anti-Anoxie- oder anti-Hypoxie-Arzneimittel.

12. Verfahren zur Herstellung einer chemischen Verbindung nach Anspruch 1, gekennzeichnet durch

a) *N*-Alkylieren eines Piperidins der Formel

H—D (III)

mit einem Reagens der Formel

L—W (II),

worin W eine reaktive Leaving-Gruppe darstellt, in einem reaktionsinerten Lösungsmittel;

b) reduktives *N*-Alkylieren eines Piperidins der Formel (III) mit einer Carbonyl-Verbindung der Formel

L'=C=O (IV),

wobei L'=C=O eine Verbindung der Formel L—H ist, worin ein —$CH_2$-Rest zu einem Carbonylrest oxidiert ist, in einem reaktionsinerten Lösungsmittel;

c) Cyklisieren eines Harnstoff- oder Thioharnstoffderivats der Formel

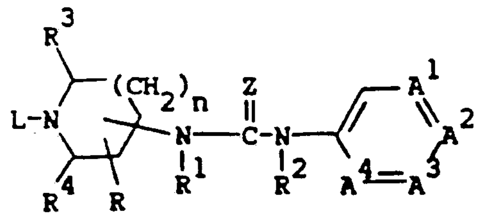

(V)

in einem reaktionsinerten Lösungsmittel in Gegenwart eines Antioxidans;

d) Umsetzen eines Piperidins der Formel (III) mit einem Oxiran der Formel

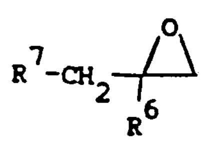

(VI)

in einem reaktionsinerten Lösungsmittel, wodurch eine Verbindung der Formel

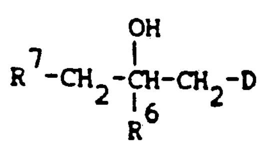

(I-a-1),

hergestellt wird, und wenn gewünscht, Acylieren von (I-a-1) mit einem Reagens

$R^{5-a}-W^1$ (VII),

welches $R^{5-a}$—$W^1$ eine Alkansäure oder ein reaktives Derivat hievon darstellt, wodurch eine Verbindung der Formel

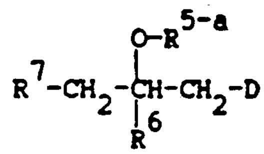

(I-a-2);

hergestellt wird;

e) Umsetzen eines Piperidins der Formel (III) mit einem Oxiran der Formel

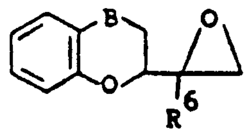

(VIII)

in einem reaktionsinerten Lösungsmittel, wodurch eine Verbindung der Formel

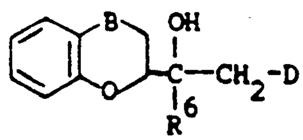

(I-a-3)

hergestellt wird, und wenn gewünscht, Acylieren von (I-a-3) mit einem Reagens (VII), wodurch eine Verbindung der Formel

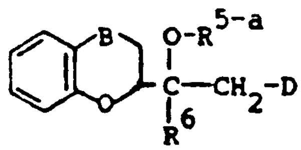

(I-a-4)

hergestellt wird;

f) Umsetzen eines Benzothiazols oder Benzoxazols der Formel

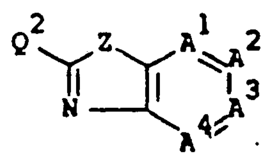

(X)

mit einem Reagens der Formel

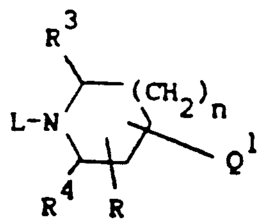

(IX)

in einem reaktionsinerten Lösungsmittel, worin $Q^1$ eine reaktive Leaving-Gruppe ist und $Q^2$ einen Rest der Formel —$NHR^1$ darstellt oder in alternativer Weise $Q^1$ ein Rest der Formel —$NHR^1$ ist und $Q^2$ eine reaktive Leaving-Gruppe darstellt, wodurch eine Verbindung der Formel

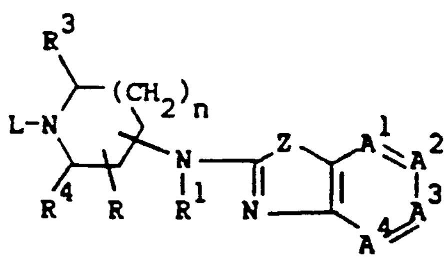

(I-b);

hergestellt wird;

g) Cyclodesulfurieren eines Thioharnstoffderivats der Formel

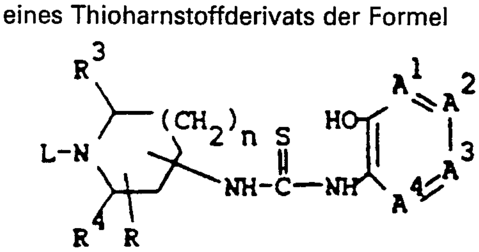

(XIV)

welches in situ durch Kondensieren eines Isothiocyanats

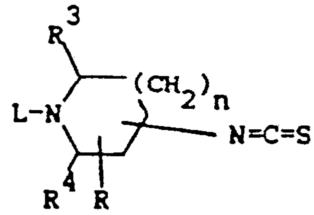

(XII)

mit einem aromatischen Aminoalkohol der Formel

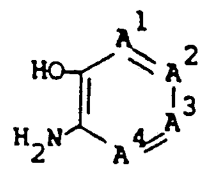

(XIII),

hergestellt werden kann, wodurch eine Verbindung der Formel

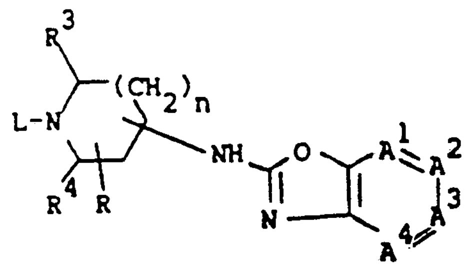

(I-b-1):

hergestellt wird;

h) Cyklisieren eines Harnstoff- oder Thioharnstoffderivats der Formel

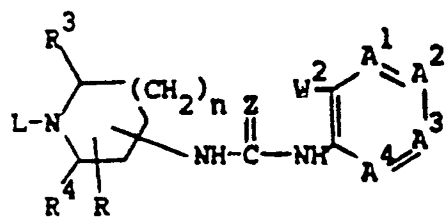

(XVII)

welches in situ durch Umsetzen eines Isocyanats oder eines Isothiocyanats der Formel

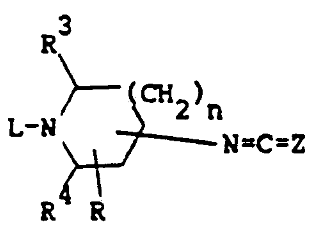

(XV)

mit einem aromatischen Amin der Formel

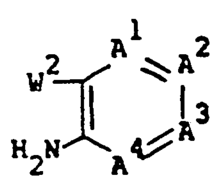

(XVI).

hergestellt werden kann, worin $W^2$ eine Leaving-Gruppe darstellt, wodurch eine Verbindung der Formel

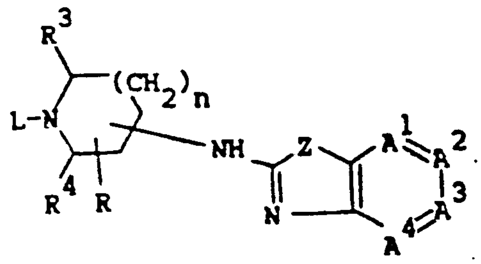

(I-b-2)

hergestellt wird;

i) Umsetzen eines Piperidins der Formel (III) mit einem Alkenderivat der Formel

$$R^{11}—C_{2-6}\text{alkendiyl-H} \qquad \text{(XVIII)}$$

in einem reaktionsinerten Lösungsmittel, wodurch eine Verbindung der Formel

$$R^{11}—C_rH_{2r}—D \qquad \text{(I-c)}$$

hergestellt wird, worin $R^{11}$ für $C_{1-6}$Alkyl, $C_{3-6}$Cycloalkyl, Pyridinyl, Pyridinyloxy, Aryl, Benzimidazolyl, Indolyl, Isoxazolyl, substituiert mit Phenyl, 3-Oxo-1,2,4-triazolo[4,5-a]pyridin-2(3*H*)-yl, einem Rest der Formel ArY, einem Rest der Formel $R^9O$ und einem Rest der Formel (g) darstellt; und r eine ganze Zahl von 2 bis einschließlich 6 ist;

j) Umsetzen eines Piperidins der Formel (III) mit einem Aziridin der Formel

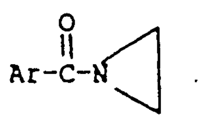

(XIX)

in einem reaktionsinerten Lösungsmittel, wodurch eine Verbindung der Formel

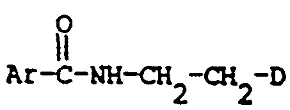

(I-d);

hergestellt wird;

k) Überführen der Verbindungen der Formel (I), worin $R^1$ Wasserstoff ist, in die Verbindungen der Formel (I), worin $R^1$ eine andere Bedeutung als Wasserstoff besitzt, durch Umsetzen des Vorherstehenden mit einem *N*-alkylierenden Mittel.

l) O-Alkylieren der Verbindungen der Formel (I), worin L ein mit Hydroxy substituierter $C_{1-6}$Alkylrest ist, um die entsprechenden O-alkylierten Verbindungen der Formel (I) herzustellen; worin D einen Rest der Formel

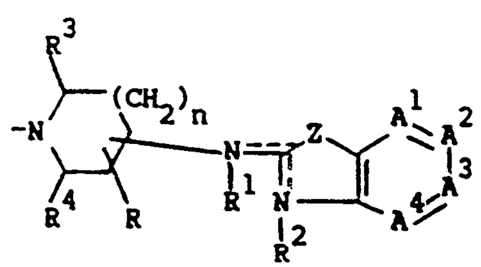

darstellt; und wenn gewünscht, Überführen der Verbindungen ineinander unter Nachvollziehen technikbekannter Umwandlungsverfahren für funktionelle Gruppen; und wenn ferner gewünscht, Überführen der Verbindungen (I) in eine therapeutisch wirksame, nicht-toxische Säureadditionssalzform durch Behandlung mit einer geeigneten Säure oder umgekehrt, Überführen des Säureadditionssalzes in die freie Basenform mit Alkali; und/oder Herstellen stereochemisch isomerer Formen hievon.

**Revendications**

1. Composé chimique répondant à la formule

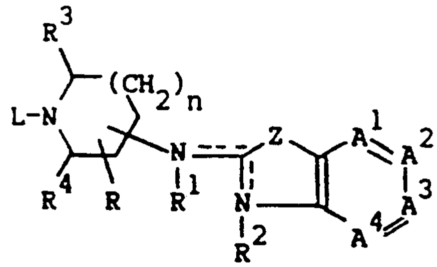

(I)

et un sel d'addition pharmaceutiquement acceptable ou une forme isomère stéréochimique possible de celui-ci, dans laquelle:

$—A^1=A^2—A^3=A^4—$ est un radical bivalent répondant à la formule

$—CH=CH—CH=CH—$ (a);
$—N=CH—CH=CH—$ (b);
$—CH=N—CH=CH—$ (c);
$—CH=CH—N=CH—$ (d);
ou $—CH=CH—CH=N—$ (e)

où un ou deux atomes d'hydrogène du radical $—A^1=A^2—A^3=A^4—$ peuvent, indépendamment l'un de l'autre, être remplacés par un halogéno, un alkyle en $C_{1-6}$, un hydroxy, un alkyloxy en $C_{1-6}$ ou un trifluorométhyle;

Z est O ou S;

n est 0 ou l'entier 1;

R est choisi dans le groupe constitué par un hydrogène, un alkyle en $C_{1-6}$, un hydroxy et un alkyloxy en $C_{1-6}$;

$R^1$ ou $R^2$ est un hydrogène, un alkyle en $C_{1-6}$ ou un aryl(alkyle en $C_{1-6}$); le pointillé entre l'atome d'azote portant $R^1$, l'atome de carbone intermédiaire et l'atome d'azote portant $R^2$ indiquant qu'une double liaison existe entre l'atome d'azote portant $R^1$ et ledit atome de carbone intermédiaire, auquel cas $R^1$ est absent, ou qu'une double liaison existe entre l'atome de carbone intermédiaire et l'azote portant $R^2$, auquel cas $R^2$ est absent; et où ledit atome de carbone intermédiaire est l'atome de carbone placé entre l'azote portant $R^1$ et l'azote portant $R^2$;

$R^3$ ou $R^4$ sont tous deux des hydrogènes ou $R^3$ et $R^4$ combinés peuvent former un radical bivalent de formule $—CH_2—CH_2—$;

L est choisi dans le groupe constitué par un alkyle en $C_{1-12}$, un alkyle en $C_{1-6}$ substitué, un aryl(alcényle en $C_{2-6}$), un cycloalkyle en $C_{3-6}$, un cycloalkyle en $C_{3-6}$ substitué, un radical de formule

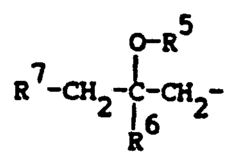

(a),

et un radical de formule

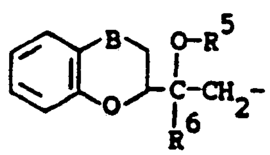

(b);

$R^5$ étant un hydrogène ou un (alkyl en $C_{1-12}$)carbonyle;

$R^6$ étant un hydrogène ou un alkyle en $C_{1-6}$;

$R^7$ étant un aryle, un radical $R^8—O$ ou un radical $R^8—S$;

B étant un radical bivalent de formule $—CH_2—$ ou $—O—$; et ledit $R^8$ étant un hydrogène; un aryle; un 2,3-dihydro-1H-indényle; un benzodioxolyle; un (2,3-dihydro-1,4-benzodioxine-2-yl)méthyle; un (2H-1-benzopyranne-2-yl)méthyle; ou un phényle substitué par un alcényloxy en $C_{2-6}$, un (alkyl en $C_{1-6}$)carbonylamino ou un (alkyl en $C_{1-6}$)phénylcarbonyle; et où ledit cycloalkyle en $C_{3-6}$ substitué est un cycloalkyle en $C_{3-6}$ qui est substitué par jusqu'à deux substituants choisis chacun indépendamment dans le groupe constitué par un aryle, un aryloxy et un cyano; ledit alkyle en $C_{1-6}$ substitué est un alkyle en $C_{1-6}$ qui est substitué par un composant du groupe constitué par un cycloalkyle en $C_{3-6}$, un pyridinyle, un pyridinyloxy, un aryle, un benzimidazolyle, un indolyle, un isoxazolyle facultativement substitué par un

phényle, un 3-oxo-1,2,4-triazolo[4,5-a]pyridine-2(3H)-yle, un radical de formule ArY-, un radical de formule $R^9O$— et un radical de formule

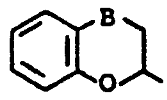

(c);

ledit Ar étant un aryle;

Y étant O, S, NH, —CONH—, —CHCN, —CHOR$^{10}$ ou —$SO_2$;

$R^9$ étant un hydrogène, un aryl(alkyle en $C_{1-6}$), un cycloalkyle en $C_{3-6}$, un phényle substitué par un phényle ou un phényle substitué par un (alkyloxy en $C_{1-6}$)carbonyle;

$R^{10}$ étant un hydrogène ou un alkyle en $C_{1-6}$; et où un aryle est un phényle facultativement substitué par jusqu'à trois substituants choisis chacun indépendamment dans le groupe constitué par un alkyle en $C_{1-6}$, un alkyloxy en $C_{1-6}$, un halogéno, un trifluorométhyle, un cyano, un (alkyl en $C_{1-6}$) carbonyle, un nitro, un amino et un aminocarbonyle.

2. Composé chimique selon la revendication 1, dans lequel L est un aryl(alcényle en $C_{2-6}$), un alkyle en $C_{1-6}$ substitué par un aryloxy, un radical de formule (a) ou un radical de formule (b), où aryle est comme défini dans la revendication 1.

3. Composé chimique selon la revendication 2, dans lequel —$A^1$=$A^2$—$A^3$=$A^4$— est —CH=CH—CH=CH—, n est l'entier 1, $R^2$ est absent et une double liaison existe entre l'azote portant $R^2$ et l'atome de carbone intermédiaire, et $R^3$ et $R^4$ sont tous deux des atomes d'hydrogène.

4. Composé chimique selon la revendication 3, dans lequel, dans le radical de formule (a), $R^5$ et $R^6$ sont tous deux un hydrogène et $R^7$ est un radical $R^8$—O—, où $R^8$ est un aryle; ou dans le radical de formule (b), B est O et $R^5$ et $R^6$ sont tous deux des atomes d'hydrogène, où aryle est comme défini dans la revendication 1.

5. Composé chimique selon la revendication 4, dans lequel $R^1$ est un alkyle en $C_{1-6}$ et aryle est un halogénophényle.

6. Composé chimique selon la revendication 1 qui est le 4-[(2-benzothiazolyl)méthylamino]-α-[(4-fluorophénoxy)méthyl]-1-pipéridine-éthanol.

7. Composition pharmaceutique comprenant un véhicule pharmaceutique approprié et, comme ingrédient actif, une quantité thérapeutique efficace d'un composé selon l'une quelconque des revendications 1 à 6.

8. Composition anti-anoxique ou antihypoxique comprenant un véhicule pharmaceutique approprié et, comme ingrédient actif, une quantité anti-anoxique ou antihypoxique efficace d'un composé selon l'une quelconque des revendications 1 à 6.

9. Procédé pour préparer une composition pharmaceutique, caractérisé en ce que l'on mélange intimement une quantité thérapeutique efficace d'un composé selon l'une quelconque des revendications 1 à 6 avec des véhicules pharmaceutiques appropriés.

10. Composé selon l'une quelconque des revendications 1 à 6 pour l'utilisation comme médicament.

11. Composé selon l'une quelconque des revendications 1 à 6 pour l'utilisation comme médicament anti-anoxique ou antihypoxique.

12. Procédé pour préparer un composé chimique selon la revendication 1, caractérisé par

a) la N-alkylation d'une pipéridine de formule

$$H—D \qquad (III),$$

avec un composé réagissant de formule

$$L—W \qquad (II),$$

dans laquelle W représente un groupe réactif labile, dans un solvant inerte dans la réaction;

b) la N-alkylation par réduction d'une pipéridine de formule (III) avec un composé carbonylique de formule

$$L'=C=O \qquad (IV),$$

dans laquelle L'=C=O est un composé de formule L—H dans laquelle un radical —$CH_2$— est oxydé en un radical carbonyle, dans un solvant inerte dans la réaction;

c) la cyclisation d'un dérivé d'urée ou de thio-urée de formule

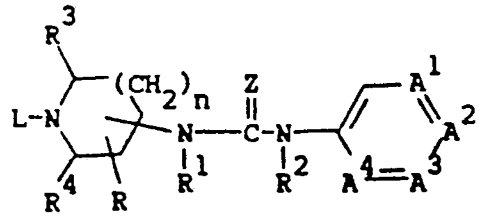

(V)

dans un solvant inerte dans la réaction en présence d'un oxydant;

d) la réaction d'une pipéridine de formule (III) avec un oxiranne de formule

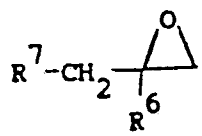

(VI)

dans un solvant inerte dans la réaction, pour préparer ainsi un composé de formule

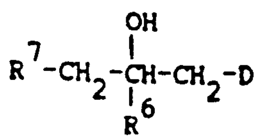

(I-a-1),

et, si on le désire, l'acylation de (I-a-1) avec un composé réagissant

$$R^{5-a}-W^1 \quad \text{(VII)},$$

ledit $R^{5-a}$—$W^1$ représentant un acide alcanoïque ou un dérivé réactif de celui-ci, pour préparer ainsi un composé de formule

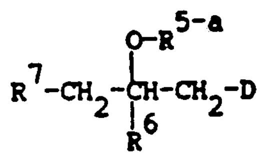

(I-a-2);

e) la réaction d'une pipéridine de formule (III) avec un oxiranne de formule

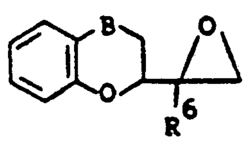

(VIII)

dans un solvant inerte dans la réaction, pour préparer ainsi un composé de formule

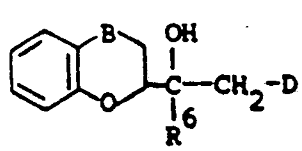

(I-a-3)

et, si on le désire, l'acylation de (I-a-3) avec un composé réagissant (VII), pour préparer ainsi un composé de formule

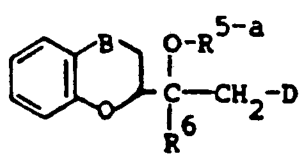

(I-a-4)

f) la réaction d'un benzothiazole ou d'un benzoxazole de formule

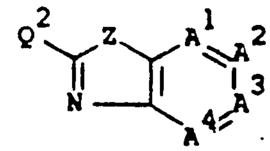

(X)

avec un composé réagissant de formule

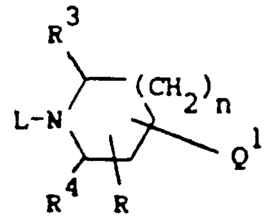

(IX)

dans un solvant inerte dans la réaction, où $Q^1$ est un groupe réactif labile et $Q^2$ est un radical de formule —$NHR^1$, ou sinon $Q^1$ est un radical de formule —$NHR^1$ et $Q^2$ est un groupe labile réactif, pour préparer ainsi un composé de formule

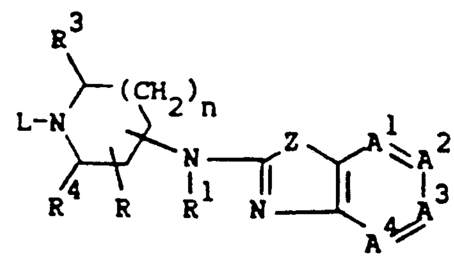

(I-b);

g) la cyclodésulfuration d'un dérivé de thio-urée de formule

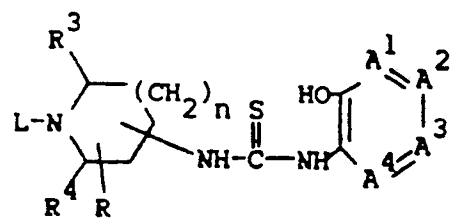

(XIV)

que l'on peut préparer in situ par condensation d'un isothiocyanate

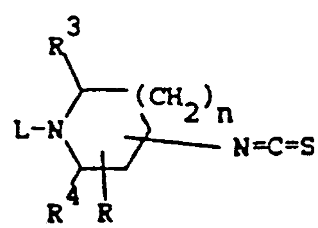

(XII)

avec un aminoalcool aromatique de formule

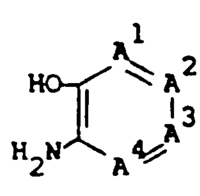

(XIII).

pour préparer ainsi un composé de formule

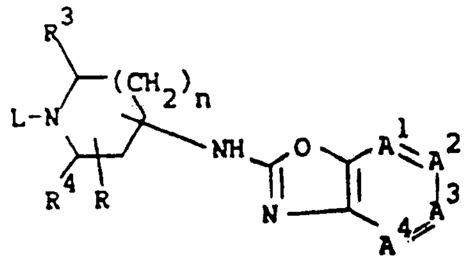

(I-b-1);

h) la cyclisation d'un dérivé d'urée ou de thio-urée de formule

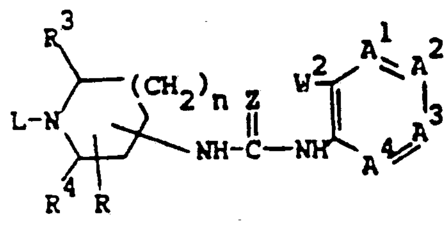

(XVII)

que l'on peut former in situ par réaction d'un isocyanate ou d'un isothiocyanate de formule

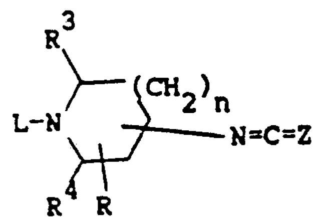

(XV)

avec une amine aromatique de formule

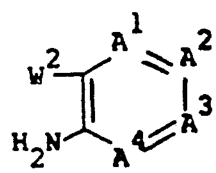

(XVI).

dans laquelle $W^2$ représente un groupe labile, pour préparer ainsi un composé de formule

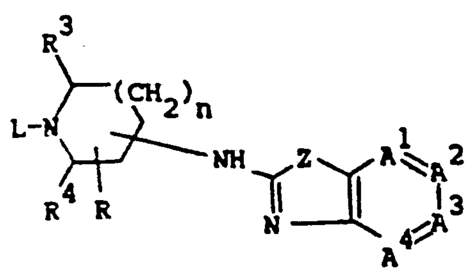

(I-b-2)

i) la réaction d'une pipéridine de formule (III) avec un dérivé d'alcène de formule

$$R^{11}\text{-alcènediyl en } C_{2-6}\text{-H} \qquad \text{(XVIII)}$$

dans un solvant inerte dans la réaction pour préparer ainsi un composé de formule

$$R^{11}—C_rH_{2r}—D \qquad \text{(I-c)}$$

dans laquelle $R^{11}$ représente un alkyle en $C_{1-6}$, un cycloalkyle en $C_{3-6}$, un pyridinyle, un pyridinyloxy, un aryle, un benzimidazolyle, un indolyle, un isoxazolyle substitué par un phényle, un 3-oxo-1,2,4-triazolo[4,5-a]pyridine-2(3H)-yle, un radical de formule ArY, un radical de formule $R^9O$ et un radical de formule (g); et r est un entier de 2 à 6 inclus;

j) la réaction d'une pipéridine de formule (III) avec une aziridine de formule

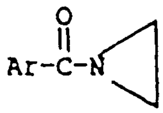

(XIX)

dans un solvant inerte dans la réaction pour préparer un composé de formule

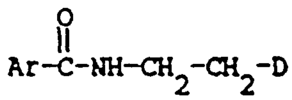

(I-d);

k) la conversion des composés de formule (I) dans laquelle $R^1$ est un hydrogène en les composés de formule (I) dans laquelle $R^1$ est autre qu'un hydrogène, par réaction des premiers avec un agent de N-alkylation;

l) la O-alkylation des composés de formule (I) dans laquelle L est un radical alkyle en $C_{1-6}$ substitué par un hydroxy, pour préparer les composés O-alkylés correspondants de formule (I); dans laquelle D représente un radical de formule

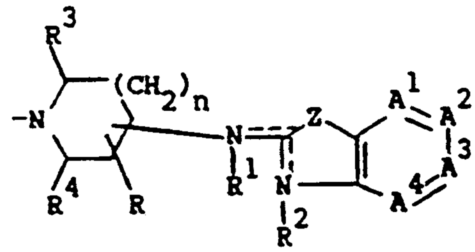

et, si on le désire, la conversion des composés les uns en les autres selon des réactions de transformation des groupes fonctionnels connues dans l'art; et, de plus, si on le désire, la conversion des composés de formule (I) en un sel d'addition d'acide non toxique thérapeutiquement actif par traitement avec un acide approprié, ou inversement la conversion du sel d'addition d'acide en la base libre avec un alcali; et/ou la préparation des formes isomères stéréochimiques correspondantes.